# EUROPEAN PATENT SPECIFICATION

(11) **EP 1 680 141 B1**
(45) Date of publication and mention of the grant of the patent: **11.08.2010**
(21) Application number: 04810418.6
(22) Date of filing: 04.11.2004
(51) Int. Cl.: A61K 39/395

(54) **METHODS OF THERAPY FOR SOLID TUMORS EXPRESSING THE CD40 CELL-SURFACE ANTIGEN**
VERFAHREN ZUR BEHANDLUNG VON SOLIDEN TUMOREN MIT EXPRESSION DES CD40-ZELLOBERFLÄCHEN-ANTIGENS
PROCEDES THERAPEUTIQUES DE TUMEURS SOLIDES EXPRIMANT L'ANTIGENE DE SURFACE CELLULAIRE CD40

(30) Priority: 04.11.2003 US 517337 P; 26.11.2003 US 525579 P; 27.04.2004 US 565710 P; 27.04.2004 US 565634 P
(43) Date of publication of application: 19.07.2006
(73) Proprietor: Novartis Vaccines and Diagnostics, Inc., Emeryville, CA 94608 (US)
(72) Inventor: LONG, Li, c/o Chiron Corporation, Emeryville, CA 94662-8097 (US); LUQMAN, Mohammad, c/o Chiron Corporation, Emeryville, CA 94662-8097 (US); YABANNAVAR, Asha, c/o Chiron Corporation, Emeryville, CA 94662-8097 (US); ZAROR, Isabel, c/o Chiron Corporation, Emeryville, CA 94662-8097 (US)
(74) Representative: Marshall, Cameron John
(86) International application number: PCT/US2004/036955
(87) International publication number: WO 2005/044305

(56) References cited:
- WO-A-95/17202
- WO-A-96/18413
- WO-A-02/060485
- US-A- 5 801 227
- US-A- 5 874 082
- LITTLE M. ET AL: "Of mice and men: hybridoma and recombinant antibodies" REVIEW IMMUNOLOGY TODAY, vol. 21, pages 364-370,
- BALINT ROBERT; LARRICK JAMES: "Antibody engineering by parsimonious mutagenesis" GENE, vol. 137, pages 109-118,

## Description

### FIELD OF THE INVENTION

The invention relates to medicaments for treating, and in vitro methods for inhibiting the growth of, solid tumors using antagonist anti-CD40 human antibodies, where the solid tumors comprise carcinoma cells that express the CD40 cell-surface antigen.

### BACKGROUND OF THE INVENTION

CD40 is a 55 kDa cell-surface antigen present on the surface of both normal and neoplastic human B cells, dendritic cells, antigen presenting cells (APCs), endothelial cells, monocytic and epithelial cells, some epithelial carcinomas, and many solid tumors, including lung, breast, ovary, and colon cancers. Transformed cells from patients with low- and high-grade B-cell lymphomas, B-cell acute lymphoblastic leukemia, multiple myeloma, chronic lymphocytic leukemia, and Hodgkin's disease express CD40. CD40 expression is also detected in two-thirds of acute myeloblastic leukemia cases and 50% of AIDS-related lymphomas. Malignant B cells from several tumors of B-cell lineage express a high degree of CD40 and appear to depend on CD40 signaling for survival and proliferation. CD40-expressing carcinomas include urinary bladder carcinoma (Paulie et al. (1989) J. Immunol. 142:590-595; Braesch-Andersen et al. (1989) J. Immunol. 142:562-567), breast carcinoma (Hirano et al. (1999) Blood 93:2999-3007; Wingett et al. (1998) Breast Cancer Res. Treat. 50:27-36); prostate cancer (Rokhlin et al. (1997) Cancer Res. 57:1758-1768), renal cell carcinoma (Kluth et al. (1997) Cancer Res. 57:891-899), undifferentiated nasopharyngeal carcinoma (UNPC) (Agathanggelou et al. (1995) Am. J. Pathol. 147:1152-1160), squamous cell carcinoma (SCC) (Amo et al. (2000) Eur. J. Dermatol. 10:438-442; Posner et al. (1999) Clin. Cancer Res. 5:2261-2270), thyroid papillary carcinoma (Smith et al. (1999) Thyroid 9:749-755), cutaneous malignant melanoma (van den Oord et al. (1996) Am. J. Pathol. 149:1953-1961), multiple myeloma (Maloney et al. (1999) Semin. Hematol. 36(1 Suppl 3):30-33), Hodgkin and Reed-Sternberg cells (primed cells) (Gruss et al. (1994) Blood 84:2305-2314), gastric carcinoma (Yamaguchi et al. (2003) Int. J. Oncol. 23(6):1697-702), sarcomas (see, for example, Lollini et al. (1998) Clin. Cancer Res. 4(8):1843-849, discussing human osteosarcoma and Ewing's sarcoma), and liver carcinoma (see, for example, Sugimoto et al. (1999) Hepatology 30(4):920-26, discussing human hepatocellular carcinoma).

The CD40 antigen is related to the human nerve growth factor (NGF) receptor, tumor necrosis factor-α (TNF-α) receptor, and Fas, suggesting that CD40 is a receptor for a ligand with important functions in B-cell activation. It has been shown to play a critical role in normal B-cell development and function. CD40 expression on APCs plays an important co-stimulatory role in the activation of both T-helper and cytotoxic T lymphocytes. The CD40 receptor is expressed on activated T cells, activated platelets, and inflamed vascular smooth muscle cells. CD40 receptors can also be found on eosinophils, synovial membranes in rheumatoid arthritis, dermal fibroblasts, and other non-lymphoid cell types. Binding of CD40L to the CD40 receptor stimulates B-cell proliferation and differentiation, antibody production, isotype switching, and B-cell memory generation. Though some carcinoma cells exhibit high levels of CD40 expression, the role of CD40 signaling in relation to CD40 expression on these cancer cells is less well understood.

WO-A-96/18413 discloses the treatment of carcinomas in epithelial cells, and the prevention of epithelial cell proliferation, using a CD40 receptor binder. The CD40 receptor binder may be used in conjunction with at least one anti-neoplastic agent and/or CD40 inducer. Immunoregulatory antibodies are disclosed by WO-A-02/060485, which discloses a combination antibody therapy for treating B cell malignancies using an immunoregulatory antibody, especially an anti-B7, anti-CD23 or anti-CD40L antibody and a B cell depleting antibody, especially anti-CD19, anti-CD20, anti-CD22 or anti-CD37.

A majority of the cancer cases are represented by the so-called solid tumors. Given their high incidence, methods for treating these cancers are needed.

### BRIEF SUMMARY OF THE INVENTION

The invention provides a human anti-CD40 monoclonal antibody that is capable of specifically binding to CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay,
for use in treating a human subject for a solid tumor comprising carcinoma cells expressing CD40 antigen.

The invention also provides the use of an effective amount of a human anti-CD40 monoclonal antibody that is capable of specifically binding to CD40 antigen in the manufacture of a medicament for treating a human subject for a solid tumor comprising carcinoma cells expressing CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent

Deposit No. PTA-5542 or the monoclonal antibody CHIR-12.12 obtainable from the hybridoma cell line deposited with the ATCC as Patent Deposit No. PTA-5543, in a competitive binding assay.

The invention also provides an *in vitro* method for inhibiting the growth of a solid tumor comprising carcinoma cells expressing CD40 antigen, said method comprising contacting said cells with an effective amount of a human anti-CD40 monoclonal antibody that is capable of specifically binding to said CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay

The invention also provides a human anti-CD40 monoclonal antibody that is capable of specifically binding to CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay,
for use in inhibiting the growth of a solid tumor comprising carcinoma cells expressing CD40 antigen.

The invention also provides the use of an effective amount of a human anti-CD40 monoclonal antibody that is capable of specifically binding to CD40 antigen in the manufacture of a medicament for inhibiting the growth of a solid tumor comprising carcinoma cells expressing CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay.

Other aspects of the invention are set out in the appended claims.

### BRIEF SUMMARY OF THE DISCLOSURE

Methods are disclosed for treating a subject for a solid tumor, where the carcinoma cells of the solid tumor express the CD40 cell-surface antigen. The methods comprise treating the subject with an antagonist anti-CD40 monoclonal antibody or an antigen-binding fragment thereof that is free of significant agonist activity when bound to CD40 antigen on a human CD40-expressing cell. Binding of the antagonist anti-CD40 monoclonal antibody (or suitable antigen-binding fragment thereof comprising the Fc portion of the antagonist anti-CD40 antibody) to CD40 antigen expressed on carcinoma cells results in antibody-dependent cell-mediated cytotoxicity (ADCC)-dependent killing of these carcinoma cells. In some embodiments, the antagonist anti-CD40 antibodies are administered in combination with one or more other cancer therapy protocols, including, but not limited to, surgery, radiation therapy, chemotherapy, cytokine therapy, or other monoclonal antibody intended for use in treatment of the solid tumor. Solid tumors that can be treated or prevented by the methods of the present disclosure include, but are not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas). Methods for inhibiting the growth of solid tumors comprising CD40-expressing carcinoma cells are also disclosed.

Suitable antibodies for use in the methods of the invention include the monoclonal antibodies described herein, which have a strong affinity for CD40 and are characterized by a dissociation equilibrium constant (K_{D}) of at least 10⁻⁶ M, preferably at least about 10⁻⁷ M to about 10⁻⁸ M, more preferably at least about 10⁻⁸ M to about 10⁻¹² M. Monoclonal antibodies and antigen-binding fragments thereof that are suitable for use in the methods of the invention are capable of specifically binding to a human CD40 antigen expressed on the surface of a human cell, particularly a human carcinoma cell of a solid tumor. They are free of significant agonist activity but exhibit antagonist activity when bound to CD40 antigen on human cells, as demonstrated for CD40-expressing normal and neoplastic human B cells. Suitable monoclonal antibodies have human constant regions; preferably they also have wholly or partially humanized framework regions; and most preferably are fully human antibodies or antigen-binding fragments thereof.

Examples of such monoclonal antibodies are the antibodies designated herein as CHIR-5.9 and CHIR-12.12, which can be recombinantly produced; the monoclonal antibodies produced by the hybridoma cell lines designated 131.2F8.5.9 (referred to herein as the cell line 5.9) and 153.8E2.D10.D6.12.12 (referred to herein as the cell line 12.12); a monoclonal antibody comprising an amino acid sequence selected from the group consisting of the sequence shown in SEQ ID NO:6, the sequence shown in SEQ ID NO:7, the sequence shown in SEQ ID NO:8, both the sequence shown in SEQ ID NO:6 and SEQ ID NO:7, and both the sequence shown in SEQ ID NO:6 and SEQ ID NO:8; a monoclonal antibody comprising an amino acid sequence selected from the group consisting of the sequence shown in SEQ ID NO:2, the sequence shown in SEQ ID NO:4, the sequence shown in SEQ ID NO:5, both the sequence shown in SEQ ID NO:2 and SEQ ID NO:4, and both the sequence shown in SEQ ID NO:2 and SEQ ID NO:5; a monoclonal antibody comprising an amino acid sequence encoded by a nucleic acid molecule comprising a nucleotide sequence selected from the group consisting of the sequence shown in SEQ ID NO:1, the sequence shown in SEQ ID NO:3, and both the sequence shown in SEQ ID NO:1 and SEQ ID NO:3; and antigen-binding fragments of these monoclonal antibodies that retain the capability of specifically binding to human CD40, and which are free of significant agonist activity but exhibit antagonist activity when bound to CD40 antigen on human cells, as demonstrated for CD40-expressing normal and neoplastic human B cells. Examples of such monoclonal antibodies also include a monoclonal antibody that binds to an epitope capable of binding the monoclonal antibody produced by the hybridoma cell line 12.12 or the monoclonal antibody produced by the hybridoma cell line 5.9; a monoclonal antibody that binds to an epitope comprising residues 82-87 of the amino acid sequence shown in SEQ ID NO: 10 or SEQ ID NO: 12; a monoclonal antibody that competes with the monoclonal antibody CHIR-12.12 or CHIR-5.9 in a competitive binding assay; and a monoclonal antibody that is an antigen-binding fragment of the CHIR-12.12 monoclonal antibody or any of the foregoing monoclonal antibodies, where the fragment retains the capability of specifically binding to the human CD40 antigen and exhibits antagonist activity when bound to this antigen, as demonstrated for CD40-expressing normal and neoplastic human B cells.

In one embodiment of the disclosure, methods of treatment comprise administering to a patient a therapeutically effective dose of a pharmaceutical composition comprising suitable antagonistic anti-CD40 antibodies or antigen-binding fragments thereof. A therapeutically effective dose of the anti-CD40 antibody or fragment thereof is in the range from about 0.01 mg/kg to about 40 mg/kg, from about 0.01 mg/kg to about 30 mg/kg, from about 0.1 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. It is recognized that the method of treatment may comprise a single administration of a therapeutically effective dose or multiple administrations of a therapeutically effective dose of the antagonist anti-CD40 antibody or antigen-binding fragment thereof.

The antagonist anti-CD40 antibodies identified herein as being suitable for use in the methods of the invention may be modified. Modifications of these antagonist anti-CD40 antibodies include, but are not limited to, immunologically active chimeric anti-CD40 antibodies, humanized anti-CD40 antibodies, and immunologically active murine anti-CD40 antibodies.

### BRIEF DESCRIPTION OF THE DRAWINGS

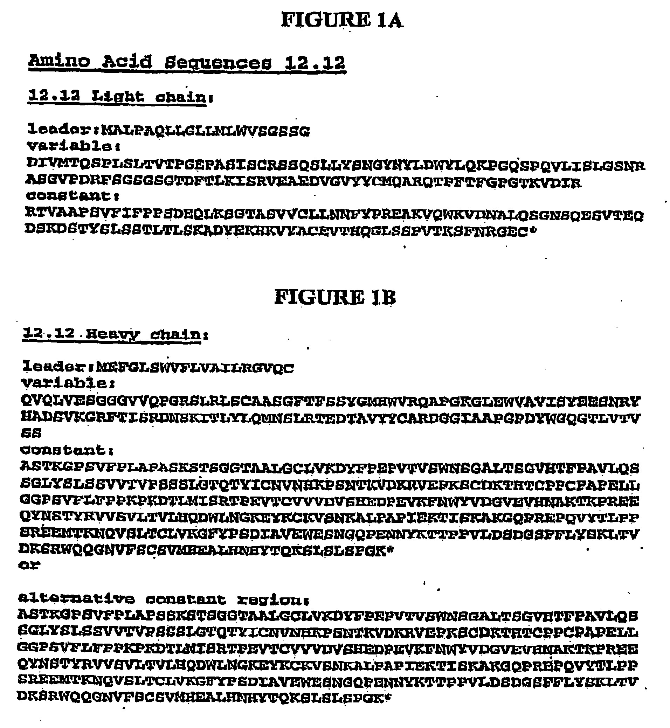

Figure 1 sets forth the amino acid sequences for the light and heavy chains of the mAb CHIR-12.12. The leader (residues 1-20 of SEQ ID NO:2), variable (residues 21-132 of SEQ ID NO:2), and constant (residues 133-239 of SEQ ID NO:2) regions of the light chain are shown in Figure 1A. The leader (residues 1-19 of SEQ ID NO:4), variable (residues 20-139 of SEQ ID NO:4), and constant (residues 140-469 of SEQ ID NO:4) regions of the heavy chain are shown in Figure 1B. The alternative constant region for the heavy chain of the mAb CHIR-12.12 shown in Figure 1 B reflects a substitution of a serine residue for the alanine residue at position 153 of SEQ ID NO:4. The complete sequence for this variant of the heavy chain of the mAb CHIR-12.12 is set forth in SEQ ID NO:5.
Figure 2 shows the coding sequence for the light chain (Figure 2A; SEQ ID NO:1) and heavy chain (Figure 2B; SEQ ID NO:3) for the mAb CHIR-12.12.

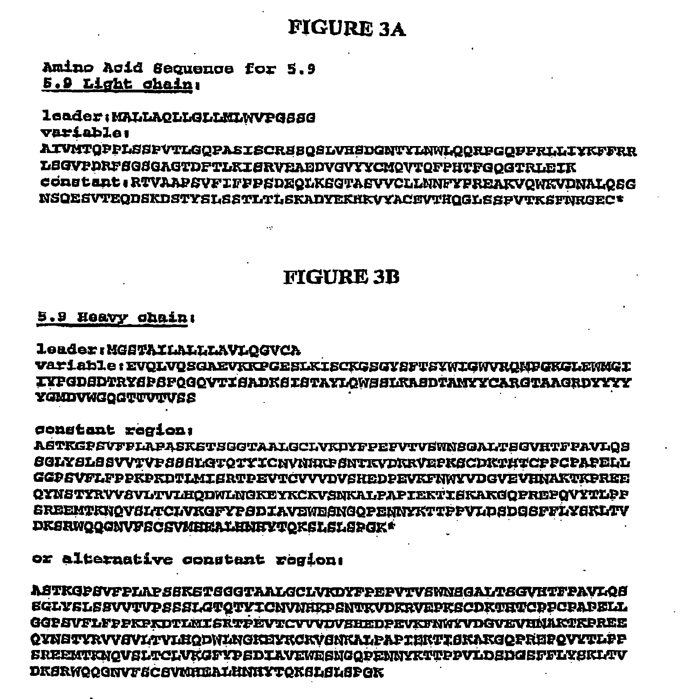

Figure 3 sets forth the amino acid sequences for the light and heavy chains of mAb CHIR-5.9. The leader (residues 1-20 of SEQ ID NO:6), variable (residues 21-132 of SEQ ID NO:6), and constant (residues 133-239 of SEQ ID NO:6) regions of the light chain are shown in Figure 3A. The leader (residues 1-19 of SEQ ID NO:7), variable (residues 20-144 of SEQ ID NO:7), and constant (residues 145-474 of SEQ ID NO:7) regions of the heavy chain are shown in Figure 3B. The alternative constant region for the heavy chain of the mAb CHIR-5.9 shown in Figure 3B reflects a substitution of a serine residue for the alanine residue at position 158 of SEQ ID NO:7. The complete sequence for this variant of the heavy chain of the mAB CHIR-5.9 is set forth in SEQ ID NO:8.
Figure 4 shows the coding sequence (Figure 4A; SEQ ID NO:9) for the short isoform of human CD40 (amino acid sequence shown in Figure 4B; SEQ ID NO:10), and the coding sequence (Figure 4C; SEQ ID NO:11) for the long isoform of human CD40 (amino acid sequence shown in Figure 4D).
Figure 5 shows antibody-dependent cell-mediated cytotoxicity (ADCC) activity of monoclonal antibodies CHIR-5.9 and CHIR-12.12 on the ovarian cancer cell lines SKO3 (Figure 5A) and Hey (Figure 5B), the skin squamous cancer cell line A431 (Figure 5C), and the colon cancer cell line HCT116 (Figure 5D).
Figure 6 shows antibody-dependent cell-mediated cytotoxicity (ADCC) activity of monoclonal antibodies CHIR-5.9 and CHIR-12.12 on the breast cancer cell lines MDA-MB231 (Figure 6A) and MDA-MB435 (Figure 6B), and the lung cancer cell lines NCI-H460 (Figure 6C) and SK-MES-1 (Figure 6D).
Figure 7 demonstrates *in vivo* anti-tumor activity of monoclonal antibodies CHIR-5.9 (denoted 5.9 in figure) and CHIR-12.12 (denoted 12.12 in figure) using a xenograft colon cancer model based on the human colon carcinoma cell line HCT116.
Figure 8 shows the effects of intraperitoneally administered anti-CD40 monoclonal antibody CHIR-12.12 (denoted 12.12 in figure) or anti-HER2 monoclonal antibody Herceptin^{®} on percent survival in an unstaged orthotopic murine model of ovarian cancer using the human ovarian cancer cell line SKOV3i.p.1.
Figure 9 compares the effects of intraperitoneally versus intravenously administered monoclonal antibody CHIR-12.12 (denoted 12.12 in figure) or Herceptin^{®} on percent survival in an unstaged orthotopic murine model of ovarian cancer using the human ovarian cancer cell line SKOV3i.p.1.
Figure 10 demonstrates *in vivo* anti-tumor activity of monoclonal antibodies CHIR-12.12 (denoted 12.12 in figure) and CHIR-5.9 (denoted 5.9 in figure) versus that observed for Herceptin^{®} using a staged murine model of ovarian cancer based on the human ovarian cancer cell line SKOV3i.p1.
Figure 11 shows thermal melting temperature ofCHIR-12.12 in different pH formulations measured by differential scanning calorimetry (DSC).

### DETAILED DESCRIPTION OF THE INVENTION

"Tumor," as used herein, refers to all neoplastic cell growth and proliferation, whether malignant or benign, and all pre-cancerous and cancerous cells and tissues. The term "solid tumor" refers to a cancer or carcinoma of body tissues other than blood, bone marrow, and lymphoid system.

The terms "cancer" and "cancerous" refer to or describe the physiological condition in mammals that is typically characterized by unregulated cell growth. Examples of cancers that are classified as solid tumors include but are not limited to lung cancer, breast cancer, ovarian cancer, colon cancer, liver cancer, gastric cancer, prostate, and skin cancer.

"Antibodies" and "immunoglobulins" (Igs) are glycoproteins having the same structural characteristics. While antibodies exhibit binding specificity to an antigen, immunoglobulins include both antibodies and other antibody-like molecules that lack antigen specificity. Polypeptides of the latter kind are, for example, produced at low levels by the lymph system and at increased levels by myelomas.

The term "antibody" is used in the broadest sense and covers fully assembled antibodies, antibody fragments that can bind antigen (e.g., Fab', F'(ab)₂, Fv, single chain antibodies, diabodies), and recombinant peptides comprising the foregoing.

The term "monoclonal antibody" as used herein refers to an antibody obtained from a population of substantially homogeneous antibodies, i.e., the individual antibodies comprising the population are identical except for possible naturally occurring mutations that may be present in minor amounts.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity determining regions (CDRs) or hypervariable regions both in the light-chain and the heavy-chain variable domains. The more highly conserved portions of variable domains are celled the framework (FR) regions. The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al. (1991) NIH Publ. No. 91-3242, Vol. I, pages 647-669).

The constant domains are not involved directly in binding an antibody to an antigen, but exhibit various effecter functions, such as Fc receptor (FcR) binding, participation of the antibody in antibody-dependent cellular toxicity, opsonization, initiation of complement dependent cytotoxicity, and mast cell degranulation.

The term "hypervariable region" when used herein refers to the amino acid residues of an antibody that are responsible for antigen binding. The hypervariable region comprises amino acid residues from a "complementarity determining region" or" CDR" (i.e., residues 24-34 (L1), 50-56 (L2), and 89-97 (L3) in the light-chain variable domain and 31-35 (H1), 50-65 (H2), and 95-102 (H3) in the heavy-chain variable domain; Kabat et al. (1991) Sequences of Proteins of Immunological Interest (5th ed., Public Health Service, National Institute of Health, Bethesda, MD) and/or those residues from a "hypervariable loop" (i.e., residues 26-32(L1), 50-52 (L2), and 91-96 (L3) in the light-chain variable domain and 26-32(H1 53-55 (H2), and 96-101 (H3) in the heavy-chain variable domain; Clothia and Lesk (1987) J. Mol. Biol. 196:901-917). "Framework" or "FR" residues are those variable domain residues other than the hypervariable region residues.

"Antibody fragments" comprise a portion of an intact antibody, preferably the antigen-binding or variable region of the intact antibody. Examples of antibody fragments include Fab, Fab', F(ab')2, and Fv fragments; diabodies; linear antibodies (Zapata et al. (1995) Protein Eng. 8(10):1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of crosslinking antigen.

"Fv" is the minimum antibody fragment that contains a complete antigen recognition and binding site. In a two-chain Fv species, this region consists of a dimer of one heavy- and one light-chain variable domain in tight, non-covalent association. In a single-chain Fv species, one heavy- and one light-chain variable domain can be covalently linked by flexible peptide linker such that the light and heavy chains can associate in a "dimeric" structure analogous to that in a two-chain Fv species. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab fragments differ from Fab' fragments by the addition of a few residues at the carboxy terminus of the heavy-chain C_{H}1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of human immunoglobulins: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. Different isotypes have different effector functions. For example, human IgG1 and IgG3 isotypes mediate antibody-dependent cell-mediated cytotoxicity (ADCC) activity.

The word "label" when used herein refers to a detectable compound or composition that is conjugated directly or indirectly to the antibody so as to generate a "labeled" antibody. The label may be detectable by itself (e.g., radioisotope labels or fluorescent labels) or, in the case of an enzymatic label, may catalyze chemical alteration of a substrate compound or composition that is detectable. Radionuclides that can serve as detectable labels include, for example, I-131, I-123, I-125, Y-90, Re-188, Re-186, At-211, Cu-67, Bi-212, and Pd-109. The label might also be a non-detectable entity such as a toxin.

The term "antagonist" is used in the broadest sense, and includes any molecule that partially or fully blocks, inhibits, or neutralizes a biological activity of a native target disclosed herein or the transcription or translation thereof.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, succinate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, polyethylene glycol (PEG), and Pluronics. Administration "in combination with" one or more further therapeutic agents includes simultaneous (concurrent) and consecutive administration in any order.

A "host cell," as used herein, refers to a microorganism or a eukaryotic cell or cell line cultured as a unicellular entity that can be, or has been, used as a recipient for a recombinant vector or other transfer polynucleotides, and include the progeny of the original cell that has been transfected. It is understood that the progeny of a single cell may not necessarily be completely identical in morphology or in genomic or total DNA complement as the original parent, due to natural, accidental, or deliberate mutation.

"Human effector cells" are leukocytes that express one or more FcRs and perform effector functions. Preferably, the cells express at least FcγRIII and carry out antigen-dependent cell-mediated cyotoxicity (ADCC) effector function. Examples of human leukocytes that mediate ADCC include peripheral blood mononuclear cells (PBMC), natural killer (NK) cells, monocytes, macrophages, eosinophils, and neutrophils, with PBMCs and NK cells being preferred. Antibodies that have ADCC activity are typically of the IgG1 or IgG3 isotype. Note that in addition to isolating IgG1 and IgG3 antibodies, such ADCC-mediating antibodies can be made by engineering a variable region from a non-ADCC antibody or variable region fragment to an IgG1 or IgG3 isotype constant region.

The terms "Fc receptor" or "FcR" are used to describe a receptor that binds to the Fc region of an antibody. The preferred FcR is a native-sequence human FcR. Moreover, a preferred FcR is one that binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see Daeron (1997) Annu. Rev. Immunol. 15:203-234). FcRs are reviewed in Ravetch and Kinet (1991) Annu. Rev. Immunol. 9:457-492 (1991); Capel et al. (1994) Immunomethods 4:25-34; and de Haas et al. (1995) J. Lab. Clin. Med. 126:330-341. Other FcRs, including those to be identified in the future, are encompassed by the term "FcR" herein. The term also includes the neonatal receptor, FcRn, which is responsible for the transfer of maternal IgGs to the fetus (Guyer et al. (1976) J. Immunol. 117:587 and Kim et al. (1994) J. Immunol. 24:249 (1994)).

There are a number of ways to make human antibodies. For example, secreting cells can be immortalized by infection with the Epstein-Barr virus (EBV). However, EBV-infected cells are difficult to clone and usually produce only relatively low yields of immunoglobulin (James and Bell (1987) J. Immunol. Methods 100:5-40). In the future, the immortalization of human B cells might possibly be achieved by introducing a defined combination of transforming genes. Such a possibility is highlighted by a recent demonstration that the expression of the telomerase catalytic subunit together with the SV40 large oncoprotein and an oncogenic allele of H-ras resulted in the tumorigenic conversion of normal human epithelial and fibroblast cells (Hahn et al. (1999) Nature 400:464-468). It is now possible to produce transgenic animals (e.g., mice) that are capable, upon immunization, of producing a repertoire of human antibodies in the absence of endogenous immunoglobulin production (Jakobovits et al. (1993) Nature 362:255-258; Lonberg and Huszar (1995) Int. Rev. Immunol. 13:65-93; Fishwild et al. (1996) Nat. Biotechnol. 14:845-851; Mendez et al. (1997) Nat. Genet. 15:146-156; Green (1999) J. Immunol. Methods 231:11-23; Tomizuka et al. (2000) Proc. Natl. Acad. Sci. USA 97:722-727; reviewed in Little et al. (2000) Immunol. Today 21:364-370). For example, it has been described that the homozygous deletion of the antibody heavy-chain joining region (J_{H}) gene in chimeric and germ-line mutant mice results in complete inhibition of endogenous antibody production (Jakobovits et al. (1993) Proc. Natl. Acad. Sci. USA 90:2551-2555).
Transfer of the human germ-line immunoglobulin gene array in such germ-line mutant mice results in the production of human antibodies upon antigen challenge (Jakobovits et al. (1993) Nature 362:255-258). Mendez et al. (1997) (Nature Genetics 15:146-156) have generated a line of transgenic mice that, when challenged with an antigen, generates high affinity fully human antibodies. This was achieved by germ-line integration of megabase human heavy-chain and light-chain loci into mice with deletion into endogenous J_{H} segment as described above. These mice (XenoMouse^{®} II technology (Abgenix; Fremont, California)) harbor 1,020 kb of human heavy-chain locus containing approximately 66 V_{H} genes, complete D_{H} and J_{H} regions, and three different constant regions, and also harbors 800 kb of human κ locus containing 32 Vκ genes, Jκ segments, and Cκ genes. The antibodies produced in these mice closely resemble that seen in humans in all respects, including gene rearrangement, assembly, and repertoire. The human antibodies are preferentially expressed over endogenous antibodies due to deletion in endogenous segment that prevents gene rearrangement in the murine locus. Such mice may be immunized with an antigen of particular interest.

Sera from such immunized animals may be screened for antibody reactivity against the initial antigen. Lymphocytes may be isolated from lymph nodes or spleen cells and may further be selected for B cells by selecting for CD138-negative and CD19-positive cells. In one aspect, such B cell cultures (BCCs) maybe fused to myeloma cells to generate hybridomas as detailed above.

In another aspect, such B cell cultures may be screened further for reactivity against the initial antigen, preferably. Such screening includes ELISA with the target/antigen protein, a competition assay with known antibodies that bind the antigen of interest, and in vitro binding to transiently transfected CHO or other cells that express the target antigen.

The present disclosure is directed to methods for treating human subjects with solid tumors that comprise CD40-expressing carcinoma cells, including, but not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas). The methods involve treatment with an anti-CD40 antibody described herein, or an antigen-binding fragment thereof, where administration of the antibody or antigen-binding fragment thereof promotes a positive therapeutic response within the subject undergoing this method of therapy. Anti-CD40 antibodies suitable for use in the methods of the invention specifically bind a human CD40 antigen expressed on the surface of a human carcinoma cell and are free of significant agonist activity, but exhibit antagonist activity when bound to the CD40 antigen on a human CD40-expressing cell, as demonstrated for CD40-expressing normal and neoplastic human B cells. These anti-CD40 antibodies and antigen-binding fragments thereof are referred to herein as antagonist anti-CD40 antibodies. Such antibodies include, but are not limited to, the fully human monoclonal antibodies CHIR-5.9 and CHIR-2.12 described below and monoclonal antibodies having the binding characteristics of monoclonal antibodies CHIR-5.9 and CHIR-12.12. These monoclonal antibodies, which can be recombinantly produced, are described below.

Antibodies that have the binding characteristics of monoclonal antibodies CHIR-5.9 and CHIR-12.12 include antibodies that competitively interfere with the binding of CHIR-5.9 or CHIR-12.12 to CD40 and/or bind the same epitopes as CHIR-5.9 and CHIR-12.12. One of skill could determine whether an antibody competitively interferes with CHIR-5.9 or CHIR-12.12 using standard methods known in the art.

When these antibodies bind CD40 displayed on the surface of CD40-expressing cells of a solid tumor (also referred to herein as CD40-expressing carcinoma cells), the antibodies are free of significant agonist activity; in some embodiments, their binding to CD40 displayed on the surface of CD40-expressing carcinoma cells results in ADCC-dependent killing of these carcinoma cells, and hence a reduction in tumor volume. Thus, the antagonist anti-CD40 antibodies suitable for use in the methods of the invention include those monoclonal antibodies that can exhibit antagonist activity toward human cells expressing the cell-surface CD40 antigen, as demonstrated for CD40-expressing normal and neoplastic human B cells.

### Antagonist Anti-CD40 Antibodies

The monoclonal antibodies CHIR-5.9 and CHIR-12.12 represent suitable antagonist anti-CD40 antibodies for use in the methods of the present invention. The CHIR-5.9 and 12.2 antibodies are fully human anti-CD40 monoclonal antibodies of the IgG₁ isotype produced from the hybridoma cell lines 131.2F8.5.9 (referred to herein as the cell line 5.9) and 153.8E2.D10.D6.12.12 (referred to herein as the cell line 12.12). These cell lines were created using splenocytes from immunized xenotypic mice containing the human IgG₁ heavy chain locus and the human κ chain locus (XenoMouse^{®} technology; Abgenix; Fremont, California). The spleen cells were fused with the mouse myeloma SP2/0 cells (Sierra BioSource). The resulting hybridomas were sub-cloned several times to create the stable monoclonal cell lines 5.9 and 12.12. Other antibodies of the invention may be prepared similarly using mice transgenic for human immunoglobulin loci or by other methods known in the art and/or described herein.

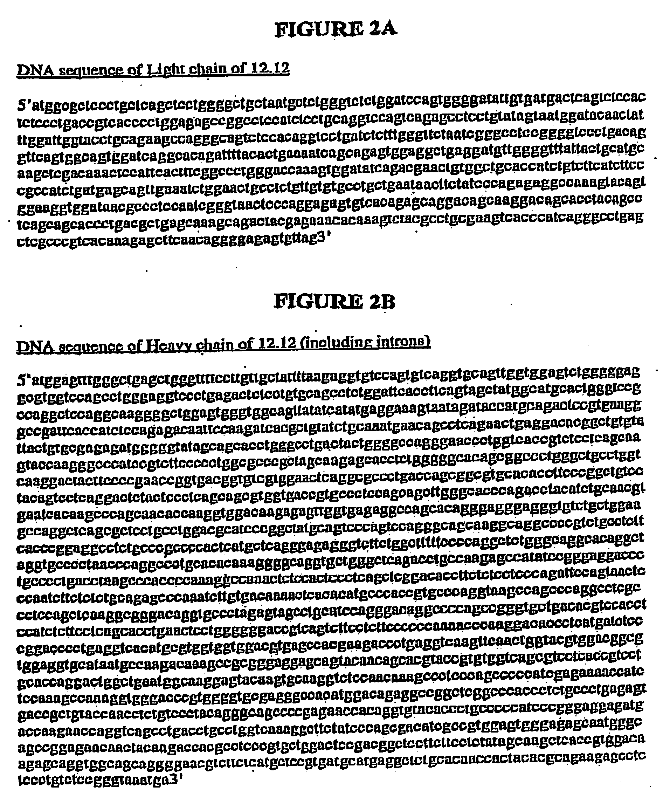

The amino acid sequences for the leader, variable, and constant regions for the light chain and heavy chain for mAb CHIR-12.12 are set forth herein in Figures 1A and 1B, respectively. See also SEQ ID NO:2 (complete sequence for the light chain of mAb CHIR-12.12), SEQ ID NO:4 (complete sequence for the heavy chain for mAb CHIR-12.12), and SEQ ID NO:5 (complete sequence for a variant of the heavy chain for mAb CHIR-12.12 set forth in SEQ ID NO:4, where the variant comprises a serine substitution for the alanine residue at position 153 of SEQ ID NO:4). The nucleotide sequences encoding the light chain and heavy chain for mAb CHIR-12.12 are set forth herein in Figures 2A and 2B, respectively. See also SEQ ID NO:1 (coding sequence for the light chain for mAb CHIR-12.12), and SEQ ID NO:3 (coding sequence for the heavy chain for mAb CHIR-12.12). The amino acid sequences for the leader, variable, and constant regions for the light chain and heavy chain of the CHIR-5.9 mAb are set forth herein in Figures 3A and 3B, respectively. See also SEQ ID NO:6 (complete sequence for the light chain of mAb CHIR-5.9), SEQ ID NO:7 (complete sequence for the heavy chain of mAb CHIR-5.9), and SEQ ID NO:8 (complete sequence for a variant of the heavy chain ofmAb CHIR-5.9 set forth in SEQ ID NO:7, where the variant comprises a serine substitution for the alanine residue at position 158 of SEQ ID NO:7). Further, hybridomas expressing CHIR-5.9 and CHIR--12.12 antibodies have been deposited with the ATCC with a patent deposit designation of PTA-5542 and PTA-5543, respectively.

In addition to antagonist activity, it is preferable that anti-CD40 antibodies suitable for use in the methods of this invention have another mechanism of action against a tumor cell. For example, native CHIR-5.9 and CHIR-12.12 antibodies have ADCC activity. Alternatively, the variable regions of the CHIR-5.9 and CHIR-12.12 antibodies can be expressed on another antibody isotype that has ADCC activity. It is also possible to conjugate native forms, recombinant forms, or antigen-binding fragments of CHIR-5.9 or CHIR-12.12 to a cytotoxin.

The CHIR-5.9 and CHIR-12.12 monoclonal antibodies bind soluble CD40 in ELISA-type assays, prevent the binding of CD40-ligand to cell-surface CD40, and displace the pre-bound CD40-ligand, as determined by flow cytometric assays. Antibodies CHIR-5.9 and CHIR-12.12 compete with each other for binding to CD40 but not with 15B8, the anti-CD40 monoclonal antibody described in PCT International Application No. PCT/US01/30857, published as WO02/28904, also titled *"Human Anti-CD40 Antibodies,"* filed October 2, 2001 (Attorney Docket No. PP 16092.003).
When tested *in vitro* for effects on proliferation of B cells from normal human subjects, CHIR-5.9 and CHIR-12.12 act as antagonistic anti-CD40 antibodies. Furthermore, CHIR-5.9 and CHIR-12.12 do not induce strong proliferation of human lymphocytes from normal subjects. These antibodies are able to kill CD40-expressing target cells (lymphoma lines and solid tumor cell lines) by antibody dependent cellular cytotoxicity (ADCC). The binding affinity of CHIR-5.9 for human CD40 is 1.2x10⁻⁸M and the binding affinity of CHIR-12.12 is 5x10⁻¹⁰M, as determined by the Biacore™ assay.

Suitable antagonist anti-CD40 antibodies for use in the methods of the present invention exhibit a strong single-site binding affinity for the CD40 cell-surface antigen. The monoclonal antibodies of the invention exhibit a dissociation equilibrium constant. (K_{D}) for CD40 of at least 10⁻⁵ M, at least 3 X 10⁻⁵ M, preferably at least 10⁻⁶ M to 10⁻⁷ M, more preferably at least 10⁻⁸ M to about 10⁻¹² M, measured using a standard assay such as Biacore™. Biacore analysis is known in the art and details are provided in the "BIAapplications handbook." Methods described in WO 01/27160 can be used to modulate the binding affinity.

By "CD40 antigen," "CD40 cell surface antigen," "CD40 receptor," or "CD40" is intended a transmembrane glycoprotein that belongs to the tumor necrosis factor (TNF) receptor family (see, for example, U.S. Patent Nos. 5,674,492 and 4,708,871; Stamenkovic et al. (1989) EMBO 8:1403; Clark (1990) Tissue Antigen 36:33; Barclay et al. (1997) The Leucocyte Antigen Facts Book (2d ed.; Academic Press, San Diego)). Two isoforms of human CD40, encoded by alternatively spliced transcript variants of this gene, have been identified. The first isoform (also known as the "long isoform" or "isoform 1 ") is expressed as a 277-amino-acid precursor polypeptide (SEQ ID NO:12 (first reported as GenBank Accession No. CAA43045, and identified as isoform 1 in GenBank Accession No. Nap _001241), encoded by SEQ ID NO:11 (see GenBank Accession Nos. X60592 and NM_001250)), which has a signal sequence represented by the first 19 residues. The second isoform (also known as the "short isoform" or "isoform 2") is expressed as a 203-amino-acid precursor polypeptide (SEQ ID NO:10 (GenBank Accession No. NP_690593), encoded by SEQ ID NO:9 (GenBank Accession No. NM_152854)), which also has a signal sequence represented by the first 19 residues. The precursor polypeptides of these two isoforms of human CD40 share in common their first 165 residues (i.e., residues 1-165 of SEQ ID NO:10 and SEQ ID NO:12). The precursor polypeptide of the short isoform (shown in SEQ ID NO:10) is encoded by a transcript variant (SEQ ID NO:9) that lacks a coding segment, which leads to a translation frame shift; the resulting CD40 isoform contains a shorter and distinct C-terminus (residues 166-203 of SEQ ID NO:10) from that contained in the long isoform of CD40 (C-terminus shown in residues 166-277 of SEQ ID NO:12). For purposes of the present invention, the term "CD40 antigen," "CD40 cell surface antigen," "CD40 receptor," or "CD40" encompasses both the short and long isoforms of CD40. The anti-CD40 antibodies of the present invention bind to an epitope of human CD40 that resides at the same location within either the short isoform or long isoform of this cell surface antigen as noted herein below.

The CD40 antigen is displayed on the surface of a variety of cell types, as described elsewhere herein. By "displayed on the surface" and "expressed on the surface" is intended that all or a portion of the CD40 antigen is exposed to the exterior of the cell. The displayed or expressed CD40 antigen may be fully or partially glycosylated.

By "agonist activity" is intended that the substance functions as an agonist. An agonist combines with a receptor on a cell and initiates a reaction or activity that is similar to or the same as that initiated by the receptor's natural ligand. For example, an agonist of CD40 induces any or all of, but not limited to, the following responses: B cell proliferation and differentiation, antibody production, intercellular adhesion, B cell memory generation, isotype switching, up-regulation of cell-surface expression of MHC Class II and CD80/86, and secretion of pro-inflammatory cytokines such as IL-8, IL-12, and TNF. By "antagonist activity" is intended that the substance functions as an antagonist. For example, an antagonist of CD40 prevents or reduces induction of any of the responses induced by binding of the CD40 receptor to an agonist ligand, particularly CD40L. The antagonist may reduce induction of any one or more of the responses to agonist binding by 5%, 10%, 15%, 20%, 25%, 30%, 35%, preferably 40%, 45%, 50%, 55%, 60%, more preferably 70%, 80%, 85%, and most preferably 90%, 95%, 99%, or 100%. Methods for measuring anti-CD40 antibody and CD40-ligand binding specificity and antagonist activity are known to one of skill in the art and include, but are not limited to, standard competitive binding assays, assays for monitoring immunoglobulin secretion by B cells, B cell proliferation assays, Banchereau-Like-B cell proliferation assays, T cell helper assays for antibody production, co-stimulation ofB cell proliferation assays, and assays for up-regulation of B cell activation markers. See, for example, such assays disclosed in WO 00/75348, U.S. Patent No. 6,087,329.

By "significant" agonist activity is intended an agonist activity of at least 30%, 35%, 40%, 45%, 50%, 60%, 70%, 75%, 80%, 85%, 90%, 95%, or 100% greater than the agonist activity induced by a neutral substance or negative control as measured in an assay of a B cell response. Preferably, "significant" agonist activity is an agonist activity that is at least 2-fold greater or at least 3-fold greater than the agonist activity induced by a neutral substance or negative control as measured in an assay of a B cell response. Thus, for example, where the B cell response of interest is B cell proliferation, "significant" agonist activity would be induction of a level of B cell proliferation that is at least 2-fold greater or at least 3-fold greater than the level of B cell proliferation induced by a neutral substance or negative control. In one embodiment, a non-specific immunoglobulin, for example IgG1, that does not bind to CD40 serves as the negative control. A substance "free of significant agonist activity" would exhibit an agonist activity of not more than about 25% greater than the agonist activity induced by a neutral substance or negative control, preferably not more than about 20% greater, 15% greater, 10% greater, 5% greater, 1% greater, 0.5% greater, or even not more than about 0.1% greater than the agonist activity induced by a neutral substance or negative control as measured in an assay of a B cell response. The antagonist anti-CD40 antibodies useful in the methods of the present invention are free of significant agonist activity as noted above when bound to a CD40 antigen on a human cell. In one embodiment of the invention, the antagonist anti-CD40 antibody is free of significant agonist activity in one B cell response. In another embodiment of the invention, the antagonist anti-CD40 antibody is free of significant agonist activity in assays of more than one B cell response (e.g., proliferation and differentiation, or proliferation, differentiation, and antibody production).

As used herein "anti-CD40 antibody" encompasses any antibody that specifically recognizes the CD40 cell surface antigen, including polyclonal antibodies, monoclonal antibodies, single-chain antibodies, and fragments thereof such as Fab, F(ab')₂, Fᵥ, and other fragments which retain the antigen binding function of the parent anti-CD40 antibody. Of particular interest to the methods of the present invention are the binding properties exhibited by the CHIR-5.9 and CHIR-12.12 monoclonal antibodies described above. Such antibodies include, but are not limited to the following: (1) the monoclonal antibodies produced by the hybridoma cell lines designated 131.2F8.5.9 (referred to herein as the cell line 5.9) and 153.8E2.D10.D6.12.12 (referred to herein as the cell line 12.12), deposited with the ATCC as Patent Deposit No. PTA-5542 and Patent Deposit No. PTA-5543, respectively; (2) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5; (3) a monoclonal antibody comprising an amino acid sequence selected from the group consisting of the sequence shown in SEQ ID NO:6, the sequence shown in SEQ ID NO:7, the sequence shown in SEQ ID NO:8, both the sequences shown in SEQ ID NO:6 and SEQ ID NO:7, and both the sequences shown in SEQ ID NO:6 and SEQ ID NO:8; (4) a monoclonal antibody having an amino acid sequence encoded by a nucleic acid molecule comprising a nucleotide sequence selected from the group consisting of the nucleotide sequence shown in SEQ ID NO:1, the nucleotide sequence shown in SEQ ID NO:3, and both the sequences shown in SEQ ID NO:1 and SEQ ID NO:3; (5) a monoclonal antibody that binds to an epitope capable of binding the monoclonal antibody produced by the hybridoma cell line 5.9 or the hybridoma cell line 12.12; (6) a monoclonal antibody that binds to an epitope comprising residues 82-87 of the amino acid sequence shown in SEQ ID NO:10 or SEQ ID NO:12; (7) a monoclonal antibody that competes with the monoclonal antibody CHIR-5.9 or CHIR-12.12 in a competitive binding assay; and (8) a monoclonal antibody that is an antigen-binding fragment of the CHIR-12.12 or CHIR-5.9 monoclonal antibody or the foregoing monoclonal antibodies in preceding items (1)-(7), where the fragment retains the capability of specifically binding to the human CD40 antigen. Those skilled in the art recognize that the antagonist antibodies and antigen-binding fragments of these antibodies disclosed herein include antibodies and antigen-binding fragments thereof that are produced recombinantly using methods well known in the art and described herein below, and include, for example, monoclonal antibodies CHIR-5.9 and CHIR-12.12 that have been recombinantly produced.

### Production of Antagonist Anti-CD40 Antibodies

The antagonist anti-CD40 antibodies for use in the methods of the present invention can be produced using any antibody production method known to those of skill in the art. Thus, polyclonal sera may be prepared by conventional methods. In general, a solution containing the CD40 antigen is first used to immunize a suitable animal, preferably a mouse, rat, rabbit, or goat. Rabbits or goats are preferred for the preparation of polyclonal sera due to the volume of serum obtainable, and the availability of labeled anti-rabbit and anti-goat antibodies.

Polyclonal sera can be prepared in a transgenic animal, preferably a mouse bearing human immunoglobulin loci. In a preferred embodiment, Sf9 cells expressing CD40 are used as the immunogen. Immunization can also be performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization. Polyclonal antisera are obtained by bleeding the immunized animal into a glass or plastic container, incubating the blood at 25°C for one hour, followed by incubating at 4°C for 2-18 hours. The serum is recovered by centrifugation (e.g., 1,000 x g for 10 minutes). About 20-50 ml per bleed may be obtained from rabbits.

Production of the Sf 9 *(Spodoptera frugiperda)* cells is disclosed in U.S. Patent No. 6,004,552, incorporated herein by reference. Briefly, sequences encoding human CD40 were recombined into a baculovirus using transfer vectors. The plasmids were co-transfected with wild-type baculovirus DNA into Sf 9 cells. Recombinant baculovirus- infected Sf 9 cells were identified and clonally purified.

Preferably the antibody is monoclonal in nature. By "monoclonal antibody" is intended an antibody obtained from a population of substantially homogeneous antibodies, i.e., the individual antibodies comprising the population are identical except for possible naturally occurring mutations that may be present in minor amounts. The term is not limited regarding the species or source of the antibody. The term encompasses whole immunoglobulins as well as fragments such as Fab, F(ab')2, Fv, and others which retain the antigen binding function of the antibody. Monoclonal antibodies are highly specific, being directed against a single antigenic site, i.e., the CD40 cell surface antigen in the present invention. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256:495, or may be made by recombinant DNA methods (see, *e.g*., U.S. Patent No. 4,816,567). The "monoclonal antibodies" may also be isolated from phage antibody libraries using the techniques described in, for example, Clackson et al. (1991) Nature 352:624-628; Marks et al. (1991) J. Mol. Biol. 222:581-597; and U.S. Patent No. 5,514,548.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), nonlinear amino acid residues (referred to herein as "nonlinear epitopes"; these epitopes are not arranged sequentially), or both linear and nonlinear amino acid residues.

Monoclonal antibodies can be prepared using the method of Kohler et al. (1975) Nature 256:495-496, or a modification thereof. Typically, a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

Where the antagonist anti-CD40 antibodies of the invention are to be prepared using recombinant DNA methods, the DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells described herein serve as a preferred source of such DNA. Once isolated, the DNA may be placed into expression vectors, which are then transfected into host cells such as *E*. *coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al. (1993) Curr. Opinion in Immunol. 5:256 and Phickthun (1992) *Immunol. Revs.* 130:151. As an alternative to the use of hybridomas, antibody can be produced in a cell line such as a CHO cell line, as disclosed in U.S. Patent Nos. 5,545,403; 5,545,405; and 5,998,144. Briefly the cell line is transfected with vectors capable of expressing a light chain and a heavy chain, respectively. By transfecting the two proteins on separate vectors, chimeric antibodies can be produced. Another advantage is the correct glycosylation of the antibody.

In some embodiments, the antagonist anti-CD40 antibody, for example, the CHIR-12.12 or CHIR-5.9 antibody, or antigen-binding fragment thereof is produced in CHO cells using the GS gene expression system (Lonza Biologics, Portsmouth, New Hampshire), which uses glutamine synthetase as a marker. See, also U.S. Patent Nos. 5,122,464; 5,591,639; 5,658,759; 5,770,359; 5,827,739; 5,879,936; 5,891,693; and 5,981,216.

Monoclonal antibodies to CD40 are known in the art. See, for example, the sections dedicated to B-cell antigen in McMichael, ed. (1987; 1989) Leukocyte Typing III and IV (Oxford University Press, New York); U.S. Patent Nos. 5,674,492; 5,874,082; 5,677,165; 6,056,959; WO 00/63395; International Publication Nos. WO 02/28905 and WO 02/28904; Gordon et al. (1988) J. Immunol. 140:1425; Valle et al. (1989) Eur. J. Immunol. 19:1463; Clark et al. (1986) PNAS 83:4494; Paulie et al. (1989) J. Immunol. 142:590; Gordon et al. (1987) Eur. J. Immunol. 17:1535; Jabara et al. (1990) J. Exp. Med. 172:1861; Zhang et al. (1991) J. Immunol. 146:1836; Gascan et al. (1991) J. Immunol. 147:8; Banchereau et al. (1991) Clin. Immunol. Spectrum 3:8; and Banchereau et al. (1991) Science 251:70. Of particular interest to the present invention are the antagonist anti-CD40 antibodies disclosed herein that share the binding characteristics of the monoclonal antibodies CHIR-5.9 and CHIR-12.12 described above.

The term "CD40-antigen epitope" as used herein refers to a molecule that is capable of immunoreactivity with the anti-CD40 monoclonal antibodies of this invention, excluding the CD40 antigen itself. CD40-antigen epitopes may comprise proteins, protein fragments, peptides, carbohydrates, lipids, and other molecules, but for the purposes of the present invention are most commonly proteins, short oligopeptides, oligopeptide mimics (i e, organic compounds which mimic the antibody binding properties of the CD40 antigen), or combinations thereof. Suitable oligopeptide mimics are described, inter alia, in PCT application US 91/04282.

Additionally, the term "anti-CD40 antibody" as used herein encompasses chimeric anti-CD40 antibodies; such chimeric anti-CD40 antibodies for use in the methods of the invention have the binding characteristics of the CHIR-5.9 and CHIR-12.12 monoclonal antibodies described herein. By "chimeric" antibodies is intended antibodies that are most preferably derived using recombinant deoxyribonucleic acid techniques and which comprise both human (including immunologically "related" species, e.g., chimpanzee) and non-human components. Thus, the constant region of the chimeric antibody is most preferably substantially identical to the constant region of a natural human antibody; the variable region of the chimeric antibody is most preferably derived from a non-human source and has the desired antigenic specificity to the CD40 cell-surface antigen. The non-human source can be any vertebrate source that can be used to generate antibodies to a human CD40 cell-surface antigen or material comprising a human CD40 cell-surface antigen. Such non-human sources include, but are not limited to, rodents (e.g., rabbit, rat, mouse, etc.; see, for example, U.S. Patent No. 4,816,567, herein incorporated by reference) and non-human primates (e.g., Old World Monkey, Ape, etc.; see, for example, U.S. Patent Nos. 5,750,105 and 5,756,096; herein incorporated by reference). As used herein, the phrase "immunologically active" when used in reference to chimeric anti-CD40 antibodies means a chimeric antibody that binds human CD40.

Chimeric and humanized anti-CD40 antibodies are also encompassed by the term anti-CD40 antibody as used herein. Chimeric antibodies comprise segments of antibodies derived from different species. Rituxan® (IDEC Pharmaceuticals Corp., San Diego, California) is an example of a chimeric antibody with a murine variable region and a human constant region.

By "humanized" is intended forms of anti-CD40 antibodies that contain minimal sequence derived from non-human immunoglobulin sequences. For the most part, humanized antibodies are human immunoglobulins (recipient antibody) in which residues from a hypervariable region (also known as complementarity determining region or CDR) of the recipient are replaced by residues from a hypervariable region of a non-human species (donor antibody) such as mouse, rat, rabbit, or nonhuman primate having the desired specificity, affinity, and capacity. The phrase "complementarity determining region" refers to amino acid sequences which together define the binding affinity and specificity of the natural Fv region of a native immunoglobulin binding site. See, e.g., Chothia et al (1987) J. Mol. Biol. 196:901-917; Kabat et al (1991) U.S. Dept. of Health and Human Services, NIH Publication No. 91-3242). The phrase "constant region" refers to the portion of the antibody molecule that confers effector functions. In previous work directed towards producing non-immunogenic antibodies for use in therapy of human disease, mouse constant regions were substituted by human constant regions. The constant regions of the subject humanized antibodies were derived from human immunoglobulins. However, these humanized antibodies still elicited an unwanted and potentially dangerous immune response in humans and there was a loss of affinity. Humanized anti-CD40 antibodies for use in the methods of the present invention have binding characteristics similar to those exhibited by the CHIR-5.9 and CHIR-12.12 monoclonal antibodies described herein.

Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting rodent or mutant rodent CDRs or CDR sequences for the corresponding sequences of a human antibody. See also U.S. Patent Nos. 5,225,539; 5,585,089; 5,693,761; 5,693,762; 5,859,205. In some instances, residues within the framework regions of one or more variable regions of the human immunoglobulin are replaced by corresponding non-human residues (see, for example, U.S. Patent Nos. 5,585,089; 5,693,761; 5,693,762; and 6,180,370). Furthermore, humanized antibodies may comprise residues that are not found in the recipient antibody or in the donor antibody. These modifications are made to further refine antibody performance (e.g., to obtain desired affinity). In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. For further details see Jones et al. (1986) Nature 331:522-525; Riechmann et al. (1988) Nature 332:323-329; and Presta (1992) Curr. Op. Struct. Biol. 2:593-596. Accordingly, such "humanized" antibodies may include antibodies wherein substantially less than an intact human variable domain has been substituted by the corresponding sequence from a non-human species. In practice, humanized antibodies are typically human antibodies in which some CDR residues and possibly some framework residues are substituted by residues from analogous sites in rodent antibodies. See, for example, U.S. Patent Nos. 5,225,539; 5,585,089; 5,693,761; 5,693,762; 5,859,205. See also U.S. Patent No. 6,180,370, and International Publication No. WO 01/27160, where humanized antibodies and techniques for producing humanized antibodies having improved affinity for a predetermined antigen are disclosed.

Also encompassed by the term anti-CD40 antibodies are xenogeneic or modified anti-CD40 antibodies produced in a non-human mammalian host, more particularly a transgenic mouse, characterized by inactivated endogenous immunoglobulin (Ig) loci. In such transgenic animals, competent endogenous genes for the expression of light and heavy subunits of host immunoglobulins are rendered non-functional and substituted with the analogous human immunoglobulin loci. These transgenic animals produce human antibodies in the substantial absence of light or heavy host immunoglobulin subunits. See, for example, U.S. Patent Nos. 5,877,397 and 5,939,598.

Preferably, fully human antibodies to CD40 are obtained by immunizing transgenic mice. One such mouse is obtained using XenoMouse^{®} technology (Abgenix; Fremont, California), and is disclosed in U.S. Patent Nos. 6,075,181, 6,091,001, and 6,114,598. To produce the antibodies disclosed herein, mice transgenic for the human Ig G₁ heavy chain locus and the human K light chain locus were immunized with Sf 9 cells expressing human CD40. Mice can also be transgenic for other isotypes. Fully human antibodies useful in the methods of the present invention are characterized by binding properties similar to those exhibited by the CHIR-5.9 and CHIR-12.12 monoclonal antibodies disclosed herein.

Fragments of the anti-CD40 antibodies are suitable for use in the methods of the invention so long as they retain the desired affinity of the full-length antibody. Thus, a fragment of an anti-CD40 antibody will retain the ability to bind to the CD40 cell-surface antigen expressed on a human cell, particularly the CD40 cell surface antigen on CD40-expressing carcinoma cells of solid tumors. Such fragments are characterized by properties similar to the corresponding full-length antagonist anti-CD40 antibody, that is, the fragments will specifically bind a human CD40 antigen expressed on the surface of a human cell, and are free of significant agonist activity but exhibit antagonist activity when bound to a CD40 antigen on a human CD40-expressing cell. Such fragments are referred to herein as "antigen-binding" fragments. Suitable antigen-binding fragments of an antibody comprise a portion of a full-length antibody, generally the antigen-binding or variable region thereof Examples of antibody fragments include, but are not limited to, Fab, F(ab')₂, and Fv fragments and single-chain antibody molecules. By "Fab" is intended a monovalent antigen-binding fragment of an immunoglobulin that is composed of the light chain and part of the heavy chain. By F(ab')₂ is intended a bivalent antigen-binding fragment of an immunoglobulin that contains both light chains and part of both heavy chains. By "single-chain Fv" or "sFv" antibody fragments is intended fragments comprising the V_{H} and V_{L} domains of an antibody, wherein these domains are present in a single polypeptide chain. See, for example, U.S. Patent Nos. 4,946,778, 5,260,203, 5,455,030, and 5,856,456. Generally, the Fv polypeptide further comprises a polypeptide linker between the V_{H} and V_{L} domains that enables the sFv to form the desired structure for antigen binding. For a review of sFv see Pluckthun (1994) in The Pharmacology of Monoclonal Antibodies, Vol. 113, ed. Rosenburg and Moore (Springer-Verlag, New York), pp. 269-315. Antigen-binding fragments of the antagonist anti-CD40 antibodies disclosed herein can also be conjugated to a cytotoxin to effect killing of the target cancer cells, as described herein below.

Antibodies or antibody fragments can be isolated from antibody phage libraries generated using the techniques described in, for example, McCafferty et al. (1990) Nature 348:552-554 (1990) and U.S. Patent No. 5,514,548. Clackson et al. (1991) Nature 352:624-628 and Marks et al. (1991) J. Mol. Biol. 222:581-597 describe the isolation of murine and human antibodies, respectively, using phage libraries. Subsequent publications describe the production of high affinity (nM range) human antibodies by chain shuffling (Marks et al. (1992) Bio/Technology 10:779-783), as well as combinatorial infection and *in vivo* recombination as a strategy for constructing very large phage libraries (Waterhouse et al. (1993) Nucleic. Acids Res. 21:2265-2266). Thus, these techniques are viable alternatives to traditional monoclonal antibody hybridoma techniques for isolation of monoclonal antibodies.

Various techniques have been developed for the production of antibody fragments. Traditionally, these fragments were derived *via* proteolytic digestion of intact antibodies (see, e.g., Morimoto et al. (1992) Journal of Biochemical and Biophysical Methods 24:107-117 (1992) and Brennan et al. (1985) Science 229:81). However, these fragments can now be produced directly by recombinant host cells. For example, the antibody fragments can be isolated from the antibody phage libraries discussed above. Alternatively, Fab'-SH fragments can be directly recovered from E. *coli* and chemically coupled to form F(ab')₂ fragments (Carter et al. (1992) Bio/Technology 10:163-167). According to another approach, F(ab')₂ fragments can be isolated directly from recombinant host cell culture. Other techniques for the production of antibody fragments will be apparent to the skilled practitioner.

Antagonist anti-CD40 antibodies useful in the methods of the present invention include the CHIR-5.9 and CHIR-12.12 monoclonal antibodies disclosed herein as well as antibodies differing from this antibody but retaining the CDRs; and antibodies with one or more amino acid addition(s), deletion(s), or substitution(s), wherein the antagonist activity is measured by inhibition of B-cell proliferation and/or differentiation. The invention also encompasses de-immunized antagonist anti-CD40 antibodies, which can be produced as described in, for example, International Publication Nos. WO 98/52976 and WO 0034317. In this manner, residues within the antagonist anti-CD40 antibodies of the invention are modified so as to render the antibodies non- or less immunogenic to humans while retaining their antagonist activity toward human CD40-expressing cells, wherein such activity is measured by assays noted elsewhere herein. Also included within the scope of the claims are fusion proteins comprising an antagonist anti-CD40 antibody of the invention, or a fragment thereof, which fusion proteins can be synthesized or expressed from corresponding polynucleotide vectors, as is known in the art. Such fusion proteins are described with reference to conjugation of antibodies as noted below.

The antibodies of the present invention can have sequence variations produced using methods described in, for example, Patent Publication Nos. EP 0 983 303 A1, WO 00/34317, and WO 98/52976. For example, it has been shown that sequences within the CDR can cause an antibody to bind to MHC Class II and trigger an unwanted helper T-cell response. A conservative substitution can allow the antibody to retain binding activity yet lose its ability to trigger an unwanted T-cell response. Any such conservative or non-conservative substitutions can be made using art-recognized methods, such as those noted elsewhere herein, and the resulting antibodies will fall within the scope of the invention. The variant antibodies can be routinely tested for antagonist activity, affinity, and specificity using methods described herein.

An antibody produced by any of the methods described above, or any other method not disclosed herein, will fall within the scope of the invention if it possesses at least one of the following biological activities: inhibition of immunoglobulin secretion by normal human peripheral B cells stimulated by T cells; inhibition of proliferation of normal human peripheral B cells stimulated by Jurkat T cells; inhibition of proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted below. These assays can be performed as described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

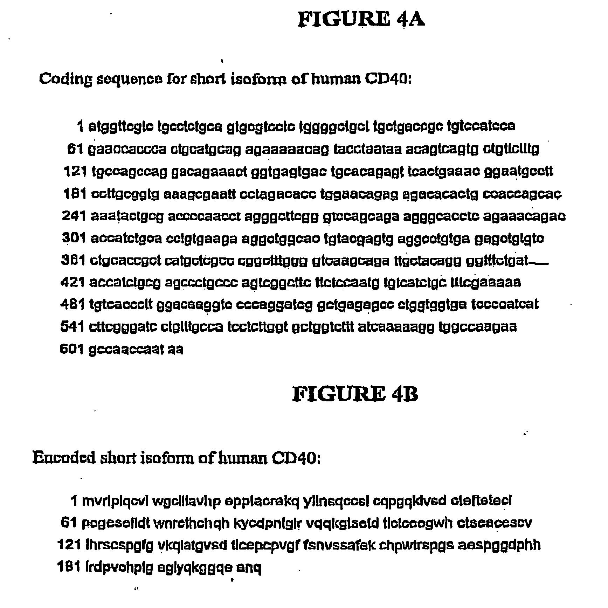

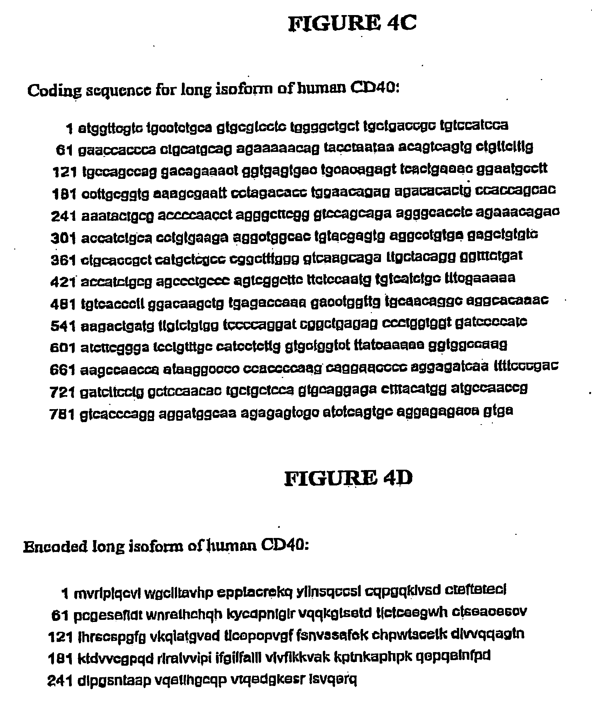

A representative assay to detect antagonistic anti-CD40 antibodies specific to the CD40-antigen epitopes identified herein is a "competitive binding assay." Competitive binding assays are serological assays in which unknowns are detected and quantitated by their ability to inhibit the binding of a labeled known ligand to its specific antibody. This is also referred to as a competitive inhibition assay. In a representative competitive binding assay, labeled CD40 polypeptide is precipitated by candidate antibodies in a sample, for example, in combination with monoclonal antibodies raised against one or more epitopes of the monoclonal antibodies of the invention. Anti-CD40 antibodies that specifically react with an epitope of interest can be identified by screening a series of antibodies prepared against a CD40 protein or fragment of the protein comprising the particular epitope of the CD40 protein of interest. For example, for human CD40, epitopes of interest include epitopes comprising linear and/or nonlinear amino acid residues of the short isoform of human CD40 (see GenBank Accession No. NP_690593) set forth in Figure 4B (SEQ ID NO: 10), encoded by the sequence set forth in Figure 4A (SEQ ID NO:9; see also GenBank Accession No. NM_152854), or of the long isoform of human CD40 (see GenBank Accession Nos. CAA43045 and NP_001241) set forth in Figure 4D (SEQ ID NO:12), encoded by the sequence set forth in Figure 4C (SEQ ID NO:11; see GenBank Accession Nos. X60592 and NM_001250). Alternatively, competitive binding assays with previously identified suitable antagonist anti-CD40 antibodies could be used to select monoclonal antibodies comparable to the previously identified antibodies.

Antibodies employed in such immunoassays may be labeled or unlabeled. Unlabeled antibodies may be employed in agglutination; labeled antibodies may be employed in a wide variety of assays, employing a wide variety of labels. Detection of the formation of an antibody-antigen complex between an anti-CD40 antibody and an epitope of interest can be facilitated by attaching a detectable substance to the antibody. Suitable detection means include the use of labels such as radionuclides, enzymes, coenzymes, fluorescers, chemiluminescers, chromogens, enzyme substrates or co-factors, enzyme inhibitors, prosthetic group complexes, free radicals, particles, dyes, and the like. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material is luminol; examples of bioluminescent materials include luciferase, luciferin, and aequorin; and examples of suitable radioactive material include ¹²⁵I, ¹³¹I, ³⁵S, or ³H. Such labeled reagents may be used in a variety of well-known assays, such as radioimmunoassays, enzyme immunoassays, e.g., ELISA, fluorescent immunoassays, and the like. See for example, U.S. Patent Nos. 3,766,162; 3,791,932; 3,817,837; and 4,233,402.

Any of the previously described antagonist anti-CD40 antibodies or antibody fragments thereof may be conjugated prior to use in the methods of the present invention. Methods for producing conjugated antibodies are known in the art. Thus, the anti-CD40 antibody may be labeled using an indirect labeling or indirect labeling approach. By "indirect labeling" or "indirect labeling approach" is intended that a chelating agent is covalently attached to an antibody and at least one radionuclide is inserted into the chelating agent. See, for example, the chelating agents and radionuclides described in Srivagtava and Mease (1991) Nucl. Med. Bio. 18:589-603, herein incorporated by reference. Suitable labels include fluorophores, chromophores, radioactive atoms (particularly ³²P and ¹²⁵I), electron-dense reagents, enzymes, and ligands having specific binding partners. Enzymes are typically detected by their activity. For example, horseradish peroxidase is usually detected by its ability to convert 3,3 ',5,5 '-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefore. Other specific binding partners include biotin and avidin or streptavidin, Ig G and protein A, and the numerous receptor-ligand couples known in the art. It should be understood that the above description is not meant to categorize the various labels into distinct classes, as the same label may serve in several different modes. For example, ¹²⁵I may serve as a radioactive label or as an electron-dense reagent. HRP may serve as enzyme or as antigen for a mAb. Further, one may combine various labels for desired effect. For example, mAbs and avidin also require labels in the practice of this invention: thus, one might label a mAb with biotin, and detect its presence with avidin labeled with ¹²⁵I, or with an anti-biotin mAb labeled with HRP. Other permutations and possibilities will be readily apparent to those of ordinary skill in the art, and are considered as equivalents within the scope of the instant invention.

Alternatively, the anti-CD40 antibody may be labeled using "direct labeling" or a "direct labeling approach, " where a radionuclide is covalently attached directly to an antibody (typically via an amino acid residue). Preferred radionuclides are provided in Srivagtava and Mease (1991) *supra.* The indirect labeling approach is particularly preferred. See also, for example, International Publication Nos. WO 00/52031 and WO 00/52473, where a linker is used to attach a radioactive label to antibodies; and the labeled forms of anti-CD40 antibodies described in U.S. Patent No. 6,015,542.

Further, an antibody (or fragment thereof) may be conjugated to a therapeutic moiety such as a cytotoxin, a therapeutic agent, or a radioactive metal ion or radioisotope. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). Radioisotopes include, but are not limited to, I-131, I-123, I-125, Y-90, Re-188, Re-186, At-211, Cu-67, Bi-212, Bi-213, Pd-109, Tc-99, In-111, and the like. The conjugates of the invention can be used for modifying a given biological response; the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, interferon-alpha, interferon-beta, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known. See, for example, Arnon et al. (1985) "Monoclonal Antibodies for Immunotargeting of Drugs in Cancer Therapy," in Monoclonal Antibodies and Cancer Therapy, ed. Reisfeld et al. (Alan R. Liss, Inc.), pp. 243-256; ed. Hellstrom et al. (1987) "Antibodies for Drug Delivery," in Controlled Drug Delivery, ed. Robinson et al. (2d ed; Marcel Dekker, Inc.), pp. 623-653; Thorpe (1985) "Antibody Carriers of Cytotoxic Agents in Cancer Therapy: A Review, " in Monoclonal Antibodies '84: Biological and Clinical Applications, ed. Pinchera et al. pp. 475-506 (Editrice Kurtis, Milano, Italy, 1985); "Analysis, Results, and Future Prospective of the Therapeutic Use of Radiolabeled Antibody in Cancer Therapy, " in Monoclonal Antibodies for Cancer Detection and Therapy, ed. Baldwin et al. (Academic Press, New York, 1985), pp. 303-316; and Thorpe et al. (1982) Immunol. Rev. 62:119-158.

Alternatively, an antibody can be conjugated to a second antibody to form an antibody heteroconjugate as described in U.S. Patent No. 4,676,980. In addition, linkers maybe used between the labels and the antibodies of the invention (see U.S. Patent No. 4,831,175). Antibodies or, antigen-binding fragments thereof may be directly labeled with radioactive iodine, indium, yttrium, or other radioactive particle known in the art (U.S. Patent No. 5,595,721). Treatment may consist of a combination of treatment with conjugated and nonconjugated antibodies administered simultaneously or subsequently (WO 00/52031 and WO 00/52473).

### Variants of Antagonist Anti-CD40 Antibodies

Suitable biologically active variants of the antagonist anti-CD40 antibodies can be used in the methods of the present invention. Such variants will retain the desired binding properties of the parent antagonist anti-CD40 antibody. Methods for making antibody variants are generally available in the art.

For example, amino acid sequence variants of an antagonist anti-CD40 antibody, for example, the CHIR-5.9 or CHIR-12.12 monoclonal antibody described herein, can be prepared by mutations in the cloned DNA sequence encoding the antibody of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein. Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.). Conservative substitutions, such as exchanging one amino acid with another having similar properties, may be preferred. Examples of conservative substitutions include, but are not limited to, Gly⇔Ala, Val⇔Ile⇔Leu, Asp⇔4Glu, Lysr⇔Arg, Asn⇔Gln, and Phe⇔Trp⇔Tyr.

In constructing variants of the antagonist anti-CD40 antibody polypeptide of interest, modifications are made such that variants continue to possess the desired activity, i.e., similar binding affinity and are capable of specifically binding to a human CD40 antigen expressed on the surface of a human cell, and being free of significant agonist activity but exhibiting antagonist activity when bound to a CD40 antigen on a human CD40-expressing cell. Obviously, any mutations made in the DNA encoding the variant polypeptide must not place the sequence out of reading frame and preferably will not create complementary regions that could produce secondary mRNA structure. See EP Patent Application Publication No. 75,444.

In addition, the constant region of an antagonist anti-CD40 antibody can be mutated to alter effector function in a number of ways. For example, see U.S. Patent No. 6,737,056B1 and U.S. Patent Application Publication No. 2004/0132101A1, which disclose Fc mutations that optimize antibody binding to Fc receptors.

Preferably, variants of a reference antagonist anti-CD40 antibody have amino acid sequences that have at least 70% or 75% sequence identity, preferably at least 80% or 85% sequence identity, more preferably at least 90%, 91%, 92%, 93%, 94% or 95% sequence identity to the amino acid sequence for the reference antagonist anti-CD40 antibody molecule, for example, the CHIR-5.9 or CHIR-12.12 monoclonal antibody described herein, or to a shorter portion of the reference antibody molecule. More preferably, the molecules share at least 96%, 97%, 98% or 99% sequence identity. For purposes of the present invention, percent sequence identity is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference antagonist anti-CD40 antibody by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

The precise chemical structure of a polypeptide capable of specifically binding CD40 and retaining antagonist activity, particularly when bound to CD40 antigen on malignant B cells or CD40-expressing cells of a solid tumor, depends on a number of factors. As ionizable amino and carboxyl groups are present in the molecule, a particular polypeptide may be obtained as an acidic or basic salt, or in neutral form. All such preparations that retain their biological activity when placed in suitable environmental conditions are included in the definition of antagonist anti-CD40 antibodies as used herein. Further, the primary amino acid sequence of the polypeptide may be augmented by derivatization using sugar moieties (glycosylation) or by other supplementary molecules such as lipids, phosphate, acetyl groups and the like. It may also be augmented by conjugation with saccharides. Certain aspects of such augmentation are accomplished through post-translational processing systems of the producing host; other such modifications may be introduced *in vitro.* In any event, such modifications are included in the definition of an anti-CD40 antibody used herein so long as the antagonist properties of the anti-CD40 antibody are not destroyed. It is expected that such modifications may quantitatively or qualitatively affect the activity, either by enhancing or diminishing the activity of the polypeptide, in the various assays. Further, individual amino acid residues in the chain may be modified by oxidation, reduction, or other derivatization, and the polypeptide may be cleaved to obtain fragments that retain activity. Such alterations that do not destroy antagonist activity do not remove the polypeptide sequence from the definition of anti-CD40 antibodies of interest as used herein.

The art provides substantial guidance regarding the preparation and use of polypeptide variants. In preparing the anti-CD40 antibody variants, one of skill in the art can readily determine which modifications to the native protein nucleotide or amino acid sequence will result in a variant that is suitable for use as a therapeutically active component of a pharmaceutical composition used in the methods of the present invention.

### Methods of Therapy Using the Antagonist Anti-CD40 Antibodies of the Invention

Methods of the disclosure are directed to the use of antagonist anti-CD40 antibodies to treat subjects (i.e., patients) having solid tumors that comprise cells expressing the CD40 cell-surface antigen. By "CD40-expressing carcinoma cell" is intended any malignant (i.e., neoplastic) or pre-malignant cell of a solid tumor that expresses the CD40 cell-surface antigen. Methods for detecting CD40 expression in cells are well known in the art and include, but are not limited to, PCR techniques, immunohistochemistry, flow cytometry, Western blot, ELISA, and the like. Solid tumors that can be treated using the methods of the present disclosure include, but are not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas).

"Treatment" is herein defined as the application or administration of an antagonist anti-CD40 antibody or antigen-binding fragment thereof to a subject, or application or administration of an antagonist anti-CD40 antibody or fragment thereof to an isolated tissue or cell line from a subject, where the subject has a solid tumor, a symptom associated with a solid tumor, or a predisposition toward development of a solid tumor, where the purpose is to cure, heal, alleviate, relieve, alter, remedy, ameliorate, improve, or affect the solid tumor, any associated symptoms of the solid tumor, or the predisposition toward development of the solid tumor. By "treatment" is also intended the application or administration of a pharmaceutical composition comprising the antagonist anti-CD40 antibodies or fragments thereof to a subject, or application or administration of a pharmaceutical composition comprising the anti-CD40 antibodies or fragments thereof to an isolated tissue or cell line from a subject, who has a solid tumor, a symptom associated with a solid tumor, or a predisposition toward development of the solid tumor, where the purpose is to cure, heal, alleviate, relieve, alter, remedy, ameliorate, improve, or affect the solid tumor, any associated symptoms of the solid tumor, or the predisposition toward development of the solid tumor.

By "anti-tumor activity" is intended a reduction in the rate of malignant CD40-expressing cell proliferation or accumulation, and hence a decline in growth rate of an existing tumor or in a tumor that arises during therapy, and/or destruction of existing neoplastic (tumor) cells or newly formed neoplastic cells, and hence a decrease in the overall size of a tumor during therapy. Therapy with at least one anti-CD40 antibody (or antigen-binding fragment thereof) causes a physiological response that is beneficial with respect to treatment of solid tumors in a human, where the solid tumors comprise CD40-expressing carcinoma cells. It is recognized that the methods of the disclosure may be useful in preventing further tumor outgrowths arising during therapy.

In accordance with the methods of the present disclosure, at least one antagonist anti-CD40 antibody (or antigen-binding fragment thereof) as defined elsewhere herein is used to promote a positive therapeutic response with respect to a solid tumor. By "positive therapeutic response" with respect to cancer treatment is intended an improvement in the disease in association with the anti-tumor activity of these antibodies or fragments thereof, and/or an improvement in the symptoms associated with the disease. That is, an anti-proliferative effect, the prevention of further tumor outgrowths, a reduction in tumor size, a reduction in the number of cancer cells, and/or a decrease in one or more symptoms mediated by stimulation of CD40-expressing cells can be observed. Thus, for example, an improvement in the disease may be characterized as a complete response. By "complete response" is intended an absence of clinically detectable disease with normalization of any previously abnormal radiographic studies, bone marrow, and cerebrospinal fluid (CSF). Such a response must persist for at least one month following treatment according to the methods of the disclosure. Alternatively, an improvement in the disease may be categorized as being a partial response. By "partial response" is intended at least about a 50% decrease in all measurable tumor burden (i.e., the number of tumor cells present in the subject) in the absence of new lesions and persisting for at least one month. Such a response is applicable to measurable tumors only.

Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bioluminescent imaging, for example, luciferase imaging, bone scan imaging, and tumor biopsy sampling including bone marrow aspiration (BMA). In addition to these positive therapeutic responses, the subject undergoing therapy with the antagonist anti-CD40 antibody or antigen-binding fragment thereof may experience the beneficial effect of an improvement in the symptoms associated with the disease.

By "therapeutically effective dose or amount" or "effective amount" is intended an amount of antagonist anti-CD40 antibody or antigen-binding fragment thereof that, when administered brings about a positive therapeutic response with respect to treatment of a patient with a solid tumor comprising CD40-expressing carcinoma cells. In some embodiments of the invention, a therapeutically effective dose of the anti-CD40 antibody or fragment thereof is in the range from about 0.01 mg/kg to about 40 mg/kg, from about 0.01 mg/kg to about 30 mg/kg, from about 0.1 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. It is recognized that the method of treatment may comprise a single administration of a therapeutically effective dose or multiple administrations of a therapeutically effective dose of the antagonist anti-CD40 antibody or antigen-binding fragment thereof.

A further embodiment of the disclosure is the use of antagonist anti-CD40 antibodies for diagnostic monitoring of protein levels in tissue as part of a clinical testing procedure, e.g., to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ³⁵S, or ³H.

In some preferred embodiments, the antagonist anti-CD40 antibodies of the invention, or antigen-binding fragments thereof, are administered in combination with at least one other cancer therapy, including, but not limited to, surgery, radiation therapy, chemotherapy, cytokine therapy, or other monoclonal antibody intended for use in treatment of the solid tumor of interest, where the additional cancer therapy is administered prior to, during, or subsequent to the anti-CD40 antibody therapy. Thus, where the combined therapies comprise administration of an anti-CD40 antibody or antigen-binding fragment thereof in combination with administration of another therapeutic agent, as with chemotherapy, cytokine therapy, or other monoclonal antibody, the methods of the disclosure encompass coadministration, using separate formulations or a single pharmaceutical formulation, and consecutive administration in either order, wherein preferably there is a time period where both (or all) active agents simultaneously exert their therapeutic activities. Where the methods of the present disclosure comprise combined therapeutic regimens, these therapies can be given simultaneously, i.e., the anti-CD40 antibody or antigen-binding fragment thereof is administered concurrently or within the same time frame as the other cancer therapy (i.e., the therapies are going on concurrently, but the anti-CD40 antibody or antigen-binding fragment thereof is not administered precisely at the same time as the other cancer therapy). Alternatively, the anti-CD40 antibody of the present invention or antigen-binding fragment thereof may also be administered prior to or subsequent to the other cancer therapy. Sequential administration of the different cancer therapies may be performed regardless of whether the treated subject responds to the first course of therapy to decrease the possibility of remission or relapse.

In some embodiments of the invention, the anti-CD40 antibodies described herein, or antigen-binding fragments thereof, are administered in combination with chemotherapy or cytokine therapy, wherein the antibody and the chemotherapeutic agent(s) or cytokine(s) may be administered sequentially, in either order, or simultaneously (i.e., concurrently or within the same time frame). Examples of suitable chemotherapeutic agents include, but are not limited to, CPT-11 (Irinotecan), which can be used, for example, in treating colorectal cancer and non-small cell lung cancer; gemcitabine, which can be used, for example, in treating lung cancer, breast cancer, and epithelial ovarian cancer; and other chemotherapeutic agents suitable for treatment of solid tumors. Cytokines of interest include, but are not limited to, alpha interferon, gamma interferon, interleukin-2 (IL-2), IL-12, IL-15, and IL-21, granulocyte macrophage colony stimulating factor (GM-CSF), granulocyte colony stimulating factor (G-CSF), or biologically active variants of these cytokines.

In other embodiments of the invention, the anti-CD40 antibodies described herein, or antigen-binding fragments thereof, are administered in combination with other monoclonal antibodies intended for treatment of the solid tumor. Thus, for example, where the subject is undergoing treatment for a breast cancer comprising CD40-expressing carcinoma cells, therapy could include administration of effective amounts of an antagonist anti-CD40 antibody described herein, or antigen-binding fragment thereof, in combination with administration of effective amounts of Herceptin® (Genentech, Inc., San Francisco, California), which targets the Her2 receptor protein on Her2+ breast cancer cells. Similarly, where the subject is undergoing treatment for colorectal cancer comprising CD40-expressing carcinoma cells, therapy could include administration of effective amounts of an antagonist anti-CD40 antibody described herein, or antigen-binding fragment thereof, in combination with administration of effective amounts of the humanized monoclonal antibody Avastin™ (also known as bevacizumab; Genentech, Inc., San Francisco, California), which binds to and inhibits vascular endothelial growth factor (VEGF), a protein that plays a critical role in tumor angiogenesis. Other examples of monoclonal antibodies intended for treatment of solid tumors that can be used in combination with the anti-CD40 antibodies of the present invention include, but are not limited to, anti-EGFR antibody targeting the epidermal growth factor receptor (for example, IMC-C225 (ImClone Systems, New York, New York) (see, for example, Mendelsohn and Baselga (2000) Oncogene 19:6550-6565 and Solbach et al. (2002) Int. J. Cancer 101:390-394); anti-IGF-1 receptor antibody, targeting the IGF-1 receptor protein (see, for example, Maloney et al. (2003) Cancer Res. 63:5073-5083 and Hailey et al. (2002) Mol. Cancer. Ther. 1:1349-1353; anti-MUC1 antibody, targeting the tumor-associated antigen MUC1; anti-α5β1, anti-αvβ5, and anti-αvβ3, targeting these respective integrins, which regulate cell adhesion and signaling processes involved in cell proliferation and survival (see, for example, Laidler et al. (2000) Acta Biochimica Polonica 47(4):1159-1170 and Cruet-Hennequart et al. (2003) Oncogene 22(11):1688-1702); anti-P-cadherin antibody, targeting this cadherin family member (see, for example, copending U.S. Patent Application 20030194406); and anti-VE-cadherin antibody, targeting angiogenic-related function of this endothelial cell-specific adhesion molecule (see, for example, Liao et al. (2002) Cancer Res. 62:2567-2575).

The anti-CD40 antibodies of the invention and the other monoclonal antibody can be administered sequentially, in either order, or simultaneously (i.e., concurrently or within the same time frame). Where more than one type of monoclonal antibody is administered, the methods of the present disclosure can further comprise exposure to radiation and/or chemotherapy as warranted for the cancer undergoing treatment and as recommended by the supervising medical practitioner.

### Pharmaceutical Formulations and Modes of Administration

Anti-CD40 antibodies for use in the methods of this invention are administered at a concentration that is therapeutically effective to prevent or treat solid tumors comprising CD40-expressing carcinoma cells, including ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas). To accomplish this goal, the antibodies may be formulated using a variety of acceptable excipients known in the art. Typically, the antibodies are administered by injection, either intravenously, intraperitoneally, or intratumorally. Methods to accomplish this administration are known to those of ordinary skill in the art. It may also be possible to obtain compositions which may be topically or orally administered, or which may be capable of transmission across mucous membranes.

Intravenous administration occurs preferably by infusion over a period of about 1 to about 10 hours, more preferably over about 1 to about 8 hours, even more preferably over about 2 to about 7 hours, still more preferably over about 4 to about 6 hours, depending upon the anti-CD40 antibody being administered. The initial infusion with the pharmaceutical composition may be given over a period of about 4 to about 6 hours with subsequent infusions delivered more quickly. Subsequent infusions may be administered over a period of about 1 to about 6 hours, including, for example, about 1 to about 4 hours, about 1 to about 3 hours, or about 1 to about 2 hours.

A pharmaceutical composition of the invention is formulated to be compatible with its intended route of administration. Examples of possible routes of administration include parenteral, (e.g., intravenous (IV), intramuscular (IM), intradermal, subcutaneous (SC), intraperitoneal, intratumoral, or infusion), oral and pulmonary (e.g., inhalation), nasal, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose, pH can be adjusted with acids or bases, such as hydrochloric acid or sodium hydroxide. The parenteral preparation can be enclosed in ampoules, disposable syringes, or multiple dose vials made of glass or plastic.

The anti-CD40 antibodies are typically provided by standard technique within a pharmaceutically acceptable buffer, for example, sterile saline, sterile buffered water, propylene glycol, combinations of the foregoing, etc. Methods for preparing parenterally administrable agents are described in Remington's Pharmaceutical Sciences (18th ed.; Mack Publishing Company, Eaton, Pennsylvania, 1990).

See also, for example, WO 98/56418, which describes stabilized antibody pharmaceutical formulations suitable for use in the methods of the present disclosure.

The amount of at least one anti-CD40 antibody or fragment thereof to be administered is readily determined by one of ordinary skill in the art without undue experimentation given the disclosure set forth herein. Factors influencing the mode of administration and the respective amount of at least one antagonist anti-CD40 antibody (or fragment thereof) include, but are not limited to, the particular type of tumor undergoing therapy, the severity of the disease, the history of the disease, and the age, height, weight, health, and physical condition of the individual undergoing therapy. Similarly, the amount of antagonist anti-CD40 antibody or fragment thereof to be administered will be dependent upon the mode of administration and whether the subject will undergo a single dose or multiple doses of this anti-tumor agent. Generally, a higher dosage of anti-CD40 antibody or fragment thereof is preferred with increasing weight of the patient undergoing therapy. The dose of anti-CD40 antibody or fragment thereof to be administered is in the range from about 0.003 mg/kg to about 50 mg/kg, preferably in the range of 0.01 mg/kg to about 40 mg/kg. Thus, for example, the dose can be 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg.

In another embodiment of the disclosure, the method comprises administration of multiple doses of antagonist anti-CD40 antibody or fragment thereof. The method may comprise administration of 1, 2, 3, 4, 5, 6, 7, 8, 9, 10, 15, 20, 25, 30, 35, 40, or more therapeutically effective doses of a pharmaceutical composition comprising an antagonist anti-CD40 antibody or fragment thereof. The frequency and duration of administration of multiple doses of the pharmaceutical compositions comprising anti-CD40 antibody or fragment thereof can be readily determined by one of skill in the art without undue experimentation given the disclosure set forth herein. Moreover, treatment of a subject with a therapeutically effective amount of an antibody can include a single treatment or, preferably, can include a series of treatments. In a preferred example, a subject is treated with antagonist anti-CD40 antibody or antigen-binding fragment thereof in the range of between about 0.1 to 20 mg/kg body weight, once per week for between about 1 to 10 weeks, preferably between about 2 to 8 weeks, more preferably between about 3 to 7 weeks, and even more preferably for about 4, 5, or 6 weeks. Treatment may occur annually to prevent relapse or upon indication of relapse. It will also be appreciated that the effective dosage of antibody or antigen-binding fragment thereof used for treatment may increase or decrease over the course of a particular treatment. Changes in dosage may result and become apparent from the results of diagnostic assays as described herein. Thus, in one embodiment, the dosing regimen includes a first administration of a therapeutically effective dose of at least one anti-CD40 antibody or fragment thereof on days 1, 7, 14, and 21 of a treatment period. In another embodiment, the dosing regimen includes a first administration of a therapeutically effective dose of at least one anti-CD40 antibody or fragment thereof on days 1, 2, 3, 4, 5, 6, and 7 of a week in a treatment period. Further embodiments include a dosing regimen having a first administration of a therapeutically effective dose of at least one anti-CD40 antibody or fragment thereof on days 1, 3, 5, and 7 of a week in a treatment period; a dosing regimen including a first administration of a therapeutically effective dose of at least one anti-CD40 antibody or fragment thereof on days 1 and 3 of a week in a treatment period; and a preferred dosing regimen including a first administration of a therapeutically effective dose of at least one anti-CD40 antibody or fragment thereof on day 1 of a week in a treatment period. The treatment period may comprise 1 week, 2 weeks, 3 weeks, a month, 3 months, 6 months, or a year. Treatment periods may be subsequent or separated from each other by a day, a week, 2 weeks, a month, 3 months, 6 months, or a year.

In some embodiments, the therapeutically effective doses of antagonist anti-CD40 antibody or antigen-binding fragment thereof ranges from about 0.003 mg/kg to about 50 mg/kg, from about 0.01 mg/kg to about 40 mg/kg, from about 0.01 mg/kg to about 30 mg/kg, from about 0.1 mg/kg to about 30 mg/kg, from about 0.5 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. Thus, for example, the dose of any one antagonist anti-CD40 antibody or antigen-binding fragment thereof, for example the anti-CD40 monoclonal antibody CHIR-12.12 or CHIR-5.9 or antigen-binding fragment thereof, can be 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg. The same therapeutically effective dose of an antagonist anti-CD40 antibody or antigen-binding fragment thereof can be administered throughout each week of antibody dosing. Alternatively, different therapeutically effective doses of an antagonist anti-CD40 antibody or antigen-binding fragment thereof can be used over the course of a treatment period.

In other embodiments, the initial therapeutically effective dose of an antagonist anti-CD40 antibody or antigen-binding fragment thereof as defined elsewhere herein can be in the lower dosing range (i.e., about 0.003 mg/kg to about 20 mg/kg) with subsequent doses falling within the higher dosing range (i.e., from about 20 mg/kg to about 50 mg/kg).

In alternative embodiments, the initial therapeutically effective dose of an antagonist anti-CD40 antibody or antigen-binding fragment thereof as defined elsewhere herein can be in the upper dosing range (i.e., about 20 mg/kg to about 50 mg/kg) with subsequent doses falling within the lower dosing range (i.e., 0.003 mg/kg to about 20 mg/kg). Thus, in one embodiment, the initial therapeutically effective dose of the antagonist anti-CD40 antibody or antigen-binding fragment thereof is about 20 mg/kg to about 35 mg/kg, including about 20 mg/kg, about 25 mg/kg, about 30 mg/kg, and about 35 mg/kg, and subsequent therapeutically effective doses of the antagonist anti-CD40 antibody or antigen binding fragment thereof are about 5 mg/kg to about 15 mg/kg, including about 5 mg/kg, 8 mg/kg, 10 mg/kg, 12 mg/kg, and about 15 mg/kg.

In some embodiments of the disclosure, antagonist anti-CD40 antibody therapy is initiated by administering a "loading dose" of the antibody or antigen-binding fragment thereof to the subject in need of antagonist anti-CD40 antibody therapy. By "loading dose" is intended an initial dose of the antagonist anti-CD40 antibody or antigen-binding fragment thereof that is administered to the subject, where the dose of the antibody or antigen-binding fragment thereof administered falls within the higher dosing range (i.e., from about 20 mg/kg to about 50 mg/kg). The "loading dose" can be administered as a single administration, for example, a single infusion where the antibody or antigen-binding fragment thereof is administered IV, or as multiple administrations, for example, multiple infusions where the antibody or antigen-binding fragment thereof is administered IV, so long as the complete "loading dose" is administered within about a 24-hour period. Following administration of the "loading dose," the subject is then administered one or more additional therapeutically effective doses of the antagonist anti-CD40 antibody or antigen-binding fragment thereof Subsequent therapeutically effective doses can be administered, for example, according to a weekly dosing schedule, or once every two weeks, once every three weeks, or once every four weeks. In such embodiments, the subsequent therapeutically effective doses generally fall within the lower dosing range (i.e., 0.003 mg/kg to about 20 mg/kg).

Alternatively, in some embodiments, following the "loading dose, " the subsequent therapeutically effective doses of the antagonist anti-CD40 antibody or antigen-binding fragment thereof are administered according to a "maintenance schedule," wherein the therapeutically effective dose of the antibody or antigen-binding fragment thereof is administered once a month, once every 6 weeks, once every two months, once every 10 weeks, once every three months, once every 14 weeks, once every four months, once every 18 weeks, once every five months, once every 22 weeks, once every six months, once every 7 months, once every 8 months, once every 9 months, once every 10 months, once every 11 months, or once every 12 months. In such embodiments, the therapeutically effective doses of the antagonist anti-CD40 antibody or antigen-binding fragment thereof fall within the lower dosing range (i.e., 0.003 mg/kg to about 20 mg/kg), particularly when the subsequent doses are administered at more frequent intervals, for example, once every two weeks to once every month, or within the higher dosing range (i.e., from about 20 mg/kg to about 50 mg/kg), particularly when the subsequent doses are administered at less frequent intervals, for example, where subsequent doses are administered about one month to about 12 months apart.

The antagonist anti-CD40 antibodies present in the pharmaceutical compositions described herein for use in the methods of the disclosure may be native or obtained by recombinant techniques, and may be from any source, including mammalian sources such as, e.g., mouse, rat, rabbit, primate, pig, and human. Preferably such polypeptides are derived from a human source, and more preferably are recombinant, human proteins from hybridoma cell lines.

The pharmaceutical compositions useful in the methods of the invention may comprise biologically active variants of the antagonist anti-CD40 antibodies of the invention. Such variants should retain the desired biological activity of the native polypeptide such that the pharmaceutical composition comprising the variant polypeptide has the same therapeutic effect as the pharmaceutical composition comprising the native polypeptide when administered to a subject. That is, the variant anti-CD40 antibody will serve as a therapeutically active component in the pharmaceutical composition in a manner similar to that observed for the native antagonist antibody, for example CHIR-5.9 or CHIR-12.12 as expressed by the hybridoma cell line 5.9 or 12.12, respectively. Methods are available in the art for determining whether a variant anti-CD40 antibody retains the desired biological activity, and hence serves as a therapeutically active component in the pharmaceutical composition. Biological activity of antibody variants can be measured using assays specifically designed for measuring activity of the native antagonist antibody, including assays described in the present invention.

Any pharmaceutical composition comprising an antagonist anti-CD40 antibody having the binding properties described herein as the therapeutically active component can be used in the methods of the disclosure. Thus liquid, lyophilized, or spray-dried compositions comprising one or more of the antagonist anti-CD40 antibodies of the invention may be prepared as an aqueous or nonaqueous solution or suspension for subsequent administration to a subject in accordance with the methods of the disclosure. Each of these compositions will comprise at least one of the antagonist anti-CD40 antibodies of the present invention as a therapeutically or prophylactically active component. By "therapeutically or prophylactically active component" is intended the anti-CD40 antibody is specifically incorporated into the composition to bring about a desired therapeutic or prophylactic response with regard to treatment, prevention, or diagnosis of a solid tumor within a subject when the pharmaceutical composition is administered to that subject. Preferably the pharmaceutical compositions comprise appropriate stabilizing agents, bulking agents, or both to minimize problems associated with loss of protein stability and biological activity during preparation and storage.

Formulants may be added to pharmaceutical compositions comprising an antagonist anti-CD40 antibody of the invention. These formulants may include, but are not limited to, oils, polymers, vitamins, carbohydrates, amine acids, salts, buffers, albumin, surfactants, or bulking agents. Preferably carbohydrates include sugar or sugar alcohols such as mono-, di-, or polysaccharides, or water soluble glucans. The saccharides or glucans can include fructose, glucose, mannose, sorbose, xylose, maltose, sucrose, dextran, pullulan, dextrin, α and β cyclodextrin, soluble starch, hydroxyethyl starch, and carboxymethylcellulose, or mixtures thereof. "Sugar alcohol" is defined as a C₄ to C₈ hydrocarbon having a hydroxyl group and includes galactitol, inositol, mannitol, xylitol, sorbitol, glycerol, and arabitol. These sugars or sugar alcohols may be used individually or in combination. The sugar or sugar alcohol concentration is between 1.0% and 7% w/v., more preferably between 2.0% and 6.0% w/v. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546. Preferred polymers are polyoxyethylated polyols and polyethylene glycol (PEG). PEG is soluble in water at room temperature and has the general formula: R(O--CH₂ --CH₂)ₙ O--R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1,000 and 40,000, more preferably between 2,000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water-soluble polyoxyethylated polyols are also useful in the present invention. They include polyoxyethylated sorbitol, polyoxyethylated glucose, polyoxyethylated glycerol (POG), and the like. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG The structure for POG is shown in Knauf et al. (1988) J. Bio. Chem. 263:15064-15070, and a discussion of POG/IL-2 conjugates is found in U.S. Patent No. 4,766,106.

Another drug delivery system for increasing circulatory half-life is the liposome. Methods of preparing liposome delivery systems are discussed in Gabizon et al. (1982) Cancer Research 42:4734; Cafiso (1981) Biochem Biophys Acta 649:129; and Szoka (1980) Ann. Rev. Biophys. Eng. 9:467. Other drug delivery systems are known in the art and are described in, *e.g.,* Poznansky et al. (1980) Drug Delivery Systems (R.L. Juliano, ed., Oxford, N.Y.) pp. 253-315; Poznansky (1984) Pharm Revs 36:277.

The formulants to be incorporated into a pharmaceutical composition should provide for the stability of the antagonist anti-CD40 antibody or antigen-binding fragment thereof. That is, the antagonist anti-CD40 antibody or antigen-binding fragment thereof should retain its physical and/or chemical stability and have the desired biological activity, i.e., one or more of the antagonist activities defined herein above, including, but not limited to, inhibition of immunoglobulin secretion by normal human peripheral B cells stimulated by T cells; inhibition of survival and/or proliferation of normal human peripheral B cells stimulated by Jurkat T cells; inhibition of survival and/or proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted elsewhere herein.

Methods for monitoring protein stability are well known in the art. See, for example, Jones (1993) Adv. Drug Delivery Rev. 10:29-90; Lee, ed. (1991) Peptide and Protein Drug Delivery (Marcel Dekker, Inc., New York, New York); and the stability assays disclosed herein below. Generally, protein stability is measured at a chosen temperature for a specified period of time. In preferred embodiments, a stable antibody pharmaceutical formulation provides for stability of the antagonist anti-CD40 antibody or antigen-binding fragment thereof when stored at room temperature (about 25°C) for at least 1 month, at least 3 months, or at least 6 months, and/or is stable at about 2-8°C for at least 6 months, at least 9 months, at least 12 months, at least 18 months, at least 24 months.

A protein such as an antibody, when formulated in a pharmaceutical composition, is considered to retain its physical stability at a given point in time if it shows no visual signs (i.e., discoloration or loss of clarity) or measurable signs (for example, using size-exclusion chromatography (SEC) or UV light scattering) of precipitation, aggregation, and/or denaturation in that pharmaceutical composition. With respect to chemical stability, a protein such as an antibody, when formulated in a pharmaceutical composition, is considered to retain its chemical stability at a given point in time if measurements of chemical stability are indicative that the protein (i.e., antibody) retains the biological activity of interest in that pharmaceutical composition. Methods for monitoring changes in chemical stability are well known in the art and include, but are not limited to, methods to detect chemically altered forms of the protein such as result from clipping, using, for example, SDS-PAGE, SEC, and/or matrix-assisted laser desorption ionization/time of flight mass spectrometry; and degradation associated with changes in molecular charge (for example, associated with deamidation), using, for example, ion-exchange chromatography. See, for example, the methods disclosed herein below.

An antagonist anti-CD40 antibody or antigen-binding fragment thereof, when formulated in a pharmaceutical composition, is considered to retain a desired biological activity at a given point in time if the desired biological activity at that time is within about 30%, preferably within about 20% of the desired biological activity exhibited at the time the pharmaceutical composition was prepared as determined in a suitable assay for the desired biological activity. Assays for measuring the desired biological activity of the antagonist anti-CD40 antibodies disclosed herein, and antigen-binding fragments thereof, can be performed as described in the Examples herein. See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

In some embodiments of the invention, the antagonist anti-CD40 antibody, for example, the CHIR-12.12 or CHIR-5.9 monoclonal antibody, or antigen-binding fragment thereof is formulated in a liquid pharmaceutical formulation. The antagonist anti-CD40 antibody or antigen binding fragment thereof can be prepared using any method known in the art, including those methods disclosed herein above. In one embodiment, the antagonist anti-CD40 antibody, for example, the CHIR-12.12 or CHIR-5.9 monoclonal antibody, or antigen-binding fragment thereof is recombinantly produced in a CHO cell line.

Following its preparation and purification, the antagonist anti-CD40 antibody or antigen-binding fragment thereof can be formulated as a liquid phannaceutical formulation in the manner set forth herein. Where the antagonist anti-CD40 antibody or antigen-binding, fragment thereof is to be stored prior to its formulation, it can be frozen, ro example, at ≤ -20°C, and then thawed at room temperature for further formulation. The liquid pharmaceutical formulation comprises a therapeutically effective amount of the antagonist anti-CD40 antibody or antigen-binding fragment thereof. The amount of antibody or antigen-binding fragment thereof present in the formulation takes into consideration the route of administration and desired dose volume.

In this manner, the liquid pharmaceutical composition comprises the antagonist anti-CD40 antibody, for example, the CHIR-12.12 or CHIR-5.9 antibody, or antigen-binding fragment thereof at a concentration of about 0.1 mg/ml to about 50.0 mg/ml, about 0.5 mg/ml to about 40.0 mg/ml, about 1.0 mg/ml to about 30.0 mg/ml, about 5.0 mg/ml to about 25.0 mg/ml, about 5.0 mg/ml to about 20.0 mg/ml, or about 15.0 mg/ml to about 25.0 mg/ml. In some embodiments, the liquid pharmaceutical composition comprises the antagonist anti-CD40 antibody or antigen-binding fragment thereof at a concentration of about 0.1 mg/ml to about 5.0 mg/ml, about 5.0 mg/ml to about 10.0 mg/ml, about 10.0 mg/ml to about 15.0 mg/ml, about 15.0 mg/ml to about 20.0 mg/ml, about 20.0 mg/ml to about 25.0 mg/ml, about 25.0 mg/ml to about 30.0 mg/ml, about 30.0 mg/ml to about 35.0 mg/ml, about 35.0 mg/ml to about 40.0 mg/ml, about 40.0 mg/ml to about 45.0 mg/ml, or about 45.0 mg/ml to about 50.0 mg/ml. In other embodiments, the liquid pharmaceutical composition comprises the antagonist anti-CD40 antibody or antigen-binding fragment thereof at a concentration of about 15.0 mg/ml, about 16.0 mg/ml, about 17.0 mg/ml, about 18.0 mg/ml, about 19.0 mg/ml, about 20.0 mg/ml, about 21.0 mg/ml, about 22.0 mg/ml, about 23.0 mg/ml, about 24.0 mg/ml, or about 25.0 mg/ml. The liquid pharmaceutical composition comprises the antagonist anti-CD40 antibody, for example, the CHIR-12.12 or CHIR-5.9 antibody, or antigen-binding fragment thereof and a buffer that maintains the pH of the formulation in the range of about pH 5.0 to about pH 7.0, including about pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, and other such values within the range of about pH 5.0 to about pH 7.0. In some embodiments, the buffer maintains the pH of the formulation in the range of about pH 5.0 to about pH 6.5, about pH 5.0 to about pH 6.0, about pH 5.0 to about pH 5.5, about pH 5.5 to about 7.0, about pH 5.5 to about pH 6.5, or about pH 5.5 to about pH 6.0.

Any suitable buffer that maintains the pH of the liquid anti-CD40 antibody formulation in the range of about pH 5.0 to about pH 7.0 can be used in the formulation, so long as the physicochemical stability and desired biological activity of the antibody are retained as noted herein above. Suitable buffers include, but are not limited to, conventional acids and salts thereof, where the counter ion can be, for example, sodium, potassium, ammonium, calcium, or magnesium. Examples of conventional acids and salts thereof that can be used to buffer the pharmaceutical liquid formulation include, but are not limited to, succinic acid or succinate, citric acid or citrate, acetic acid or acetate, tartaric acid or tartarate, phosphoric acid or phosphate, gluconic acid or gluconate, glutamic acid or glutamate, aspartic acid or aspartate, maleic acid or maleate, and malic acid or malate buffers. The buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, 15 mM, or other such values within the range of about 5 mM to about 15 mM.

In some embodiments of the invention, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof and succinate buffer or citrate buffer at a concentration that maintains the pH of the formulation in the range of about pH 5.0 to about pH 7.0, preferably about pH 5.0 to about pH 6.5. By "succinate buffer" or "citrate buffer" is intended a buffer comprising a salt of succinic acid or a salt of citric acid, respectively. In a preferred embodiment, the succinate or citrate counterion is the sodium cation, and thus the buffer is sodium succinate or sodium citrate, respectively. However, any cation is expected to be effective. Other possible succinate or citrate cations include, but are not limited to, potassium, ammonium, calcium, and magnesium. As noted above, the succinate or citrate buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, or about 15 mM. In other embodiments, the liquid pharmaceutical formulation comprises the antagonist anti-CD40 antibody, for example, the CHIR-12.12 or CHIR-5.9 monoclonal antibody, or antigen-binding fragment thereof at a concentration of about 0.1 mg/ml to about 50.0 mg/ml, or about 5.0 mg/ml to about 25.0 mg/ml, and succinate or citrate buffer, for example, sodium succinate or sodium citrate buffer, at a concentration of about 1 mM to about 20 mM, about 5 mM to about 15 mM, preferably about 10 mM.

Where it is desirable for the liquid pharmaceutical formulation to be near isotonic, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0 can further comprise an amount of an isotonizing agent sufficient to render the formulation near isotonic. By "near isotonic" is intended the aqueous formulation has an osmolarity of about 240 mmol/kg to about 360 mmol/kg, preferably about 240 to about 340 mmol/kg, more preferably about 250 to about 330 mmol/kg, even more preferably about 260 to about 320 mmol/kg, still more preferably about 270 to about 310 mmol/kg. Methods of determining the isotonicity of a solution are known to those skilled in the art. See, for example, Setnikar et al. (1959) J. Am. Pharm. Assoc. 48:628.

Those skilled in the art are familiar with a variety of pharmaceutically acceptable solutes useful in providing isotonicity in pharmaceutical compositions. The isotonizing agent can be any reagent capable of adjusting the osmotic pressure of the liquid pharmaceutical formulation of the present invention to a value nearly equal to that of a body fluid. It is desirable to use a physiologically acceptable isotonizing agent. Thus, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, can further comprise components that can be used to provide isotonicity, for example, sodium chloride; amino acids such as alanine, valine, and glycine; sugars and sugar alcohols (polyols), including, but not limited to, glucose, dextrose, fructose, sucrose, maltose, mannitol, trehalose, glycerol, sorbitol, and xylitol; acetic acid, other organic acids or their salts, and relatively minor amounts of citrates or phosphates. The ordinary skilled person would know of additional agents that are suitable for providing optimal tonicity of the liquid formulation.

In some preferred embodiments, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, further comprises sodium chloride as the isotonizing agent. The concentration of sodium chloride in the formulation will depend upon the contribution of other components to tonicity. In some embodiments, the concentration of sodium chloride is about 50 mM to about 300 mM, about 50 mM to about 250 mM, about 50 mM to about 200 mM, about 50 mM to about 175 mM, about 50 mM to about 150 mM, about 75 mM to about 175 mM, about 75 mM to about 150 mM, about 100 mM to about 175 mM, about 100 mM to about 200 mM, about 100 mM to about 150 mM, about 125 mM to about 175 mM, about 125 mM to about 150 mM, about 130 mM to about 170 mM, about 130 mM to about 160 mM, about 135 mM to about 155 mM, about 140 mM to about 155 mM, or about 145 mM to about 155 mM. In one such embodiment, the concentration of sodium chloride is about 150 mM. In other such embodiments, the concentration of sodium chloride is about 150 mM, the buffer is sodium succinate or sodium citrate buffer at a concentration of about 5 mM to about 15 mM, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, and the formulation has a pH of about pH 5.0 to about pH 7.0, about pH 5.0 to about pH 6.0, or about pH 5.5 to about pH 6.5. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, about 150 mM sodium chloride, and about 10 mM sodium succinate or sodium citrate, at a pH of about pH 5.5.

Protein degradation due to freeze thawing or mechanical shearing during processing of a liquid pharmaceutical formulations of the present invention can be inhibited by incorporation of surfactants into the formulation in order to lower the surface tension at the solution-air interface. Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, and further comprises a surfactant. In other embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, an isotonizing agent such as sodium chloride at a concentration of about 50 mM to about 300 mM, and further comprises a surfactant.

Typical surfactants employed are nonionic surfactants, including polyoxyethylene sorbitol esters such as polysorbate 80 (Tween 80) and polysorbate 20 (Tween 20); polyoxypropylene-polyoxyethylene esters such as Pluronic F68; polyoxyethylene alcohols such as Brij 35; simethicone; polyethylene glycol such as PEG400; lysophosphatidylcholine; and polyoxyethylene-p-t-octylphenol such as Triton X-100. Classic stabilization of pharmaceuticals by surfactants or emulsifiers is described, for example, in Levine et al. (1991) J. Parenteral Sci. Technol. 45(3):160-165. A preferred surfactant employed in the practice of the present invention is polysorbate 80. Where a surfactant is included, it is typically added in an amount from about 0.001 % to about 1.0% (w/v), about 0.001% to about 0.5%, about 0.001% to about 0.4%, about 0.001% to about 0.3%, about 0.001% to about 0.2%, about 0.005% to about 0.5%, about 0.005% to about 0.2%, about 0.01% to about 0.5%, about 0.01% to about 0.2%, about 0.03% to about 0.5%, about 0.03% to about 0.3%, about 0.05% to about 0.5%, or about 0.05% to about 0.2%.

Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, the buffer is sodium succinate or sodium citrate buffer at a concentration of about 1 mM to about 50 mM, about 5 mM to about 25 mM, or about 5 mM to about 15 mM; the formulation has a pH of about pH 5.0 to about pH 7.0, about pH 5.0 to about pH 6.0, or about pH 5.5 to about pH 6.5; and the formulation further comprises a surfactant, for example, polysorbate 80, in an amount from about 0.001% to about 1.0% or about 0.001% to about 0.5%. Such formulations can optionally comprise an isotonizing agent, such as sodium chloride at a concentration of about 50 mM to about 300 mM, about 50 mM to about 200 mM, or about 50 mM to about 150 mM. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, including about 20.0 mg/ml; about 50 mM to about 200 mM sodium chloride, including about 150 mM sodium chloride; sodium succinate or sodium citrate at about 5 mM to about 20 mM, including about 10 mM sodium succinate or sodium citrate; sodium chloride at a concentration of about 50 mM to about 200 mM, including about 150 mM; and optionally a surfactant, for example, polysorbate 80, in an amount from about 0.001% to about 1.0%, including about 0.001% to about 0.5%; where the liquid pharmaceutical formulation has a pH of about pH 5.0 to about pH 7.0, about pH 5.0 to about pH 6.0, about pH 5.0 to about pH 5.5, about pH 5.5 to about pH 6.5, or about pH 5.5 to about pH 6.0.

The liquid pharmaceutical formulation can be essentially free of any preservatives and other carriers, excipients, or stabilizers noted herein above. Alternatively, the formulation can include one or more preservatives, for example, antibacterial agents, pharmaceutically acceptable carriers, excipients, or stabilizers described herein above provided they do not adversely affect the physicochemical stability of the antagonist anti-CD40 antibody or antigen-binding fragment thereof. Examples of acceptable carriers, excipients, and stabilizers include, but are not limited to, additional buffering agents, co-solvents, surfactants, antioxidants including ascorbic acid and methionine, chelating agents such as EDTA, metal complexes (for example, Zn-protein complexes), and biodegradable polymers such as polyesters. A thorough discussion of formulation and selection of pharmaceutically acceptable carriers, stabilizers, and isomolytes can be found in Remington's Pharmaceutical Sciences (18th ed.; Mack Publishing Company, Eaton, Pennsylvania, 1990).

After the liquid pharmaceutical formulation or other pharmaceutical composition described herein is prepared, it can be lyophilized to prevent degradation. Methods for lyophilizing liquid compositions are known to those of ordinary skill in the art. Just prior to use, the composition may be reconstituted with a sterile diluent (Ringer's solution, distilled water, or sterile saline, for example) that may include additional ingredients. Upon reconstitution, the composition is preferably administered to subjects using those methods that are known to those skilled in the art.

### Use of Antagonist Anti-CD40 Antibodies in the Manufacture of Medicaments

The present invention also provides for the use of an antagonist anti-CD40 antibody or antigen-binding fragment thereof in the manufacture of a medicament for treating a subject for a solid tumor comprising carcinoma cells expressing CD40 antigen, wherein the medicament is coordinated with treatment with at least one other cancer therapy. Examples of such tumors include, but are not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas).

By "coordinated" is intended the medicament is to be used either prior to, during, or after treatment of the subject with at least one other cancer therapy. Examples of other cancer therapies include, but are not limited to, surgery; radiation therapy; chemotherapy, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; cytokine therapy, including, but not limited to, alpha-interferon therapy, gamma-interferon therapy, therapy with interleukin-2 (IL-2), IL-12, IL-15, and IL-21, granulocyte macrophage colony stimulating factor (GM-CSF), granulocyte colony stimulating factor (G-CSF), or biologically active variants of these cytokines; or other monoclonal antibody intended for use in treatment of the solid tumor of interest, for example, Herceptin® (Genentech, Inc., San Francisco, California), which targets the Her2 receptor protein on Her2+ breast cancer cells; the humanized monoclonal antibody Avastin™ (also known as bevacizumab; Genentech, Inc., San Francisco, California), which binds to and inhibits vascular endothelial growth factor (VEGF), and has use in treatment of colon cancer; anti-EGFR antibody targeting the epidermal growth factor receptor (for example, IMC-C225 (ImClone Systems, New York, New York); anti-IGF-1 receptor antibody, targeting the IGF-1 receptor protein; anti-MUC1 antibody, targeting the tumor-associated antigen MUC1; anti-α5β1, anti-αvβ5, and anti-αvβ3, targeting these respective integrins, which regulate cell adhesion and signaling processes involved in cell proliferation and survival; anti-P-cadherin antibody, targeting this cadherin family member (see, for example, U.S. Patent Application Publication No. 20030194406); and anti-VE-cadherin antibody, targeting angiogenic-related function of this endothelial cell-specific adhesion molecule; where treatment with the additional cancer therapy, or additional cancer therapies, occurs prior to, during, or subsequent to treatment of the subject with the medicament comprising the antagonist anti-CD40 antibody or antigen-binding fragment thereof, as noted herein above.

Thus, for example, in some embodiments, the invention provides for the use of the monoclonal antibody CHIR-12.12 or CHIR-5.9, or antigen-binding fragment thereof, in the manufacture of a medicament for treating a subject for a solid tumor comprising carcinoma cells expressing CD40 antigen, wherein the medicament is coordinated with treatment with chemotherapy, where the chemotherapeutic agent is selected from the group consisting of CPT-11 (Irinotecan), which can be used, for example, in treating colorectal cancer and non-small cell lung cancer; gemcitabine, which can be used, for example, in treating lung cancer, breast cancer, and epithelial ovarian cancer; and other chemotherapeutic agents suitable for treatment of solid tumors; where treatment with the additional cancer therapy, or additional cancer therapies, occurs prior to, during, or subsequent to treatment of the subject with the medicament comprising the antagonist anti-CD40 antibody or antigen-binding fragment thereof, as noted herein above.

In other embodiments, the invention provides for the use of the monoclonal antibody CHIR-12.12 or CHIR-5.9, or antigen-binding fragment thereof, in the manufacture of a medicament for treating a subject for a solid tumor comprising carcinoma cells expressing CD40 antigen, wherein the medicament is coordinated with treatment with at least one other anti-cancer antibody selected from the group consisting of Herceptin® (Genentech, Inc., San Francisco, California), which targets the Her2 receptor protein on Her2+ breast cancer cells; the humanized monoclonal antibody Avastin™ (also known as bevacizumab; Genentech, Inc., San Francisco, California), which binds to and inhibits vascular endothelial growth factor (VEGF), and has use in treatment of colon cancer; anti-EGFR antibody targeting the epidermal growth factor receptor (for example, IMC-C225 (ImClone Systems, New York, New York); anti-IGF-1 receptor antibody, targeting the IGF-1 receptor protein; anti-MUC1 antibody, targeting the tumor-associated antigen MUC1; anti-α5β1, anti-αvβ5, and anti-αvβ3, targeting these respective integrins, which regulate cell adhesion and signaling processes involved in cell proliferation and survival; anti-P-cadherin antibody, targeting this cadherin family member (see, for example, U.S. Patent Application Publication No. 20030194406); and anti-VE-cadherin antibody, targeting angiogenic-related function of this endothelial cell-specific adhesion molecule; where treatment with the additional cancer therapy, or additional cancer therapies, occurs prior to, during, or subsequent to treatment of the subject with the medicament comprising the antagonist anti-CD40 antibody or antigen-binding fragment thereof, as noted herein above.

The invention also provides for the use of an antagonist anti-CD40 antibody, for example, the monoclonal antibody CHIR-12.12 or CHIR-5.9 disclosed herein, or antigen-binding fragment thereof in the manufacture of a medicament for treating a subject for a solid tumor comprising carcinoma cells expressing CD40 antigen, wherein the medicament is used in a subject that has been pretreated with at least one other cancer therapy. By "pretreated" or "pretreatment" is intended the subject has received one or more other cancer therapies (i.e., been treated with at least one other cancer therapy) prior to receiving the medicament comprising the antagonist anti-CD40 antibody or antigen-binding fragment thereof. "Pretreated" or "pretreatment" includes subjects that have been treated with at least one other cancer therapyy within 2 years, within 18 months, within 1 year, within 6 months, within 2 months, within 6 weeks, within 1 month, within 4 weeks, within 3 weeks, within 2 weeks, within 1 week, within 6 days, within 5 days, within 4 days, within 3 days, within 2 days, or even within 1 day prior to initiation of treatment with the medicament comprising the antagonist anti-CD40 antibody, for example, the monoclonal antibody CHIR-12.12 or CHIR-5.9 disclosed herein, or antigen-binding fragment thereof. It is not necessary that the subject was a responder to pretreatment with the prior cancer therapy, or prior cancer therapies. Thus, the subject that receives the medicament comprising the antagonist anti-CD40 antibody or antigen-binding fragment thereof could have responded, or could have failed to respond (i.e. the cancer was refractory), to pretreatment with the prior cancer therapy, or to one or more of the prior cancer therapies where pretreatment comprised multiple cancer therapies. Examples of other cancer therapies for which a subject can have received pretreatment prior to receiving the medicament comprising the antagonist anti-CD40 antibody or antigen-binding fragment thereof include, but are not limited to, surgery; radiation therapy; chemotherapy, where suitable chemotherapeutic agents include, but are not limited to, those listed herein above; other anti-cancer monoclonal antibody therapy, including, but not limited to, those anti-cancer antibodies listed herein above; cytokine therapy, including the cytokine therapies listed herein above; or any combination thereof.

"Treatment" in the context of coordinated use of a medicament described herein with one or more other cancer therapies is herein defined as the application or administration of the medicament or of the other cancer therapy to a subject, or application or administration of the medicament or other cancer therapy to an isolated tissue or cell line from a subject, where the subject has a solid tumor comprising carcinoma cells expressing CD40 antigen, a symptom associated with such a cancer, or a predisposition toward development of such a cancer, where the purpose is to cure, heal, alleviate, relieve, alter, remedy, ameliorate, improve, or affect the cancer, any associated symptoms of the cancer, or the predisposition toward the development of the cancer.

The following examples are offered by way of illustration and not by way of limitation.

### EXPERIMENTAL

### Introduction

The antagonist anti-CD40 antibodies used in the examples below are CHIR-5.9 and CHIR-12.12. The CHIR-5.9 and CHIR-12.12 anti-CD40 antibodies are human IgG₁ subtype anti-human CD40 monoclonal antibodies (mAbs) generated by immunization of transgenic mice bearing the human IgG₁ heavy chain locus and the human κ chain locus (XenoMouse^{®} technology; Abgenix; Fremont, California). SF9 insect cells expressing CD40 extracellular domain were used as immunogen.

Briefly, splenocytes from immunized mice were fused with SP 2/0 or P 3 x 63Ag8.653 murine myeloma cells at a ratio of 10:1 using 50% polyethylene glycol as previously described by de Boer et al. (1988) J. Immunol. Meth. 113:143. The fused cells were resuspended in complete IMDM medium supplemented with hypoxanthine (0.1 mM), aminopterin (0.01 mM), thymidine (0.016 mM), and 0.5 ng/ml hIL-6 (Genzyme, Cambridge, Massachusetts). The fused cells were then distributed between the wells of 96-well tissue culture plates, so that each well contained 1 growing hybridoma on average.

After 10-14 days, the supernatants of the hybridoma populations were screened for specific antibody production. For the screening of specific antibody production by the hybridoma clones, the supernatants from each well were pooled and tested for anti-CD40 activity specificity by ELISA first. The positives were then used for fluorescent cell staining of EBV-transformed B cells using a standard FACS assay. Positive hybridoma cells were cloned twice by limiting dilution in IMDM/FBS containing 0.5 ng/ml hIL-6.

A total of 31 mice spleens were fused with the mouse myeloma SP2/0 cells to generate 895 antibodies that recognize recombinant CD40 in ELISA. On average approximately 10% of hybridomas produced using Abgenix XenoMouse^{®} technology (Abgenix; Fremont, California) may contain mouse lambda light chain instead of human kappa chain. The antibodies containing mouse light lambda chain were selected out. A subset of 260 antibodies that also showed binding to cell-surface CD40 were selected for further analysis. Stable hybridomas selected during a series of subcloning procedures were used for further characterization in binding and functional assays.

Clones from 7 other hybridomas were identified as having antagonistic activity. Based on their relative antagonistic potency and ADCC activities, two hybridoma clones were selected for further evaluation (Table 1 below). They are named 131.2F8.5.9 (5.9) and 153.8E2.D10.D6.12.12 (12.12).

**Table 1. Summary of initial set of data with anti-CD40 IgG1 antibodies CHIR-5.9 and CHIR-12.12.**

| **Mother Hybridoma** | **Hybridoma clones** | **cell surface binding** | **Antagonist** | **ADCC** | **CDC** | **CMCC#** | **V-region DNA sequence** |
|---|---|---|---|---|---|---|---|
| 131.2F5 | 131.2F5.8.5.9 | +++ | +++ | ++ | - | 12047 | Yes |
| 153.8E2 | 153.8E2D10D6.12.12 | +++ | +++ | ++++ | - | 12056 | Yes |

Mouse hybridoma line 131.2F8.5.9 (CMCC#12047) and hybridoma line 153.8E2.D10.D6.12.12 (CMCC#12056) have been deposited with the American Type Culture Collection [ATCC; 10801 University Blvd., Manassas, Virginia 20110-2209 (USA)] under Patent Deposit Number PTA-5542 and PTA-5543, respectively.

The cDNAs encoding the variable regions of the candidate antibodies were amplified by PCR, cloned, and sequenced. The amino acid sequences for the light chain and heavy chain of the CHIR-12.12 antibody are set forth in Figures 1A and 1B, respectively. See also SEQ ID NO:2 (light chain for mAb CHIR-12.12) and SEQ ID NO:4 (heavy chain for mAb CHIR-12.12). A variant of the heavy chain for mAb CHIR-12.12 is shown in Figure 1B (see also SEQ ID NO:5), which differs from SEQ ID NO:4 in having a serine residue substituted for the alanine residue at position 153 of SEQ ID NO:4. The nucleotide sequences encoding the light chain and heavy chain of the CHIR-12.12 antibody are set forth in Figures 2A and 2B, respectively. See also SEQ ID NO:1 (coding sequence for light chain for mAb CHIR-12.12) and SEQ ID NO:3 (coding sequence for heavy chain for mAb CHIR-12.12). The amino acid sequences for the light chain and heavy chain of the CHIR-5.9 antibody are set forth in Figures 3A and 3B, respectively. See also SEQ ID NO:6 (light chain for mAb CHIR-5.9) and SEQ ID NO:7 (heavy chain for mAb CHIR-5.9). A variant of the heavy chain for mAb CHIR-5.9 is shown in Figure 3B (see also SEQ ID NO:8), which differs from SEQ ID NO:7 in having a serine residue substituted for the alanine residue at position 158 of SEQ ID NO:7.

As expected for antibodies derived from independent hybridomas, there is substantial variation in the nucleotide sequences in the complementarity determining regions (CDRs). The diversity in the CDR3 region of V_{H} is believed to most significantly determine antibody specificity.

As shown by FACS analysis, CHIR-5.9 and CHIR-12.12 bind specifically to human CD40 and can prevent CD40-ligand binding. Both mAbs can compete off CD40-ligand pre-bound to cell surface CD40. The binding affinity of CHIR-5.9 to human CD40 is 1.2×10⁻⁸ M and the binding affinity of CHIR-12.12 to human CD40 is 5×10⁻¹⁰ M.

The CHIR-12.12 and CHIR-5.9 monoclonal antibodies are strong antagonists and inhibit *in vitro* CD40 ligand-mediated proliferation of normal B cells, as well as inhibiting *in vitro* CD40 ligand-mediated proliferation of cancer cells from NHL and CLL patients. *In vitro,* both antibodies kill primary cancer cells from NHL patients by ADCC. Dose-dependent anti-tumor activity was seen in a xenograft human lymphoma model.

### Example 1: CD40 Is Expressed on a High Proportion of Solid Tumors

A variety of solid tumor-derived cultured cancer cell lines and patient biopsies have been found to express CD40. A high % of biopsied samples from breast, lung, ovary, and skin cancer patients were found to express CD40.

### Example 2: Ability of Candidate Monoclonal Antibody CHIR-12.12 to Bind to CD40 Expressed on Several Human Carcinomas

Carcinoma tissue samples were obtained from individuals (N=10) with ovarian, lung, mammary, or colon cancer and frozen for subsequent analysis of antibody binding using immunohistochemistry. The percent of neoplastic cells in the various human carcinomas able to bind to the CHIR-12.12 mAb was determined. As can be seen from Table 2, 60% of the ovarian and lung carcinoma samples fell into the highest category of percent binding (i.e., 50-100% of the cells in these samples were able to bind to the CHIR-12.12 mAb). Thirty percent of the mammary and 10% of the colon carcinoma samples fell into the highest category of percent binding.

**Table 2. Ability of CHIR-12.12 mAb to bind to several human carcinomas.**

| **Carcinomas** | **Percent of Neoplastic Cells Binding to CHIR-12.12 mAb** | | | | |
|---|---|---|---|---|---|
| | **Negative** | **<5%** | **5-25%** | **25-50%** | **50-100%** |
| ***Ovarian*** | **0 %** | **0 %** | **30%** | **10%** | **60%** |
| ***n=10*** | | | | | |
| ***Lung*** | **0 %** | **20%** | **20%** | **0 %** | **60%** |
| ***n=10*** | | | | | |
| ***Mammary*** | **0 %** | **30%** | **20%** | **20%** | **30%** |
| ***N=10*** | | | | | |
| ***Colon*** | **0 %** | **70 %** | **10%** | **10%** | **10%** |
| ***N=10*** | | | | | |

### Example 3: CHIR-5.9 and CHIR-12.12 Are Able to Kill CD40-Bearing Target Cells by ADCC

The candidate antibodies can kill CD40-bearing target cells (lymphoma lines and solid tumor cell lines) by the mechanism of ADCC. Both CHIR-5.9 and CHIR-12.12 are fully human antibodies of IgG1 isotype and are expected to have the ability to induce the killing of target cells by the mechanism of ADCC. They were tested for their ability to kill cancer cell lines in *in vitro* assays. Two human lymphoma cell lines (Ramos and Daudi) and one human colon cancer cell line (HCT116) were initially selected as target cells for these assays. PBMC or enriched NK cells from 8 normal volunteer donors were used as effector cells in these assays. Higher ADCC was seen against lymphoma cell lines that the colon cancer cell line. A more potent ADCC response was observed with CHIR-12.12 compared with CHIR-5.9 against both the lymphoma and colon cancer cell line target cells. Lymphoma cell lines also express CD20, the target antigen for rituximab (Rituxan®; IDEC Pharmaceuticals Corp., San Diego, California), which allowed for comparison of the ADCC activity of these two candidate mAbs with rituximab ADCC activity. For lymphoma cell line target, an average specific lysis of 35%, 59%, and 47% was observed for CHIR-5.9, CHIR-12.12, and rituximab respectively when used at 1 µg/ml concentration. For colon cancer cell line target, an average specific lysis of 20% and 39% were observed for CHIR-5.9 and CHIR-12.12, respectively. See Table 3 below. The two antibodies did not show much activity in complement dependent cytotoxicity (CDC) assays.

**Table 3. Anti-CD40 mAB dependent killing of lymphoma and colon cancer cell lines by ADCC.**

| **Anti-CD40 mAb dependent killing of lymphoma and colon cancer cell lines by ADCC** | | | | | | | | | | | | | | | | |
|---|---|---|---|---|---|---|---|---|---|---|---|---|---|---|---|---|
| Exp# | Effector cell | E:T ratio | **Target cells : Human lymphoma cell line (Ramos or Daudi)** | | | | | | | | **Target cells: Human Colon Carcinoma (HCT116)** | | | | | |
| | | | % lysis IgG1 | Abs conc(ug/ml) | **5.9** | | **12.12** | | **Rituxan** | | % lysis IgG1 | Abs conc(ug/ml) | **5.9** | | **12.12** | |
| | | | | | % lysis | % lysis - % lysis IgG1 | % lysis | % lysis - % lysis IgG1 | % lysis | % lysis-% lysis IgG1 | | | % lysis | % lysis - % lysis ctrl | % lysis | % lysis - % lysis ctrl |
| ADCC-005 | huNK | 3 | 17.05 | 5 | 30.75 | **13.70** | 65.22 | **48.17** | ND | ND | ND | ND | ND | ND | ND | ND |
| Alarmor Blue | huNK | 3 | 40.81 | 5 | 58.62 | **17.81** | 87.87 | **47.06** | ND | ND | ND | ND | ND | ND | ND | ND |
| ADCC-006 | huNK | 2 | -3.09 | 10 | 3.50 | **6.59** | 43.71 | **46.8** | 34.82 | **37.91** | 11.14 | 10 | 12.78 | **1.64** | 33.6 | **22.46** |
| Alarmor Blue | | | -8.62 | 1 | -10.10 | **-1.48** | 45.13 | **53.75** | 37.07 | **45.69** | 13.6 | 1 | 8.921 | **-4.68** | 21.99 | **8.39** |
| | | | -11 | 0.1 | -14.80 | **-3.80** | 39.82 | **50.82** | 33.61 | **44.61** | 15.49 | 0.1 | 8.82 | **-6.67** | 26.15 | **10.66** |
| | | | -4.54 | 0.01 | 2.53 | **7.07** | 50.07 | **54.61** | 28.49 | **33.03** | 13.62 | 0.01 | 17.24 | **3.62** | 28.07 | **14.45** |
| 51Cr | huNK | 5 | 1.5 | 10 | 32.09 | **30.59** | 47.24 | **45.742** | ND | ND | 33.1 | 10 | 120.02 | **86.92** | 170.41 | **137.31** |
| | | | 2.4 | 1 | 18.01 | **15.61** | 37.42 | **35.022** | ND | ND | 23.1 | 2 | 138.4 | **115.3** | 166.62 | **143.52** |
| | | | 2.5 | 0.1 | 14.67 | **12.17** | 37.63 | **35.131** | ND | ND | 46.4 | 0.4 | 141.41 | **95.01** | 159.45 | **113.05** |
| ADCC-009 | huNK | 10 | 2.32 | 5 | 66.20 | **63.88** | 97.70 | **95.38** | 86.2 | **83.88** | 26.4 | 5 | 41.3 | **14.9** | 62.3 | **35.9** |
| Calcien AM | | | 0.48 | 1 | 67.20 | **66.72** | 123.00 | **122.52** | 88.2 | **87.72** | 32.8 | 1 | 43.8 | **11** | 73.8 | **41** |
| | | | -1.43 | 0.2 | 78.40 | **79.83** | 118.00 | **119.43** | 88.8 | **90.23** | 33.5 | 0.2 | 42.6 | **9.1** | 71.4 | **37.9** |
| | | | 3.39 | 0.04 | 69.10 | **65.71** | 109.00 | **105.61** | 84.9 | **81.51** | 26 | 0.04 | 54.8 | **28.8** | 66 | **40** |
| ADCC-011 | huNK | 8 | 3.18 | 1 | 15.36 | **12.19** | 51.59 | **48.42** | 22.44 | **19.27** | 11.63 | 1 | 6.31 | **-5.32** | 19.96 | **8.33** |
| Calcien AM | | | 4.58 | 0.01 | 7.39 | **2.81** | 46.80 | **42.22** | 14.68 | **10.10** | 5.885 | 0.01 | -0.05 | **-5.94** | 15.25 | **9.37** |
| | | | 5.41 | 0.002 | 6.35 | **0.94** | 5.10 | **-0.31** | 9.58 | **4.16** | 4.379 | 0.002 | 3.96 | **-0.42** | .0.75 | **-5.13** |
| | | | 7.03 | 0.0004 | 7.76 | **0.73** | 5.99 | **-1.04** | 5.99 | **-1.04** | 1.883 | 0.0004 | 5.13 | **3.25** | 2.825 | **0.94** |
| ADCC-012 | huNK | 10 | 13.34 | 10 | 73.31 | **59.97** | 117.80 | **104.46** | 50.75 | **37.41** | 0.132 | 10 | 18.42 | **18.29** | 36.1 | **35.97** |
| Calcien AM | | | 13.50 | 1 | 74.76 | **61.26** | 88.64 | **75.14** | 65.97 | **52.47** | 11.15 | 1 | 28.60 | **17.45** | 42.22 | **31.07** |
| | | | 12.27 | 0.01 | 58.52 | **46.25** | 72.88 | **60.61** | 50.16 | **37.89** | 10.89 | 0.01 | 15.51 | **4.62** | 31.07 | **20.18** |
| | | | 13.61 | 0.005 | 57.50 | **43.89** | 69.45 | **55.84** | 39.28 | **25.67** | 14.01 | 0.005 | 23.31 | **9.3** | 27.55 | **13.54** |
| | | | 11.95 | 0.001 | 56.81 | **44.86** | 65.17 | **53.22** | 33.07 | **21.12** | 14.63 | 0.001 | 24.68 | **10.05** | 32.53 | **17.9** |
| ADCC-013 | PBMC | 100 | 2.54 | 1 | 21.03 | **18.49** | 37.94 | **35.40** | 32.28 | **29.74** | 13.53 | 1 | 22.04 | **8.51** | 43.98 | **30.45** |
| 51Cr | | | 2.45 | 0.1 | 15.50 | **13.05** | 30.82 | **28.37** | 27.18 | **24.73** | 15.88 | 0.1 | 21.89 | **6.01** | 43.14 | **27.26** |
| | | | 2.92 | 0.01 | 14.53 | **11.61** | 22.59 | **19.67** | 12.79 | **9.87** | 16.85 | 0.01 | 19.75 | **2.9** | 37.16 | **20.31** |
| | | | 2.78 | 0.001 | 3.90 | **1.12** | 8.99 | **6.21** | 3.13 | **0.35** | 15.45 | 0.001 | 14.36 | **-1.09** | 19.22 | **3.77** |
| ADCC-014 | PBMC | 100 | 4.64 | 10 | 53.54 | **48.90** | 56.12 | **51.48** | ND | ND | 24.12 | 10 | 25.24 | **1.12** | 43.09 | **18.97** |
| 51Cr | | | 4.64 | 1 | 46.84 | **42.20** | 43.00 | **38.36** | ND | ND | 24.12 | 1 | 22.68 | **-1.44** | 32.12 | **8** |
| | | | 4.64 | 0.1 | 45.63 | **40.99** | 39.94 | **35.30** | ND | ND | 24.12 | 0.1 | 24.95 | **0.83** | 31.48 | **7.36** |
| | | | 4.64 | 0.01 | 7.73 | **3.09** | 9.79 | **5.15** | ND | ND | 24.12 | 0.01 | 23.67 | **-0.45** | 26.14 | **2.02** |
| | | | 4.64 | 0.001 | 8.83 | **4.19** | 10.81 | **6.17** | ND | ND | 24.12 | 0.001 | 21.55 | **-2.57** | 21.84 | **-2.28** |
| **Average % lysis at 1 ug/ml concentration of mAbs** | | | | | | **35.31** | | **59.03** | | **47.23** | | | | **20.12** | | **38.68** |
| * The greater than 100% killing are due to incomplete killing by detergent used for 100% killing control. | | | | | | | | | | | | | | | | |

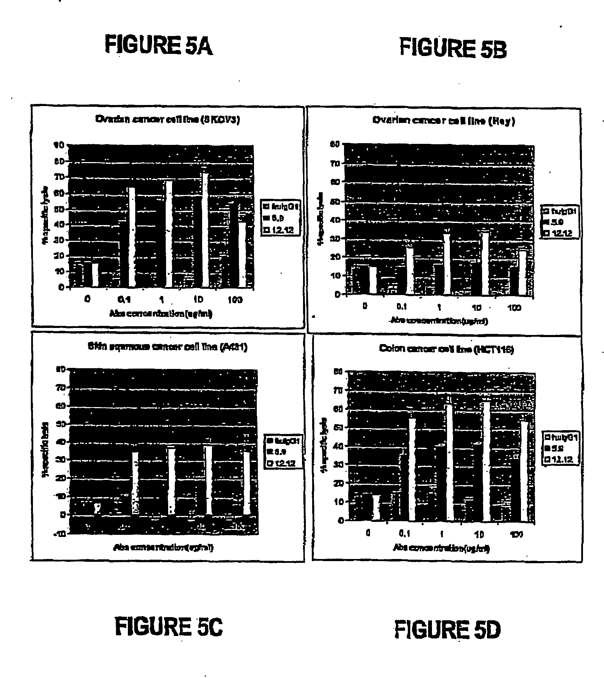

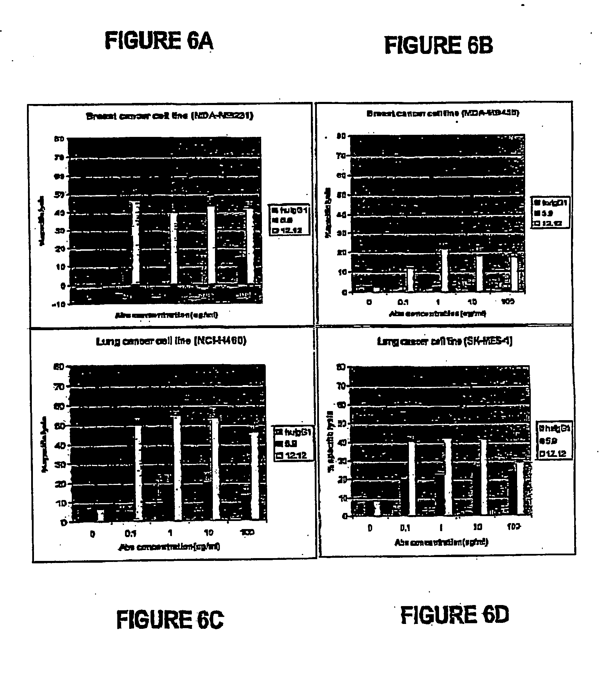

Further testing of the ADCC activity of these two monoclonal anti-CD40 antibodies was carried out on the colon cancer cell line HCT116 and seven other carcinoma cell lines, including the ovarian cancer cell lines SKOV3 and HEY, the skin squamous cancer cell line A431, the breast cancer cell lines MDA-MB231 and MDA-MB435, and the lung cancer cell lines NCI-H460 and SK-MES-1 using the procedures outlined above. As seen in Figures 5A-D and 6A-D, the CHIR-12.12 monoclonal antibody generally exhibited greater ADCC activity than the CHIR-5.9 monoclonal antibody at any given concentration and for any given cell line tested.

### Example 4: CHIR-5.9 and CHIR-12.12 Show Anti-Tumor Activity in Animal Models

### Pharmacology/in vivo efficacy

The candidate mAbs are expected to produce desired pharmacological effects to reduce tumor burden by either/both of two anti-tumor mechanisms, blockade of proliferation/survival signal and induction of ADCC. The currently available xenograft human lymphoma models use long-term lymphoma cell lines that, in contrast to primary cancer cells, do not depend on CD40 stimulation for their growth and survival. Therefore the component of these mAbs' anti-tumor activity based on blocking the tumor proliferation/survival signal is not expected to contribute to anti-tumor efficacy in these models. The efficacy in these models is dependent on the ADCC, the second anti-tumor mechanism associated with the CHIR-5.9 and CHIR-12.12 mAbs.

### Xenograft human B cell lymphoma models

Two xenograft human lymphoma models based on Namalwa and Daudi cell lines were assessed for anti-tumor activities of candidate mAbs. To further demonstrate their therapeutic activity, these candidate mAbs were evaluated in an unstaged (i.e., prophylactic) and staged (i.e., therapeutic) xenograft human lymphoma model based on the Daudi cell line.

To summarize, T cell-deficient nude mice were whole-body irradiated at 3 Gy to further suppress the immune system one day before tumor inoculation. Tumor cells were inoculated subcutaneously in the right flank at 5x10⁶ cells per mouse. Treatment was initiated either one day after tumor implantation (unstaged (prophylactic) subcutaneous xenograft human B cell lymphoma models, Namalwa and Daudi) or when tumor volume reached 200 mm³ (staged (therapeutic) Daudi model, usually 15 days after tumor inoculation). Tumor-bearing mice were injected anti-CD40 mAbs intraperitoneally (i.p.) once a week. Doses for the unstaged Namalwa model were as follows: 0.01 mg/kg, 0.03 mg/kg, 0.1 mg/kg, 1 mg/kg, and 10 mg/kg (mAb CHIR-12.12); 1 mg/kg (mAb CHIR-5.9); and 10 mg/kg (rituximab). Doses for the unstaged Daudi model were as follows: 0.01 mg/kg, 0.1 mg/kg, and 1 mg/kg (mAb CHIR-12.12 and mAb CHIR-5.9); 1 mg/kg (rituximab). Doses for the staged Daudi model were as follows: 0.01 mg/kg, 0.1 mg/kg, 1 mg/kg, and 10 mg/kg (mAb CHIR-12.12); 1 mg/kg (mAb CHIR-5.9); and 1 mg/kg (rituximab). Tumor volumes were recorded twice a week. When tumor volume in any group reached 2500 mm³, the study was terminated. Note that in the staged Daudi model, tumor volume data was analyzed up to day 36 due to the death of some mice after that day. Complete regression (CR) was counted until the end of the study. Data were analyzed using ANOVA or Kruskal-Wallis test and corresponding post-test for multi-group comparison.

In the unstaged Namalwa model, anti-CD40 mAb CHIR-12.12, but not rituximab, significantly (p= <0.01) inhibited the growth of Namalwa tumors (tumor volume reduction of 60% versus 25% for rituxamab, n=10 mice/group) (data not shown). Thus, in this model, anti-CD40 mAb CHIR-12.12 was more potent than rituximab. It is noteworthy that the second candidate mAb, CHIR-5.9, was at least as efficacious as rituximab at a dose 1/10^{th} that of rituximab. Both anti-CD40 mAb CHIR-12.12 and rituximab significantly prevented tumor development in the unstaged Daudi tumor model (14/15 resistance to tumor challenge) (data not shown).

When these anti-CD40 monoclonal antibodies were further compared in a staged xenograft Daudi model, in which treatment started when the subcutaneous tumor was palpable, anti-CD40 mAb CHIR-12.12 at 1 mg/kg caused significant tumor reduction (p=0.003) with 60% complete regression (6/10), while rituximab at the same dose did not significantly inhibit the tumor growth nor did it cause complete regression (0/10) (data not shown).

In summary, the anti-CD40 mAb CHIR-12.12 significantly inhibited tumor growth in experimental lymphoma models. At the same dose and regimen, mAb CHIR-12.12 showed better anti-cancer activity than did Rituxan® (rituximab). Further, no clinical sign of toxicity was observed at this dose and regimen. These data suggest that the anti-CD40 mAb CHIR-12.12 has potent anti-human lymphoma activity *in vitro* and in xenograft models and could be clinically effective for the treatment of lymphoma.

### Xenograft human colon carcinoma model

These candidate mAbs were further evaluated for their therapeutic anti-tumor activity in a solid tumor model. Similar to many human solid tumors, human colon carcinoma cell line HCT 116 expresses CD40 and was selected for a xenograft colon cancer model. Tumor cells were inoculated subcutaneously in the right flank of T-cell deficient nude mice (this tumor can grow in nude mice without prior irradiation) at 5 X 10⁶ cells per mouse. One day after tumor inoculation, mice received intraperitoneal (i.p.) injection of anti-CD40 mAbs once a week for a total of 5 doses.

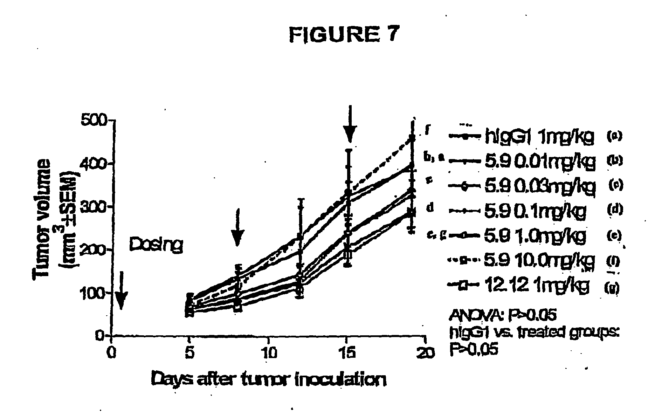

Treatment with anti-CD40 mAbs showed a reproducible trend toward tumor growth inhibition in two repeated studies. The data from one of these two studies is shown in Figure 7. Interestingly, a reversal of anti-tumor activity was observed at the highest dose (10 mg/kg) in this model suggesting an optimal dose/regimen may be needed to achieve best tumor growth inhibition. Monoclonal antibody CHIR-12.12, which showed higher ADCC activity *in vitro* and higher anti-tumor efficacy in a lymphoma model, was tested only at a single dose of 1 mg/kg in the colon carcinoma model. A dose titration of CHIR-12.12 is performed to determine the full potential of its anti-tumor efficacy in this xenograft human colon cancer model.

### Unstaged (prophylactic) orthotopic ovarian cancer model

The CHIR-12.12 mAb was also evaluated for its therapeutic anti-tumor activity in an unstaged (prophylactic) orthotopic murine model of ovarian cancer using the ovarian cancer cell line SKOV3i/p.1. Tumor cells were inoculated intraperitoneally (i.p.) into T-cell deficient nude mice at 2 X 10⁶ cells per mouse. Beginning the first day after tumor inoculation, mice received i.p. injections of various doses of the CHIR-12.12 mAbs or Herceptin® (Genentech, Inc., San Francisco, California), which is under clinical investigation for treatment of ovarian cancer. Antibody was dosed once a week for a total of 6 doses. Percent survival was calculated over time.

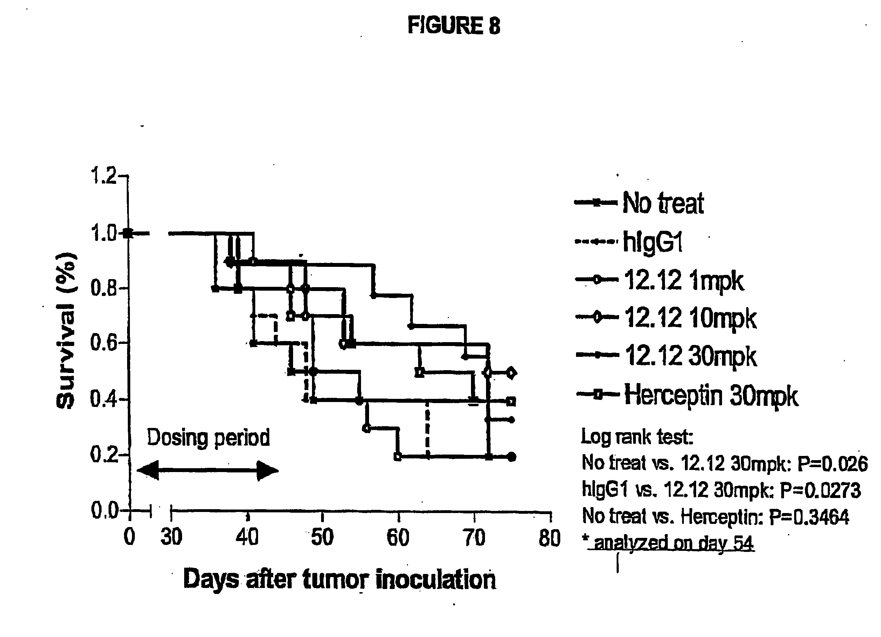

Treatment with the CHIR-12.12 mAb prolonged survival time in a dose-dependent manner (Figure 8). Fifty-four days after tumor inoculation, percent survival was significantly higher for the group receiving 30 mg/kg of the CHIR-12.12 mAb than for the untreated control group. Though a similar dose of Herceptin® showed a trend toward prolonging survival, percent survival at 54 days post-inoculation was not significantly greater than that observed for the untreated control group.

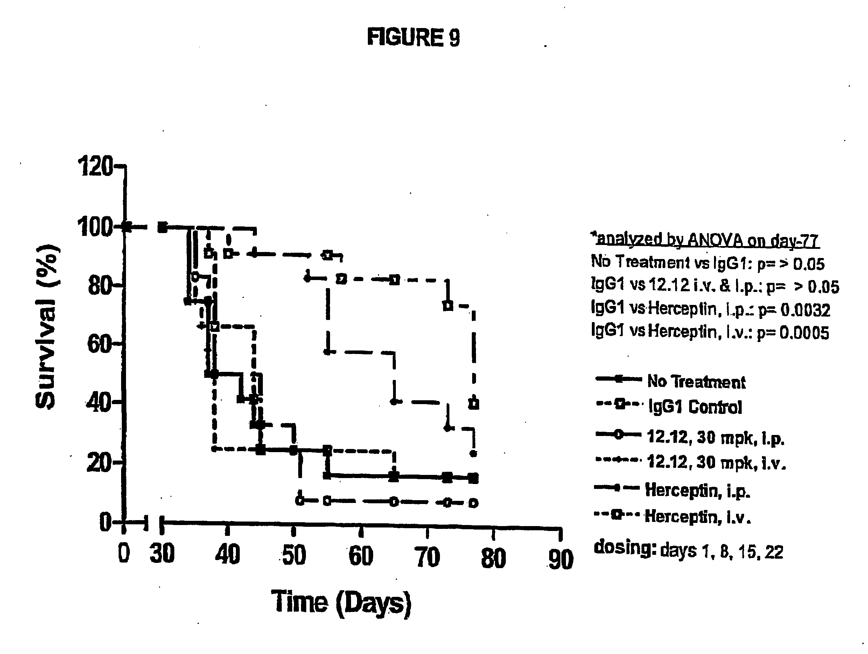

Figure 9 shows a comparison of the effects of the CHIR-12.12 mAb on percent survival in this unstaged orthotopic murine model of ovarian cancer when the antibody is administered intraperitoneally (i.p.) versus intravenously (i.v.). Treatment protocol was as described above. As can be seen in this figure, i.p. injection of the CHIR-12.12 mAb yielded improved percent survival relative to that observed with i.v. administration of this antibody.

### Staged (therapeutic) murine model of ovarian cancer

The CHIR-5.9 and CHIR-12.12 mAbs were further evaluated for their therapeutic anti-tumor activity in a staged (therapeutic) murine model of ovarian cancer using the ovarian cancer cell line SKOV3i.p.1. For this study, tumor cells were inoculated subcutaneously into the right flank of T-cell deficient nude mice at 5 X 10⁶ cells per mouse with 10% matrigel. Beginning 6 days after tumor inoculation (when the tumor volume reached 100-200 mm³), mice received injections of these mAbs intraperitoneally once a week for a total of 4 doses. Tumor volume was measured twice a week following the first day of antibody dosing.

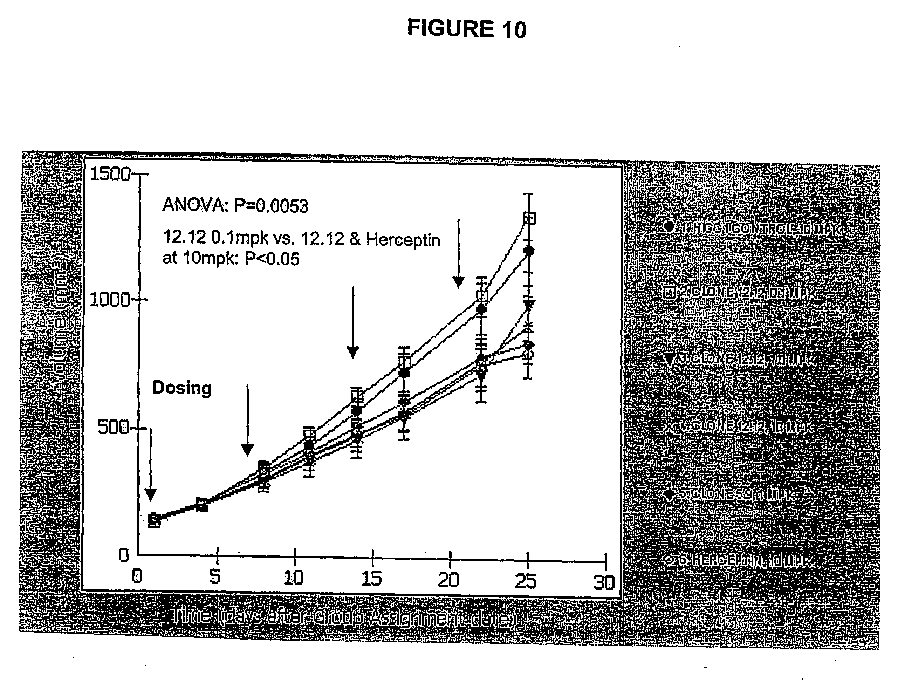

The two candidate mAbs significantly inhibited tumor growth relative to that observed for the untreated control group (Figure 10) at the higher antibody concentrations tested (1 mg/kg for the CHIR-5.9 mAb, and 10 mg/kg for the CHIR-12.12 mAb). For the CHIR-12.12 mAb (the only antibody for which dose was varied), inhibition of tumor growth occurred in a dose-dependent manner, with the greatest tumor reduction occurring at the highest dose (i.e., 10 mg/kg). At this highest dose, the CHIR-12.12 mAb was just as efficacious as an equivalent dose of Herceptin®.

### Example 5: CHIR-5.9 and CHIR-12.12 Bind to a Different Epitope on CD40 than 15B8

The candidate monoclonal antibodies CHIR-5.9 and CHIR-12.12 compete with each other for binding to CD40 but not with 15B8, an IgG₂ anti-CD40 mAb (see International Publication No. WO 02/28904). Antibody competition binding studies using Biacore were designed using CM5 biosensor chips with protein A immobilized via amine coupling, which was used to capture either anti-CD40, CHIR-12.12, or 15B8. Normal association/dissociation binding curves are observed with varying concentrations of CD40-his (data not shown). For competition studies, either CHIR-12.12 or 15B8 were captured onto the protein A surface. Subsequently a CD40-his /CHIR-5.9 Fab complex (100 nM CD40:1 µM CHIR-5.9 Fab), at varying concentrations, was flowed across the modified surface. In the case of CHIR-12.12, there was no association of the complex observed, indicating CHIR-5.9 blocks binding of CHIR-12.12 to CD40-his. For 15B8, association of the Fab CHIR-5.9 complex was observed indicating CHIR-5.9 does not block binding of 15B8 to CD40 binding site. However, the off rate of the complex dramatically increased (data not shown).

It has also been determined that 15B8 and CHIR-12.12 do not compete for CD40-his binding. This experiment was set up by capturing CHIR-12.12 on the protein A biosensor chip, blocking residual protein A sites with control hIgG₁, binding CD40-his and then flowing 15B8 over the modified surface. 15B8 did bind under these conditions indicating CHIR-12.12 does not block 15B8 from binding to CD40.

### Example 6: Binding Properties of CHIR-12.12 and CHIR-5.9 mAB

Protein A was immobilized onto CM5 biosensor chips via amine coupling. Human anti-CD40 monoclonal antibodies, at 1.5 µg/ml, were captured onto the modified biosensor surface for 1.5 minutes at 10 µl/min. Recombinant soluble CD40-his was flowed over the biosensor surface at varying concentrations. Antibody and antigen were diluted in 0.01 M HEPES pH 7.4, 0.15 M NaCl, 3 mM EDTA, 0.005% Surfactant P20 (HBS-EP). Kinetic and affinity constants were determined using the Biaevaluation software with a 1:1 interaction model/global fit.

As shown in Table 4 below, there is 121-fold difference in the off rate of CHIR-5.9 and CHIR-12.12 resulting in 24-fold higher affinity for CHIR-12.12.

| Antibody | **Ka(M-1 sec-1))** | **kd(sec-1)** | **KD(nM)** |
|---|---|---|---|
| Anti-CD40, CHIR-5.9 | (12.35 ± 0.64) x 10⁵ | (15.0 ± 1.3) x 10⁻³ | 12.15 ± 0.35 |
| Anti-CD40, CHIR-12.12 | (2.41 ± 0.13) x 10⁵ | (1.24 ± 0.06) x 10⁻⁴ | 0.51 ± 0.02 |

### Example 7: Characterization of Epitope for Monoclonal Antibodies CHIR-12.12 and CHIR-5.9

To determine the location of the epitope on CD40 recognized by monoclonal antibodies CHIR-12.12 and CHIR-5.9, SDS-PAGE and Western blot analysis were performed. Purified CD40 (0.5 µg) was separated on a 4-12% NUPAGE gel under reducing and non-reducing conditions, transferred to PVDF membranes, and probed with monoclonal antibodies at 10 µg/ml concentration. Blots were probed with alkaline phosphatase conjugated anti-human IgG and developed using the Western Blue^{R} stabilized substrate for alkaline phosphatase (Promega).

Results indicate that anti-CD40 monoclonal antibody CHIR-12.12 recognizes epitopes on both the non-reduced and reduced forms of CD40, with the non-reduced form of CD40 exhibiting greater intensity than the reduced form of CD40 (Table 5; blots not shown). The fact that recognition was positive for both forms of CD40 indicates that this antibody interacts with a conformational epitope part of which is a linear sequence. Monoclonal antibody CHIR-5.9 primarily recognizes the non-reduced form of CD40 suggesting that this antibody interacts with a primarily conformational epitope (Table 5; blots not shown).

**Table 5. Domain identification.**

| | Domain 1 | Domain 2 | Domain 3 | Domain 4 |
|---|---|---|---|---|
| mAb CHIR-12.12 | - | + | - | - |
| mAb CHIR-5.9 | - | + | - | - |
| mAb 15B8 | + | - | - | - |

To map the antigenic region on CD40, the four extracellular domains of CD40 were cloned and expressed in insect cells as GST fusion proteins. The secretion of the four domains was ensured with a GP67 secretion signal. Insect cell supernatant was analyzed by SDS-PAGE and western blot analysis to identify the domain containing the epitope.

Monoclonal antibody CHIR-12.12 recognizes an epitope on Domain 2 under both reducing and non-reducing conditions (Table 6; blots not shown). In contrast, monoclonal antibody CHIR-5.9 exhibits very weak recognition to Domain 2 (Table 6; blots not shown). Neither of these antibodies recognize Domains 1, 3, or 4 in this analysis.

**Table 6. Domain 2 analysis.**

| | Reduced | Non-reduced |
|---|---|---|
| mAb CHIR-12.12 | ++ | +++ |
| mAb CHIR-5.9 | + | + |

To define more precisely the epitope recognized by mAb CHIR-12.12, peptides were synthesized from the extracellular Domain 2 of CD40, which corresponds to the sequence PCGESEFLDTWNRETHCHQHKYCDPNLGLRVQQKGTSETDTICT (residues 61-104 of the sequence shown in SEQ ID NO:10 or SEQ ID NO:12). SPOTs membranes (Sigma) containing thirty-five 10mer peptides with a 1-amino-acid offset were generated. Western blot analysis with mAb CHIR-12.12 and anti-human IgG beta-galactosidase as secondary antibody was performed. The blot was stripped and reprobed with mAb CHIR-5.9 to determine the region recognized by this antibody

SPOTs analysis probing with anti-CD40 monoclonal antibody CHIR-12.12 at 10 µg/ml yielded positive reactions with spots 18 through 22. The sequence region covered by these peptides is shown in Table 7.

**Table 7. Results of SPOTs analysis probing with anti-CD40 monoclonal antibody CHIR-12.12.**

| **Spot Number** | **Sequence Region** |
|---|---|
| 18 | HQHK**YCDPNL** (residues 78-87 of SEQ ID NO:10 or 12) |
| 19 | QHK**YCDPNL**G (residues 79-88 of SEQ ID NO:10 or12) |
| 20 | HK**YCDPNL**GL (residues 80-89 of SEQ ID NO:10 or 12) |
| 21 | K**YCDPNL**GLR (residues 81-90 of SEQ ID NO:10 or 12) |
| 22 | **YCDPNL**GLRV (residues 82-91 of SEQ ID NO:10 or 12) |

These results correspond to a linear epitope of: YCDPNL (residues 82-87 of the sequence shown in SEQ ID NO:10 or SEQ ID NO:12). This epitope contains Y82, D84, and N86, which have been predicted to be involved in the CD40-CD40 ligand interaction.

SPOTs analysis with mAb CHIR-5.9 showed a weak recognition of peptides represented by spots 20-22 shown in Table 8, suggesting involvement of the region YCDPNLGL (residues 82-89 of the sequence shown in SEQ ID NO:10 or SEQ ID NO:12) in its binding to CD40. It should be noted that the mAbs CHIR-12.12 and CHIR-5.9 compete with each other for binding to CD40 in BIACORE analysis.

**Table 8. Results of SPOTs analysis probing with anti-CD40 monoclonal antibody CHIR-5.9.**

| **Spot Number** | **Sequence Region** |
|---|---|
| 20 | HK**YCDPNLGL** (residues 80-89 of SEQ ID NO:10 or 12) |
| 21 | K**YCDPNLGL**R (residues 81-90 of SEQ ID NO:10 or 12) |
| 22 | **YCDPNLGL**RV (residues 82-91 of SEQ ID NO:10 or 12) |

The linear epitopes identified by the SPOTs analyses are within the CD40 B1 module. The sequence of the CD40 B1 module is:
HKYCDPNLGLRVQQKGTSETDTIC (residues 80-103 of SEQ ID NO:10 or 12).

Within the linear epitope identified for CHIR-12.12 is C83. It is known that this cysteine residue forms a disulphide bond with C103. It is likely that the conformational epitope of the CHIR-12.12 mAb contains this disulfide bond (C83-C103) and/or surrounding amino acids conformationally close to C103.

### Example 8: CHIR-12.12 Blocks CD40L-Mediated CD40 Survival and Signaling Pathways in Normal Human B Cells

Soluble CD40 ligand (CD40L) activates B cells and induces various aspects of functional responses, including enhancement of survival and proliferation, and activation of NFκB, ERK/MAPK, PI3K/Akt, and p38 signaling pathways. In addition, CD40L-mediated CD40 stimulation provides survival signals by reduction of cleaved PARP and induction of the anti-apoptotic proteins, XIAP and Mcl-1, in normal B cells. CD40L-mediated CD40 stimulation also recruits TRAF2 and TRAF3 to bind CD40 cytoplasmic domain.

The following studies demonstrate that CHIR-12.12 directly inhibited all of these stimulation effects on normal human B cells. For example, CHIR-12.12 treatment resulted in increased cleavage of caspase-9, caspase-3, and PARP as well as reduction of XIAP and Mcl-1 in a time- and dose-dependent manner, restoring B cell apoptosis. Treatment with CHIR-12.12 also inhibited phosphorylation of IκB kinase (IKK) α and β (NFκB pathway), ERK, Akt, and p38 in response to CD40L-mediated CD40 stimulation. Further, it was found that CHIR-12.12 did not trigger these apoptotic effects without initial CD40L-mediated CD40 stimulation.

### CHIR-12.12 inhibited survival mediated by CD40 ligand by inducing cleavage of PARP.

In these experiments, 0.6 x 10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0, 20 minutes, 2 hours, 6 hours, 18 hours, and 26 hours. Cleaved caspase-9, cleaved caspase-3, cleaved PARP, and β-action controls were detected in cell lysates by Western blot.

Briefly, it was observed that CD40L-mediated CD40 stimulation provided survival signals as it did not result in increases of cleaved caspase-9, cleaved caspase-3, or cleaved PARP over time, indicating that the cells were not undergoing apoptosis. However, treatment with CHIR-12.12 resulted in an increase of these cleavage products, indicating that CHIR-12.12 treatment abrogated the effects of CD40L binding on survival signaling in sCD40L-stimulated normal B cells, restoring B cell apoptosis (data not shown).

### CHIR-12.12 inhibited expression of "survival" anti-apoptotic proteins.

In these experiments, 0.6 x 10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0, 20 minutes, 2 hours, 6 hours, 18 hours, and 26 hours. Mcl-1, XIAP, CD40, and β-action controls were detected in cell lysates by Western blot. Briefly, sCD40L stimulation resulted in sustained expression of Mcl-1 and XIAP over time. However, treatment of the sCD40L-stimulated cells with CHIR 12.12 resulted in a decrease in expression of these proteins overtime (data not shown). Since Mcl-1 and XIAP are "survival" signals capable of blocking the apoptotic pathway, these results demonstrate that CHIR-12.12 treatment removes the blockade against apoptosis in sCD40L-stimulated normal B cells.

### CHIR-12.12 treatment inhibited phosphorylation of IKKα (Ser180) and IKK β (Ser 181) in normal B cells.

In these experiments, 1.0 x 10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0 and 20 minutes. Phosphorylated IKKα (ser180) and IKKβ (Ser 181) and total IKKβ controls were detected in cell lysates by Western blot.

Briefly, stimulation by sCD40L resulted in phosphorylation of IKKα (Ser180) and IKK β (Ser 181) over time; however, treatment with CHIR-12.12 abrogated this response to sCD40L stimulation in normal B cells (data not shown).

### CHIR-12.12 treatment inhibited survival mediated by CD40 ligand in a dose-dependent manner.

In these experiments, 0.6 x 10⁶ normal human B cells from healthy donors percent purity between 85-95%) were stimulated with 1 µg/ml sCD40L (Alexis Corp., Bingham, Nottinghamshire, UK). CHIR-12.12 (0.01, 0.1, 0.2, 0.5, 1.0 µg/ml) and control IgG were then added. Cells were collected at 24 hours. Cleaved PARP, and β-actin controls were detected in cell lysates by Western blot.

Briefly, CHIR-12.12 treatment resulted in increase of PARP cleavage in sCD40L stimulated cells in a dose-dependent manner and therefore abrogated the survival signaling pathway in sCD40L-stimulated normal B cells (data not shown).

### CHIR-12.12 inhibited expression of "survival" anti-apoptotic proteins in a dose-dependent manner.

In these experiments, 0.6 x 10⁶ normal human B cells from healthy donors (percent purity between 85-95%) were stimulated with 1 µg/ml sCD40L (Alexis Corp., Bingham, Nottinghamshire, UK). CHIR-12.12 (0.5, 2, and 10 µg/ml) and control IgG were then added. Cells were collected at 22 hours. Mcl-1, XIAP, cleaved PARP, and β-actin controls were detected in cell lysates by Western blot.

Briefly, CHIR-12.12 treatment reduced Mcl-1 and XIAP expression and increased cleaved PARP expression in sCD40L-stimulated cells in a dose-dependent manner, and thus abrogated these blockades to the apoptotic pathway in sCD40L-stimulated normal B cells (data not shown).

### CHIR-12.12 did not affect expression of anti-apoptotic proteins, cleaved-PARP, and XIAP, in the absence of soluble CD40L signaling.

In these experiments, 1.0 x 10⁶ normal human B cells from healthy donors (percent purity between 85-95%) were treated with CHIR-12.12 (10 µg/ml) and control IgG only (i.e., cells were not pre-stimulated with sCD40L before adding antibody). Cells were collected at 0, 4, 14, and 16 hours. XIAP, cleaved PARP, and β-actin controls were detected in cell lysates by Western blot.

Briefly, the results show that without sCD40L stimulation, the cells expressed increased concentrations of cleaved PARP, while expression of XIAP remained constant, in both IgG treated control cells and CHIR-12.12 cells (data not shown). These data indicate that CHIR-12.12 does not trigger apoptosis in normal human B cells without CD40L stimulation.

### CHIR-12.12 inhibits phosphorylation of IKKα (Ser180) and IKKβ (Ser181), Akt, ERK, and p38 in normal B cells.

In these experiments, 1.0 x 10⁶ normal human B cells from healthy donors (percent purity between 85-95%) were serum starved in 1% FBS-containing media and stimulated with 1 µg/ml sCD40L (Alexis Corp., Bingham, Nottinghamshire, UK). The cultures were treated with CHIR-12.12 (1 and 10 µg/ml) and control IgG. Cells were collected at 0 and 20 minutes. Phospho-IKKα, phospho-IKKβ, total IKKβ, phospho-ERK, total ERK, phospho-Akt, total Akt, phospho-p38, and total p38 were detected in cell lysates by Western blot.

Briefly, sCD40L stimulation resulted in increases in IKKα/β phosphorylation, ERK phosphorylation, Akt phosphorylation, and p38 phosphorylation, thus leading to survival and or proliferation of the cells. Treatment of the cells with CHIR-12.12 abrogated the effects of sCD40L stimulation on these signaling pathways in normal B cells (data not shown).

### CHIR 12.12 inhibits multiple signaling pathways such as PI3K and MEK/ERK in the CD40 signaling cascade.

In these experiments, 1.0 x 10⁶ normal human B cells from healthy donors (percent purity between 85-95%) were serum starved in 1% FBS-containing media and stimulated with 1 µg/ml sCD40L (Alexis Corp., Bingham, Nottinghamshire, UK). The cultures were also treated with CHIR-12.12 (1 and 10 µg/ml), Wortmanin, (a PI3K/Akt inhibitor; 1 and 10 µM), LY 294002 (a PI3K/Akt inhibitor; 10 and 30 µM), and PD 98095 (a MEK inhibitor; 10 and 30 µg/ml). Cells were collected at 0 and 20 minutes. Phospho-ERK, phospho-Akt, total Akt, phospho-IKKα/β, and total were detected in cell lysates by Western blot.

Briefly, the results show that CHIR-12.12 abrogated the phosphorylation of all of these signal transduction molecules, whereas the signal transduction inhibitors showed only specific abrogation of signaling, indicating that CHIR-12.12 likely inhibits upstream of these signal transduction molecules mediated by CD40L stimulation (data not shown).

### CHIR-12.12 inhibits the binding of signaling molecules TRAF2 and TRAF3 to the cytoplasmic domain of CD40 in normal B cells.

In these experiments, 4.0 x 10⁶ normal human B cells from healthy donors (percent purity between 85-95%) were serum starved for four hours in 1% FBS-containing media and stimulated with 1 µg/ml sCD40L (Alexis Corp., Bingham, Nottinghamshire, UK) for 20 minutes. Cells were collected at 0 and 20 minutes. CD40 was immunoprecipitated using polyclonal anti-CD40 (Santa Cruz Biotechnology, CA), and was probed in a Western blot with anti-TRAF2 mAb (Santa Cruz Biotechnology, CA), anti-TRAF3 mAb (Santa Cruz Biotechnology, CA), and anti-CD40 mAb (Santa Cruz Biotechnology, CA).

Briefly, the results show that TRAF2 and TRAF3 co-precipitated with CD40 after sCD40L stimulation. In contrast, treatment with CHIR-12.12 abrogated formation of the CD40-TRAF2/3 signaling complex in sCD40L-stimulated normal B cells. There were no changes in CD40 expression (data not shown).

Without being bound by theory, the results of these experiments, and the results in the examples outlined above, indicate that the CHIR-12.12 antibody is a dual action antagonist anti-CD40 monoclonal antibody having a unique combination of attributes. This fully human monoclonal antibody blocks CD40L-mediated CD40 signaling pathways for survival and proliferation of B cells; this antagonism leads to ultimate cell death. CHIR-12.12 also mediates recognition and binding by effector cells, initiating antibody dependent cellular cytotoxicity (ADCC). Once CHIR-12.12 is bound to effector cells, cytolytic enzymes are released, leading to B-cell apoptosis and lysis. CHIR-12.12 is a more potent anti-tumor antibody than is rituximab when compared in pre-clinical tumor models.

### Example 9: Liquid Pharmaceutical Formulation for Antagonist Anti-CD40 Antibodies

The objective of this study was to investigate the effects of solution pH on stability of the antagonist anti-CD40 antibody CHIR-12.12 by both biophysical and biochemical methods in order to select the optimum solution environment for this antibody. Differential Scanning Calorimetry (DSC) results showed that the conformation stability of CHIR-12.12 is optimal in formulations having pH 5.5-6.5. Based on a combination of SDS-PAGE, Size-Exclusion HPLC (SEC-HPLC), and Cation-Exchange HPLC (CEX-HPLC) analysis, the physicochemical stability of CHIR-12.12 is optimal at about pH 5.0-5.5. In view of these results, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

### Materials and Methods

The CHIR-12.12 antibody used in the formulation studies is a human monoclonal antibody produced by a CHO cell culture process. This MAb has a molecular weight of 150 kDa and consists of two light chains and two heavy chains linked together by disulfide bands. It is targeted against the CD40 cell surface receptor on CD40-expressing cells, including normal and malignant B cells, for treatment of various cancers and autoimmune/inflammatory diseases.

The anti-CD40 drug substance used for this study was a CHO-derived purified anti-CD40 (CHIR-12.12) bulk lot. The composition of the drug substance was 9.7 mg/ml CHIR-12.12 antibody in 10 mM sodium citrate, 150 mM sodium chloride, at pH 6.5. The control sample in the study was the received drug substance, followed by freezing at ≤ -60°C, thawing at RT and testing along with stability samples at predetermined time points. The stability samples were prepared by dialysis of the drug substance against different pH solutions and the CHIR-12.12 concentration in each sample was determined by UV 280 as presented in Table 9.

**Table 9. CHIR-12.12 formulations.**

| Buffer Composition | pH | CHIR-12.12 |
|---|---|---|
| | | Concentration ( mg/ml) |
| 10 mM sodium citrate, 150 mM sodium chloride | 4.5 | 9.0 |
| 10 mM sodium succinate, 150 mM sodium chloride | 5.0 | 9.3 |
| 10 mM sodium succinate, 150 mM sodium chloride | 5.5 | 9.2 |
| 10 mM sodium citrate, 150 mM sodium chloride | 6.0 | 9.7 |
| 10 mM sodium citrate, 150 mM sodium chloride | 6.5 | 9.4 |
| 10 mM sodium phosphate, 150 mM sodium chloride | 7.0 | 9.4 |
| 10 mM sodium phosphate, 150 mM sodium chloride | 7.5 | 9.5 |
| 10 mM glycine, 150 mM sodium chloride | 9.0 | 9.5 |

Physicochemical stability of the CHIR-12.12 antibody in the various formulations was assayed using the following protocols.

### Differential Scanning Calorimetry (DSC

Conformational stability of different formulation samples was monitored using a MicroCal VP-DSC upon heating 15°C to 90°C at 1°C/min.

### SDS-PAGE

Fragmentation and aggregation were estimated using 4-20% Tris-Glycine Gel under non-reducing and reducing conditions. Protein was detected by Coomassie blue staining.

### Size Exclusion Chromatograph (SEC-HPLC)

Protein fragmentation and aggregation were also measured by a Water Alliance HPLC with a Tosohaas TSK-GEL 3000SWXL column, 100 mM sodium phosphate, pH 7.0 as mobile phase at a flow rate of 0.7 ml/min.

### Cation Exchange Chromatography (CEX-HPLC)

Charge change related degradation was measured using Waters 600s HPLC system with a Dionex Propac WCX-10 column, 50 mM HEPEs, pH 7.3 as mobile phase A and 50 mM HEPES containing 500 mM NaCl, pH 7.3 as mobile phase B at a flow rate of 0.5°C/min.

### Results and Discussion

### Conformational stability study.

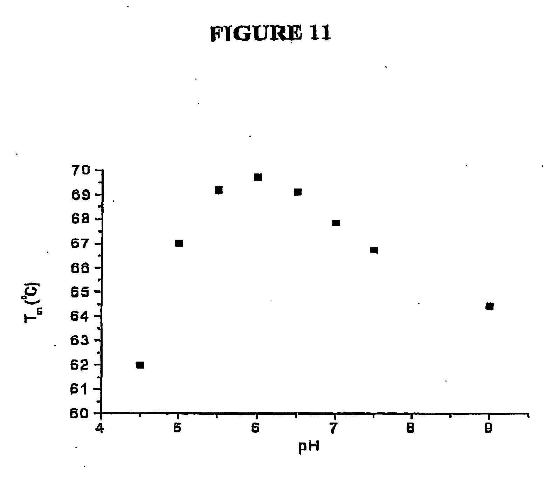

Thermal unfolding of CHIR-12.12 revealed at least two thermal transitions, probably representing unfolding melting of the Fab and the Fc domains, respectively. At higher temperatures, the protein presumably aggregated, resulting in loss of DSC signal. For the formulation screening purpose, the lowest thermal transition temperature was defined as the melting temperature, Tm, in this study. Figure 11 shows the thermal melting temperature as a function of formulation pHs. Formulations at pH 5.5-6.5 provided anti-CD40 with higher conformational stability as demonstrated by the higher thermal melting temperatures.

### SDS-PAGE analysis.

The CHIR-12.12 formulation samples at pH 4.5-9.0 were incubated at 40°C for 2 months and subjected to SDS-PAGE analysis (data not shown). Under non-reducing conditions, species with molecular weight (MW) of 23 kDa and 27 kDa were observed in formulations above pH 5.5, and species with MW of 51 kDa were observed in all formulations, but appeared less at pH 5.0-5.5. A species with MW of 100 kDa could be seen at pH 7.5 and pH 9.0.

Under reducing conditions, CHIR-12.12 was reduced into free heavy chains and light chains with MW of 50 kDa and 24 kDa, respectively. The 100 kDa species seemed not fully reducible and increased with increasing solution pH, suggesting non-disulfide covalent association might occur in the molecules. Since there were other species with unknown identities on SDS-PAGE, stability comparison of each formulation is based on the remaining purity of CHIR-12.12. Formulations at pH 5.0-6.0 provided a more stable environment to CHIR-12.12. Few aggregates were detected by SDS-PAGE (data not shown).

### SEC-HPLC analysis.

SEC-HPLC analysis detected the intact CHIR-12.12 as the main peak species, an aggregation species as a front peak species separate from the main peak species, a large fragment species as a shoulder peak on the back of the main peak species, and small fragment species were detected post-main peak species. After incubation at 5°C and 25°C for 3 months, negligible amounts of protein fragments and aggregates (<1.0%) were detected in the above formulations and the CHIR-12.12 main peak species remained greater than 99% purity (data not shown). However, protein fragments gradually developed upon storage at 40°C and more fragments formed at pH 4.5 and pH 6.5-9.0, as shown in Table 10. After incubating the CHIR-12.12 formulations at 40°C for 3 months, about 2-3% aggregates were detected in pH 7.5 and pH 9.0, while less than 1% aggregates were detected in other pH formulations (data not shown). The SEC-HPLC results indicate CHIR-12.12 is more stable at about pH 5.0-6.0.

**Table 10. SEC-HPLC results of CHIR-12.12 stability samples under real-time and accelerated storage conditions.**

| Sample | Main peak % | | | | Fragments % | | | |
|---|---|---|---|---|---|---|---|---|
| | t=0 | 40°C | 40°C | 40°C | t=0 | 40°C | 40°C | 40°C |
| | | 1 m | 2m | 3m | | 1 m | 2m | 3m |
| Control | 99.4 | 99.2 | 99.9 | 99.5 | <1.0 | <1.0 | <1.0 | <1.0 |
| pH 4.5 | 99.4 | 93.2 | 86.0 | 81.3 | <1.0 | 6.4 | 13.2 | 18.1 |
| pH 5.0 | 99.8 | 98.7 | 91.3 | 89.2 | <1.0 | <1.0 | 7.8 | 10.2 |
| pH 5.5 | 99.8 | 98.9 | 91.4 | 90.6 | <1.0 | <1.0 | 7.6 | 8.8 |
| pH 6.0 | 99.6 | 97.7 | 90.4 | 87.3 | <1.0 | 1.9 | 8.2 | 11.7 |
| pH 6.5 | 99.3 | 93.4 | 89.0 | 86.9 | <1.0 | 5.6 | 9.9 | 12.4 |
| pH 7.0 | 99.2 | 93.9 | 87.4 | 85.1 | <1.0 | 5.5 | 11.1 | 13.5 |
| pH 7.5 | 99.1 | 92.8 | 84.4 | 81.9 | <1.0 | 6.4 | 12.9 | 16.2 |
| pH 9.0 | 99.3 | 82.4 | 61.6 | 50.6 | <1.0 | 15.4 | 36.2 | 47.6 |

### CEX-HPLC analysis.

CEX-HPLC analysis detected the intact CHIR-12.12 as the main peak species, acidic variants eluted earlier than the main peak species, and C-terminal lysine addition variants eluted post-main peak species. Table 11 shows the dependence of the percentages of the remaining main peak CHIR-12.12 species and acidic variants on solution pH. The control sample already contained a high degree of acidic species (∼33%), probably due to early-stage fermentation and purification processes. The susceptibility of CHIR-12.12 to higher pH solutions is evidenced by two facts. First, the initial formulation sample at pH 9.0 (t=0) already generated 12% more acidic species than the control. Second, the percentage of acidic species increased sharply with increasing pH. The charge change-related degradation is likely due to deamidation. The above data indicate that this type of degradation of CHIR-12.12 was minimized at about pH 5.0-5.5.

**Table 11. Percentage of peak area by CEX-HPLC for CHIR-12.12 in different pH formulations under real-time and accelerated storage conditions.**

| Sample | Main peak % | | | | | Acidic variants % | | | | |
|---|---|---|---|---|---|---|---|---|---|---|
| | t=0 | 5°C | 25°C | 40°C | 40°C | t=0 | 5°C | 25°C | 40°C | 40°C |
| | | 3m | 3 m | 1 m | 2 m | | 3m | 3 m | 1 m | 2 m |
| Control | 49.2 | 49.8 | 49.8 | 49.2 | 50.3 | 32.0 | 33.7 | 33.7 | 32.0 | 33.6 |
| pH 4.5 | 48.5 | 49.7 | 43.7 | 39.7 | 30.0 | 32.5 | 32.6 | 38.0 | 44.2 | 56.4 |
| pH 5.0 | 49.6 | 49.8 | 48.3 | 40.6 | 31.4 | 32.7 | 31.8 | 35.0 | 44.3 | 57.1 |
| pH 5.5 | 50.7 | 50.3 | 48.1 | 40.0 | 30.2 | 32.6 | 31.8 | 37.8 | 48.9 | 63.3 |
| pH 6.0 | 50.2 | 49.9 | 47.9 | 37.4 | 23.9 | 33.1 | 33.6 | 38.5 | 54.9 | 72.7 |
| pH 6.5 | 49.4 | 49.9 | 42.3 | 29.7 | 14.6 | 33.3 | 33.6 | 47.7 | 65.2 | 84.6 |
| pH 7.0 | 49.7 | 49.9 | 21.9 | - | - | 34.4 | 36.4 | 64.4 | - | - |
| pH 7.5 | 49.3 | 48.3 | 12.7 | - | - | 35.5 | 40.1 | 79.2 | - | - |
| pH 9.0 | 41.3 | 31.8 | - | - | - | 44.7 | 59.9 | - | - | - |

### Conclusion

The pH has a significant effect on conformational and physicochemical stabilities of CHIR-12.12. Charge change-related degradation was determined to be the main degradation pathway for CHIR-12.12, which was minimized at pH 5.0-5.5. Based on overall stability data, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

### Example 10: Clinical Studies with CHIR-5.9 and CHIR-12.12

### Clinical Objectives

The overall objective is to provide an effective therapy for solid tumors comprising CD40-expressing carcinoma cells by targeting them with an anti-CD40 IgG1. These tumors include lung, breast, colon, ovarian, and skin carcinomas. The signal for these diseases is determined in phase II although some measure of activity may be obtained in phase I. Initially the agent is studied as a single agent, but will be combined with other agents, chemotherapeutics, and other antibodies, as development proceeds.

### Phase I

- Evaluate safety and pharmacokinetics - dose escalation in subjects with above-mentioned solid tumors.
- Choose dose based on safety, tolerability, and change in serum markers of CD40. In general an MTD is sought but other indications of efficacy (depletion of CD40+ tumor cells, etc.) may be adequate for dose finding.
- Consideration of more than one dose especially for different indications, e.g., the breast cancer dose may be different than the ovarian cancer dose. Thus, some dose finding may be necessary in phase II.
- Patients are dosed weekly with real-time pharmacokinetic (Pk) sampling.
   Initially a 4-week cycle is the maximum dosing allowed. The Pk may be highly variable depending on the disease studied, density of CD40 etc.
- This trial(s) is open to subjects with CD40-expressing solid tumors, including lung, breast, colon, ovarian, and skin carcinomas.
- Decision to discontinue or continue studies is based on safety, dose, and preliminary evidence of anti-tumor activity.
- Activity of drug as determined by response rate is determined in Phase II.
- Identify dose(s) for Phase II.

### Phase II

Several trials will be initiated in the above-mentioned tumor types with concentration on lung, ovarian, and breast cancer. More than one dose, and more than one schedule may be tested in a randomized phase II setting.

In each disease, target a population that has failed current standard of care:
- Lung: surgery, radiation therapy, chemotherapy
- Ovarian: surgery, radiation therapy, chemotherapy
- Breast: surgery, radiation therapy, chemotherapy, hormone therapy

✔ Decision to discontinue or continue with study is based on proof of therapeutic concept in Phase II
✔ Determine whether surrogate marker can be used as early indication of clinical efficacy
✔ Identify doses for Phase III

### Phase III

Phase III will depend on where the signal is detected in phase II, and what competing therapies are considered to be the standard. If the signal is in a stage of disease where there is no standard of therapy, then a single arm, well-controlled study could serve as a pivotal trial. If there are competing agents that are considered standard, then head-to-head studies are conducted.

## Claims

1. A human anti-CD40 monoclonal antibody that is capable of specifically binding to CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay,
for use in treating a human subject for a solid tumor comprising carcinoma cells expressing CD40 antigen.

2. Use of an effective amount of a human anti-CD40 monoclonal antibody that is capable of specifically binding to CD40 antigen in the manufacture of a medicament for treating a human subject for a solid tumor comprising carcinoma cells expressing CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay.

3. An *in vitro* method for inhibiting the growth of a solid tumor comprising carcinoma cells expressing CD40 antigen, said method comprising contacting said cells with an effective amount of a human anti-CD40 monoclonal antibody that is capable of specifically binding to said CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay

4. A human anti-CD40 monoclonal antibody that is capable of specifically binding to CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay,
for use in inhibiting the growth of a solid tumor comprising carcinoma cells expressing CD40 antigen.

5. Use of an effective amount of a human anti-CD40 monoclonal antibody that is capable of specifically binding to CD40 antigen in the manufacture of a medicament for inhibiting the growth of a solid tumor comprising carcinoma cells expressing CD40 antigen, said monoclonal antibody being free of significant agonist activity when bound to CD40 antigen, wherein said antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542, or the monoclonal antibody CHIR-12.12 obtainable from the hybridoma cell line deposited with the ATCC as Patent Deposit-No. PTA-5543;
b) a monoclonal antibody that binds to an epitope comprising residues 82-87 of the human CD40 sequence shown in SEQ ID NO: 10 or SEQ ID NO: 12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543, in a competitive binding assay.

6. The antibody, use or method of any of claims 1-5, wherein said antibody or antigen-binding fragment thereof is selected from the group consisting of:
(i) an antibody or antigen-binding fragment thereof comprising a light chain variable domain containing residues 44-54, 70-76 and 109-117 of SEQ ID NO:2 or SEQ ID NO:6;
(ii) an antibody or antigen-binding fragment thereof comprising a heavy chain variable domain containing residues 50-54, 69-84 and 114-121 of SEQ ID NO:4 or SEQ ID NO:7;
(iii) an antibody or antigen-binding fragment thereof comprising a light chain variable domain containing residues 46-52, 70-72 and 111-116 of SEQ ID NO:2 or SEQ ID NO:6; and
(iv) an antibody or antigen-binding fragment thereof comprising a heavy chain variable domain containing residues 45-51, 72-74, 115-120 of SEQ ID NO:4 or SEQ ID NO:7.

7. The antibody, use or method of any of claims 1-5, wherein said antibody comprises an amino acid sequence selected from the group consisting of:
(i) residues 21-132 of SEQ ID NO:2;
(ii) residues 21-239 of SEQ ID NO:2;
(iii) SEQ ID NO:2;
(iv) residues 20-139 of SEQ ID NO:4;
(v) residues 20-469 of SEQ ID NO:4;
(vi) SEQ ID NO:4;
(vii) residues 20-469 of SEQ ID NO:5;
(viii) SEQ ID NO:5;
(ix) residues 21-132 of SEQ ID NO:2 and residues 20-139 of SEQ ID NO:4;
(x) residues 21-239 of SEQ ID NO:2 and residues 20-469 of SEQ ID NO:4;
(xi) residues 21-239 of SEQ ID NO:2 and residues 20-469 of SEQ ID NO:5;
(xii) SEQ ID NO:2 and SEQ ID NO:4; and
(xiii) SEQ ID NO:2 and SEQ ID NO:5.

8. The antibody, use or method of any of claims 1-5, wherein said antibody or antigen-binding fragment thereof comprises an amino acid sequence selected from the group consisting of:
(i) residues 21-132 of SEQ ID NO:6;
(ii) residues 21-239 of SEQ ID NO:6;
(iii) SEQ ID NO:6;
(iv) residues 20-144 of SEQ ID NO:7;
(v) residues 20-474 of SEQ ID NO:7;
(vi) SEQ ID NO:7;
(vii) residues 20-474 of SEQ ID NO:8;
(viii) SEQ ID NO:8;
(ix) residues 21-132 of SEQ ID NO:6 and residues 20-144 of SEQ ID NO:7;
(x) residues 21-239 of SEQ ID NO:6 and residues 20-474 of SEQ ID NO:7;
(xi) residues 21-239 of SEQ ID NO:6 and residues 20-474 of SEQ ID NO:8
(xii) SEQ ID NO:6 and SEQ ID NO:7; and
(xiii) SEQ ID NO:6 and SEQ ID NO:8.

9. The antibody, use or method of any preceding claim, wherein said antibody is the monoclonal antibody CHIR-5.9 obtainable from the hybridoma cell line deposited with the ATCC as Patent Deposit No. PTA-5542 or CHIR-12.12 obtainable from the hybridoma cell line deposited with the ATCC as Patent Deposit No. PTA-5543.

10. The antibody, use or method of any preceding claim, wherein said solid tumor is selected from the group consisting of lung carcinoma, breast carcinoma, ovarian carcinoma, skin carcinoma, colon carcinoma, urinary bladder carcinoma, liver carcinoma, gastric carcinoma, prostate cancer, renal cell carcinoma, nasopharyngeal carcinoma, squamous cell carcinoma, thyroid papillary carcinoma, cervical carcinoma, and sarcomas.

11. The antibody, use or method of claim 10, wherein said medicament is administered in a method further comprising administering to said subject at least one other cancer therapy protocol selected from the group consisting of surgery, radiation therapy, chemotherapy, cytokine therapy, and other monoclonal antibody intended for use in treatment of said solid tumor.

12. The antibody, use or method of any of claims 1-5, wherein said monoclonal antibody is an antigen-binding fragment of a monoclonal antibody, wherein said fragment retains the capability of specifically binding to said human CD40 antigen.

13. The antibody, use or method of claim 12, wherein said antigen-binding antibody fragment is selected from the group consisting of a Fab fragment, an F(ab')₂ fragment, an Fv fragment, and a single-chain Fv fragment.

14. The antibody, use or method of any preceding claim, wherein said monoclonal antibody or antigen-binding fragment thereof binds to human CD40 antigen with an affinity (K_{D}) of at least 10⁻⁶ M.

15. The antibody, use or method of claim 14, wherein said monoclonal antibody or antigen-binding fragment thereof binds to human CD40 antigen with an affinity (K_{D}) of at least 10⁻⁸ M.

16. The antibody, use or method of any preceding claim, wherein said monoclonal antibody or antigen-binding fragment thereof is produced in a CHO cell line.

## Patentansprüche

1. Humaner monoklonaler Anti-CD40-Antikörper, der in der Lage ist, spezifisch an CD40-Antigen zu binden, wobei der monoklonale Antikörper frei von signifikanter agonistischer Aktivität ist, wenn er an CD40-Antigen gebunden ist, und wobei der Antikörper ausgewählt ist aus der Gruppe bestehend aus:
(a) einem monoklonalen Antikörper, der an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem monoklonalen Antikörper, der an ein Epitop bindet, welches die Reste 82-87 der in SEQ ID NO:10 oder SEQ ID NO:12 gezeigten humanen CD40-Sequenz umfasst;
(c) einem monoklonalen Antikörper, der an ein Epitop bindet, welches die Reste 82-89 der in SEQ ID NO:10 oder SEQ ID NO:12 gezeigten humanen CD40-Sequenz umfasst; und
(d) einem monoklonalen Antikörper, der mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert,
zur Verwendung bei der Behandlung eines humanen Subjekts im Hinblick auf einen soliden Tumor, der Karzinomzellen umfasst, welche CD40-Antigen exprimieren.

2. Verwendung einer wirksamen Menge eines humanen monoklonalen Anti-CD40-Antikörpers, der in der Lage ist, spezifisch an CD40-Antigen zu binden, bei der Herstellung eines Medikaments zur Behandlung eines humanen Subjekts im Hinblick auf einen soliden Tumor, der Karzinomzellen umfasst, welche CD40-Antigen exprimieren, wobei der monoklonale Antikörper frei von signifikanter agonistischer Aktivität ist, wenn er an das CD40-Antigen gebunden ist, wobei der Antikörper ausgewählt ist aus der Gruppe bestehend aus:
(a) einem monoklonalen Antikörper, der an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem monoklonalen Antikörper, der an ein Epitop bindet, welches die Reste 82-87 der in SEQ ID NO:10 oder SEQ ID NO:12 gezeigten humanen CD40-Sequenz umfasst;
(c) einem monoklonalen Antikörper, der an ein Epitop bindet, welches die Reste 82-89 der in SEQ ID NO:10 oder SEQ ID NO:12 gezeigten humanen CD40-Sequenz umfasst;
und
(d) einem monoklonalen Antikörper, der mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert.

3. *In vitro* Verfahren zur Inhibierung des Wachstums eines soliden Tumors, der Karzinomzellen umfasst, welche CD40-Antigen exprimieren, wobei das Verfahren das Inkontaktbringen der Zellen mit einer wirksamen Menge eines humanen monoklonalen Anti-CD40-Antikörpers umfasst, welcher in der Lage ist, spezifisch an das CD40-Antigen zu binden, wobei der monoklonale Antikörper frei von signifikanter agonistischer Aktivität ist, wenn er an CD40-Antigen gebunden ist, und wobei der Antikörper ausgewählt ist aus der Gruppe bestehend aus:
(a) einem monoklonalen Antikörper, der an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem monoklonalen Antikörper, der an ein Epitop bindet, welches die Reste 82-87 der in SEQ ID NO:10 oder SEQ ID NO:12 gezeigten humanen CD40-Sequenz umfasst;
(c) einem monoklonalen Antikörper, der an ein Epitop bindet, welches die Reste 82-89 der in SEQ ID NO:10 oder SEQ ID NO:12 gezeigten humanen CD40-Sequenz umfasst; und
(d) einem monoklonalen Antikörper, der mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert.

4. Humaner monoklonaler Anti-CD40-Antikörper, der in der Lage ist, spezifisch an CD40-Antigen zu binden, wobei der monoklonale Antikörper frei von signifikanter agonistischer Aktivität ist, wenn er an CD40-Antigen gebunden ist, und wobei der Antikörper ausgewählt ist aus der Gruppe bestehend aus:
(a) einem monoklonalen Antikörper, der an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem monoklonalen Antikörper, der an ein Epitop bindet, welches die Reste 82-87 der in SEQ ID NO:10 oder SEQ ID NO:12 gezeigten humanen CD40-Sequenz umfasst;
(c) einem monoklonalen Antikörper, der an ein Epitop bindet, welches die Reste 82-89 der in SEQ ID NO:10 oder SEQ ID NO:12 gezeigten humanen CD40-Sequenz umfasst; und
(d) einem monoklonalen Antikörper, der mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert,
zur Verwendung bei der Inhibierung des Wachstums eines soliden Tumors, der Karzinomzellen umfasst, welche CD40-Antigen exprimieren.

5. Verwendung einer wirksamen Menge eines humanen monoklonalen Anti-CD40-Antiktirpers, der in der Lage ist, spezifisch an CD40-Antigen zu binden, bei der Herstellung eines Medikaments zur Inhibierung des Wachstums eines soliden Tumors, der Karzinomzellen umfasst, welche CD40-Antigen exprimieren, wobei der monoklonale Antikörper frei von signifikanter agonistischer Aktivität ist, wenn er an CD40-Antigen gebunden ist, und wobei der Antikörper ausgewählt ist aus der Gruppe bestehend aus:
(a) einem monoklonalen Antikörper, der an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem monoklonalen Antikörper, der an ein Epitop bindet, welches die Reste 82-87 der in SEQ ID NO:10 oder SEQ ID NO:12 gezeigten humanen CD40-Sequenz umfasst;
(c) einem monoklonalen Antikörper, der an ein Epitop bindet, welches die Reste 82-89 der in SEQ ID NO:10 oder SEQ ID NO:12 gezeigten humanen CD40-Sequenz umfasst;
und
(d) einem monoklonalen Antikörper, der mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert.

6. Antikörper, Verwendung oder Verfahren nach einem der Ansprüche 1-5, wobei der Antikörper oder ein Antigenbindendes Fragment davon ausgewählt ist aus der Gruppe bestehend aus:
(i) einem Antikörper oder einem Antigen-bindenden Fragment davon, der/das eine variable Domäne einer leichten Kette umfasst, welche die Reste 44-54, 70-76 und 109-117 von SEQ ID NO:2 oder SEQ ID NO:6 enthält;
(ii) einem Antikörper oder einem Antigen-bindenden Fragment davon, der/das eine variable Domäne einer schweren Kette umfasst, welche die Reste 50-54, 69-84 und 114-121 von SEQ ID NO:4 oder SEQ ID NO:7 enthält;
(iii) einem Antikörper oder einem Antigen-bindenden Fragment davon, der/das eine variable Domäne einer leichten Kette umfasst, welche die Reste 46-52, 70-72 und 111-116 von SEQ ID NO:2 oder SEQ ID NO:6 enthält; und
(iv) einem Antikörper oder einem Antigen-bindenden Fragment davon, der/das eine variable Domäne einer schweren Kette umfasst, welche die Reste 45-51, 72-74 und 115-120 von SEQ ID NO:4 oder SEQ ID NO:7 umfasst.

7. Antikörper, Verwendung oder Verfahren nach einem der Ansprüche 1-5, wobei der Antikörper eine Aminosäuresequenz umfasst, die ausgewählt ist aus der Gruppe bestehend aus:
(i) den Resten 21-132 von SEQ ID NO:2;
(ii) den Resten 21-239 von SEQ ID NO:2;
(iii) SEQ ID NO:2;
(iv) den Resten 20-139 von SEQ ID NO:4;
(v) den Resten 20-469 von SEQ ID NO:4;
(vi) SEQ ID NO:4;
(vii) den Resten 20-469 von SEQ ID NO:5;
(viii) SEQ ID NO:5;
(ix) den Resten 21-132 von SEQ ID NO:2 und den Resten 20-139 von SEQ ID NO:4;
(x) den Resten 21-239 von SEQ ID NO:2 und den Resten 20-469 von SEQ ID NO:4;
(xi) den Resten 21-239 von SEQ ID NO:2 und den Resten 20-469 von SEQ ID NO:5;
(xii) SEQ ID NO:2 und SEQ ID NO:4; und
(xiii) SEQ ID NO:2 und SEQ ID NO:5.

8. Antikörper, Verwendung oder Verfahren nach einem der Ansprüche 1-5, wobei der Antikörper oder das Antigen-bindende Fragment davon eine Aminosäuresequenz umfasst, die ausgewählt ist aus der Gruppe bestehend aus:
(i) den Resten 21-132 von SEQ ID NO:6;
(ii) den Resten 21-239 von SEQ ID NO:6;
(iii) SEQ ID NO:6;
(iv) den Resten 20-144 von SEQ ID NO:7;
(v) den Resten 20-474 von SEQ ID NO:7;
(vi) SEQ ID NO:7;
(vii) den Resten 20-474 von SEQ ID NO:8;
(viii) SEQ ID NO:8;
(ix) den Resten 21-132 von SEQ ID NO:6 und den Resten 20-144 von SEQ ID NO:7;
(x) den Resten 21-239 von SEQ ID NO:6 und den Resten 20-474 von SEQ ID NO:7;
(xi) den Resten 21-239 von SEQ ID NO:6 und den Resten 20-474 von SEQ ID NO:8;
(xii) SEQ ID NO:6 und SEQ ID NO:7; und
(xiii) SEQ ID NO:6 und SEQ ID NO:8.

9. Antikörper, Verwendung oder Verfahren nach einem der vorhergehenden Ansprüche, wobei der Antikörper der monoklonale Antikörper CHIR-5.9 ist, welcher von der Hybridom-Zelllinie erhältlich ist, die bei der ATCC als Patent-Hinterlegungsnummer PTA-5542 hinterlegt ist, oder der monoklonale Antikörper CHIR-12.12, welcher von der Hybridom-Zelllinie erhältlich ist, die bei der ATCC als Patent-Hinterlegungsnummer PTA-5543 hinterlegt ist.

10. Antikörper, Verwendung oder Verfahren nach einem der vorhergehenden Ansprüche, wobei der solide Tumor ausgewählt ist aus der Gruppe bestehend aus Lungenkarzinom, Brustkarzinom, Ovarkarzinom, Hautkarzinom, Kolonkarzinom, Harnblasenkarzinom, Leberkarzinom, Magenkarzinom, Prostatakrebs, Nierenzellkarzinom, nasopharyngales Karzinom, Plattenepithelkarzinom, Schilddrüsenpapillarkarzinom, Gebärmutterhalskrebs und Sarkoma.

11. Antikörper, Verwendung oder Verfahren nach Anspruch 10, wobei das Medikament in einem Verfahren verabreicht wird, welches ferner die Verabreichung von mindestens einem weiteren Krebstherapie-Protokoll an das Subjekt umfasst, wobei dieses ausgewählt ist aus der Gruppe bestehend aus Operation, Strahlentherapie, Chemotherapie, Cytokintherapie und einem anderen monoklonalen Antikörper, der für die Verwendung bei der Behandlung des soliden Tumors vorgesehen ist.

12. Antikörper, Verwendung oder Verfahren nach einem der Ansprüche 1-5, wobei der monoklonale Antikörper ein Antigenbindendes Fragment eines monoklonalen Antikörpers ist, und wobei das Fragment die Fähigkeit beibehält, spezifisch an das humane CD40-Antigen zu binden.

13. Antikörper, Verwendung oder Verfahren nach Anspruch 12, wobei das Antigen-bindende Antikörper-Fragment ausgewählt ist aus der Gruppe bestehend aus einem Fab-Fragment, einem F(ab')₂ Fragment, einem Fv-Fragment und einem einzelkettigem Fv-Fragment.

14. Antikörper, Verwendung oder Verfahren nach einem der vorhergehenden Ansprüche, wobei der monoklonale Antikörper oder das Antigen-bindende Fragment davon mit einer Affinität (K_{D}) von mindestens 10⁻⁶ M an das humane CD40-Antigen bindet.

15. Antikörper, Verwendung oder Verfahren nach Anspruch 14, wobei der monoklonale Antikörper oder das Antigen-bindende Fragment davon mit einer Affinität (K_{D}) von mindestens 10⁻⁸ M an das humane CD40-Antigen bindet.

16. Antikörper, Verwendung oder Verfahren nach einem der vorhergehenden Ansprüche, wobei der monoklonale Antikörper der das Antigen-bindende Fragment davon in einer CHO-Zelllinie produziert wird.

## Revendications

1. Anticorps monoclonal anti-CD40 humain qui est capable de se lier spécifiquement à l'antigène CD40, ledit anticorps monoclonal ne présentant pas d'activité agoniste significative lorsqu'il est lié à l'antigène CD40, ledit anticorps étant choisi dans le groupe constitué par :
a) un anticorps monoclonal qui se lie à un épitope capable de se lier à l'anticorps monoclonal CHIR-5.9 pouvant être obtenu à partir de la lignée cellulaire d'hybridomes déposée auprès de l'ATCC sous le No. de dépôt de brevet PTA-5542, ou à l'anticorps monoclonal CHIR-12.12 pouvant être obtenu à partir de la lignée cellulaire d'hybridomes déposée auprès de l'ATCC sous le No. de dépôt de brevet PTA-5543 ;
b) un anticorps monoclonal qui se lie à un épitope comprenant les résidus 82 à 87 de la séquence du CD40 humain montrée dans la SÉQ ID No. : 10 ou la SÉQ ID No. : 12 ;
c) un anticorps monoclonal qui se lie à un épitope comprenant les résidus 82 à 89 de la séquence du CD40 humain montrée dans la SÉQ ID No. : 10 ou la SÉQ ID No. : 12 ; et
d) un anticorps monoclonal qui est en compétition avec l'anticorps monoclonal CHIR-5.9 pouvant être obtenu à partir de la lignée cellulaire d'hybridomes déposée auprès de l'ATCC sous le No. de dépôt de brevet PTA-5542 ou avec l'anticorps monoclonal CHIR-12.12 pouvant être obtenu à partir de la lignée cellulaire d'hybridomes déposée auprès de l'ATCC sous le No. de dépôt de brevet PTA-5543, dans un dosage par liaison compétitive,
destiné à être utilisé dans le traitement d'une tumeur solide chez un sujet humain, ladite tumeur comprenant des cellules d'un carcinome exprimant l'antigène CD40.

2. Utilisation d'une quantité efficace d'un anticorps monoclonal anti-CD40 humain qui est capable de se lier spécifiquement à l'antigène CD40 dans la fabrication d'un médicament destiné à traiter une tumeur solide chez un sujet humain, ladite tumeur comprenant des cellules d'un carcinome exprimant l'antigène CD40, ledit anticorps monoclonal ne présentant pas d'activité agoniste significative lorsqu'il est lié à l'antigène CD40, ledit anticorps étant choisi dans le groupe constitué par :
a) un anticorps monoclonal qui se lie à un épitope capable de se lier à l'anticorps monoclonal CHIR-5.9 pouvant être obtenu à partir de la lignée cellulaire d'hybridomes déposée auprès de l'ATCC sous le No. de dépôt de brevet PTA-5542, ou à l'anticorps monoclonal CHIR-12.12 pouvant être obtenu à partir de la lignée cellulaire d'hybridomes déposée auprès de l'ATCC sous le No. de dépôt de brevet PTA-5543 ;
b) un anticorps monoclonal qui se lie à un épitope comprenant les résidus 82 à 87 de la séquence du CD40 humain montrée dans la SÉQ ID No. : 10 ou la SÉQ ID No. : 12 ;
c) un anticorps monoclonal qui se lie à un épitope comprenant les résidus 82 à 89 de la séquence du CD40 humain montrée dans la SÉQ ID No. : 10 ou la SÉQ ID No. : 12 ; et
d) un anticorps monoclonal qui est en compétition avec l'anticorps monoclonal CHIR-5.9 pouvant être obtenu à partir de la lignée cellulaire d'hybridomes déposée auprès de l'ATCC sous le No. de dépôt de brevet PTA-5542 ou avec l'anticorps monoclonal CHIR-12.12 pouvant être obtenu à partir de la lignée cellulaire d'hybridomes déposée auprès de l'ATCC sous le No. de dépôt de brevet PTA-5543, dans un dosage par liaison compétitive.

3. Méthode in vitro d'inhibition de la croissance d'une tumeur solide comprenant des cellules d'un carcinome exprimant l'antigène CD40, ladite méthode comprenant la mise en contact desdites cellules avec une quantité efficace d'un anticorps monoclonal anti-CD40 humain qui est capable de se lier spécifiquement audit antigène CD40, ledit anticorps monoclonal ne présentant pas d'activité agoniste significative lorsqu'il est lié à l'antigène CD40, ledit anticorps étant choisi dans le groupe constitué par :
a) un anticorps monoclonal qui se lie à un épitope capable de se lier à l'anticorps monoclonal CHIR-5.9 pouvant être obtenu à partir de la lignée cellulaire d'hybridomes déposée auprès de l'ATCC sous le No. de dépôt de brevet PTA-5542, ou à l'anticorps monoclonal CHIR-12.12 pouvant être obtenu à partir de la lignée cellulaire d'hybridomes déposée auprès de l'ATCC sous le No. de dépôt de brevet PTA-5543 ;
b) un anticorps monoclonal qui se lie à un épitope comprenant les résidus 82 à 87 de la séquence du CD40 humain montrée dans la SÉQ ID No. : 10 ou la SÉQ ID No. : 12 ;
c) un anticorps monoclonal qui se lie à un épitope comprenant les résidus 82 à 89 de la séquence du CD40 humain montrée dans la SÉQ ID No. : 10 ou la SÉQ ID No. : 12 ; et
d) un anticorps monoclonal qui est en compétition avec l'anticorps monoclonal CHIR-5.9 pouvant être obtenu à partir de la lignée cellulaire d'hybridomes déposée auprès de l'ATCC sous le No. de dépôt de brevet PTA-5542 ou avec l'anticorps monoclonal CHIR-12.12 pouvant être obtenu à partir de la lignée cellulaire d'hybridomes déposée auprès de l'ATCC sous le No. de dépôt de brevet PTA-5543, dans un dosage par liaison compétitive.

4. Anticorps monoclonal anti-CD40 humain qui est capable de se lier spécifiquement à l'antigène CD40, ledit anticorps monoclonal ne présentant pas d'activité agoniste significative lorsqu'il est lié à l'antigène CD40, ledit anticorps étant choisi dans le groupe constitué par :
a) un anticorps monoclonal qui se lie à un épitope capable de se lier à l'anticorps monoclonal CHIR-5.9 pouvant être obtenu à partir de la lignée cellulaire d'hybridomes déposée auprès de l'ATCC sous le No. de dépôt de brevet PTA-5542, ou à l'anticorps monoclonal CHIR-12.12 pouvant être obtenu à partir de la lignée cellulaire d'hybridomes déposée auprès de l'ATCC sous le No. de dépôt de brevet PTA-5543 ;
b) un anticorps monoclonal qui se lie à un épitope comprenant les résidus 82 à 87 de la séquence du CD40 humain montrée dans la SÉQ ID No. : 10 ou la SÉQ ID No. : 12 ;
c) un anticorps monoclonal qui se lie à un épitope comprenant les résidus 82 à 89 de la séquence du CD40 humain montrée dans la SÉQ ID No. : 10 ou la SÉQ ID No. : 12 ; et
d) un anticorps monoclonal qui est en compétition avec l'anticorps monoclonal CHIR-5.9 pouvant être obtenu à partir de la lignée cellulaire d'hybridomes déposée auprès de l'ATCC sous le No. de dépôt de brevet PTA-5542 ou avec l'anticorps monoclonal CHIR-12.12 pouvant être obtenu à partir de la lignée cellulaire d'hybridomes déposée auprès de l'ATCC sous le No. de dépôt de brevet PTA-5543, dans un dosage par liaison compétitive,
destiné à être utilisé dans l'inhibition de la croissance d'une tumeur solide comprenant des cellules d'un carcinome exprimant l'antigène CD40.

5. Utilisation d'une quantité efficace d'un anticorps monoclonal anti-CD40 humain qui est capable de se lier spécifiquement à l'antigène CD40 dans la fabrication d'un médicament destiné à inhiber la croissance d'une tumeur solide comprenant des cellules d'un carcinome exprimant l'antigène CD40, ledit anticorps monoclonal ne présentant pas d'activité agoniste significative lorsqu'il est lié à l'antigène CD40, ledit anticorps étant choisi dans le groupe constitué par :
a) un anticorps monoclonal qui se lie à un épitope capable de se lier à l'anticorps monoclonal CHIR-5.9 pouvant être obtenu à partir de la lignée cellulaire d'hybridomes déposée auprès de l'ATCC sous le No. de dépôt de brevet PTA-5542, ou à l'anticorps monoclonal CHIR-12.12 pouvant être obtenu à partir de la lignée cellulaire d'hybridomes déposée auprès de l'ATCC sous le No. de dépôt de brevet PTA-5543 ;
b) un anticorps monoclonal qui se lie à un épitope comprenant les résidus 82 à 87 de la séquence du CD40 humain montrée dans la SÉQ ID No. : 10 ou la SÉQ ID No. : 12 ;
c) un anticorps monoclonal qui se lie à un épitope comprenant les résidus 82 à 89 de la séquence du CD40 humain montrée dans la SÉQ ID No. : 10 ou la SÉQ ID No. : 12 ; et
d) un anticorps monoclonal qui est en compétition avec l'anticorps monoclonal CHIR-5.9 pouvant être obtenu à partir de la lignée cellulaire d'hybridomes déposée auprès de l'ATCC sous le No. de dépôt de brevet PTA-5542 ou avec l'anticorps monoclonal CHIR-12.12 pouvant être obtenu à partir de la lignée cellulaire d'hybridomes déposée auprès de l'ATCC sous le No. de dépôt de brevet PTA-5543, dans un dosage par liaison compétitive.

6. Anticorps, utilisation ou méthode selon l'une quelconque des revendications 1 à 5, dans lesquels ledit anticorps ou fragment se liant à l'antigène de celui-ci est choisi dans le groupe constitué par :
(i) un anticorps ou fragment se liant à l'antigène de celui-ci comprenant un domaine variable de chaîne légère contenant les résidus 44 à 54, 70 à 76 et 109 à 117 de la SÉQ ID No. : 2 ou de la SÉQ ID No. : 6 ;
(ii) un anticorps ou fragment se liant à l'antigène de celui-ci comprenant un domaine variable de chaîne lourde contenant les résidus 50 à 54, 69 à 84 et 114 à 121 de la SÉQ ID No. : 4 ou de la SÉQ ID No. : 7 ;
(iii) un anticorps ou fragment se liant à l'antigène de celui-ci comprenant un domaine variable de chaîne légère contenant les résidus 46 à 52, 70 à 72 et 111 à 116 de la SÉQ ID No. : 2 ou de la SÉQ ID No. : 6 ; et
(iv) un anticorps ou fragment se liant à l'antigène de celui-ci comprenant un domaine variable de chaîne lourde contenant les résidus 45 à 51, 72 à 74 et 115 à 120 de la SÉQ ID No. : 4 ou de la SÉQ ID No. : 7.

7. Anticorps, utilisation ou méthode selon l'une quelconque des revendications 1 à 5, dans lesquels ledit anticorps comprend une séquence d'acides aminés choisie dans le groupe constitué par :
(i) les résidus 21 à 132 de la SÉQ ID No. : 2 ;
(ii) les résidus 21 à 239 de la SÉQ ID No. : 2 ;
(iii) la SÉQ ID No. : 2 ;
(iv) les résidus 20 à 139 de la SÉQ ID No. : 4 ;
(v) les résidus 20 à 469 de la SÉQ ID No. : 4 ;
(vi) la SÉQ ID No. : 4 ;
(vii) les résidus 20 à 469 de la SÉQ ID No. : 5 ;
(viii) la SÉQ ID No. : 5 ;
(ix) les résidus 21 à 132 de la SÉQ ID No. : 2 et les résidus 20 à 139 de la SÉQ ID N. 4 ;
(x) les résidus 21 à 239 de la SÉQ ID No. : 2 et les résidus 20 à 469 de la SÉQ ID No. : 4 ;
(xi) les résidus 21 à 239 de la SÉQ ID No. : 2 et les résidus 20 à 469 de la SÉQ ID No. : 5 ;
(xii) la SÉQ ID No. : 2 et la SÉQ ID No. : 4 ; et
(xiii) la SÉQ ID No. : 2 et la SÉQ ID No. : 5.

8. Anticorps, utilisation ou méthode selon l'une quelconque des revendications 1 à 5, dans lesquels ledit anticorps ou fragment se liant à l'antigène de celui-ci comprend une séquence d'acides aminés choisie dans le groupe constitué par :
(i) les résidus 21 à 132 de la SÉQ ID No. : 6 ;
(ii) les résidus 21 à 239 de la SÉQ ID No. : 6 ;
(iii) la SÉQ ID No. : 6 ;
(iv) les résidus 20 à 144 de la SÉQ ID No. : 7 ;
(v) les résidus 20 à 474 de la SÉQ ID No. : 7 ;
(vi) la SÉQ ID No. : 7 ;
(vii) les résidus 20 à 474 de la SÉQ ID No. : 8 ;
(viii) la SÉQ ID No. : 8 ;
(ix) les résidus 21 à 132 de la SÉQ ID No. : 6 et les résidus 20 à 144 de la SÉQ ID No. : 7 ;
(x) les résidus 21 à 239 de la SÉQ ID No. : 6 et les résidus 20 à 474 de la SÉQ ID No. : 7 ;
(xi) les résidus 21 à 239 de la SÉQ ID No. : 6 et les résidus 20 à 474 de la SÉQ ID No. : 8 ;
(xii) la SÉQ ID NO: 6 et la SÉQ ID NO: 7 ; et
(xiii) la SÉQ ID No. : 6 et la SÉQ ID No. : 8.

9. Anticorps, utilisation ou méthode selon l'une quelconque des revendications précédentes, dans lesquels ledit anticorps est l'anticorps monoclonal CHIR-5.9 pouvant être obtenu à partir de la lignée cellulaire d'hybridomes déposée auprès de l'ATCC sous le No. de dépôt de brevet PTA-5542 ou l'anticorps monoclonal CHIR-12.12 pouvant être obtenu à partir de la lignée cellulaire d'hybridomes déposée auprès de l'ATCC sous le No. de dépôt de brevet PTA-5543.

10. Anticorps, utilisation ou méthode selon l'une quelconque des revendications précédentes, dans lesquels ladite tumeur solide est choisie dans le groupe constitué par un carcinome bronchique, un carcinome du sein, un carcinome de l'ovaire, un carcinome cutané, un carcinome du colon, un carcinome de la vessie, un carcinome du foie, un carcinome gastrique, un cancer de la prostate, un carcinome du rein, un carcinome rhino-pharyngien, un carcinome malpighien, un carcinome papillaire thyroïdien, un carcinome du col de l'utérus, et des sarcomes.

11. Anticorps, utilisation ou méthode selon la revendication 10, dans lesquels ledit médicament est administré selon une méthode comprenant en outre l'étape consistant à administrer audit sujet au moins un autre protocole de traitement du cancer choisi dans le groupe constitué par la chirurgie, la radiothérapie, la chimiothérapie, une thérapie par cytokines, et un autre anticorps monoclonal destiné à être utilisé dans le traitement de ladite tumeur solide.

12. Anticorps, utilisation ou méthode selon l'une quelconque des revendications 1 à 5, dans lesquels ledit anticorps monoclonal est un fragment se liant à l'antigène d'un anticorps monoclonal, ledit fragment gardant la capacité à se lier spécifiquement audit antigène CD40 humain.

13. Anticorps, utilisation ou méthode selon la revendication 12, dans lesquels ledit fragment d'anticorps se liant à l'antigène est choisi dans le groupe constitué par un fragment Fab, un fragment F(ab')₂, un fragment Fv, et un fragment Fv à chaîne simple.

14. Anticorps, utilisation ou méthode selon l'une quelconque des revendications précédentes, dans lesquels ledit anticorps monoclonal ou fragment se liant à l'antigène de celui-ci se lie à l'antigène CD40 humain avec une affinité (K_{D}) d'au moins 10⁻⁶ M.

15. Anticorps, utilisation ou méthode selon la revendication 14, dans lesquels ledit anticorps monoclonal ou fragment se liant à l'antigène de celui-ci se lie à l'antigène CD40 humain avec une affinité (K_{D}) d'au moins 10⁻⁸ M.

16. Anticorps, utilisation ou méthode selon l'une quelconque des revendications précédentes, dans lesquels ledit anticorps monoclonal ou fragment se liant à l'antigène de celui-ci est produit dans une lignée cellulaire CHO.


### SEQUENCE LISTING

<110> Long, Li
   Luqman, Mohammad
   Yabannavar, Asha
   Zaror, Isabel
<120> Use of Antagonist Anti-CD40 Monoclonal
   Antibodies for Treatment of Multiple Myeloma
<130> PP22589.002 (282915)
<150> 60/565,709
   <151> 2004-04-26
<150> 60/565,710
   <151> 2004-04-27
<150> 60/525,579
   <151> 2003-11-26
<150> 60/517,337
   <151> 2003-11-04
<160> 12
<170> FastSEQ for Windows Version 4.0
<210> 1
   <211> 720
   <212> DNA
   <213> Artificial Sequence
<220>
   <223> Coding sequence for light chain of 12.12 human anti-CD40 antibody
<221> CDS
   <222> (1)...(720)
<400> 1
   atg gcg ctc cct gct cag ctc ctg ggg ctg cta atg ctc tgg gtc tct    48
   Met Ala Leu Pro Ala Gln Leu Leu Gly Leu Leu Met Leu Trp Val Ser
    1               5                  10                  15
   gga tcc agt ggg gat att gtg atg act cag tct cca ctc tcc ctg acc    96
   Gly Ser Ser Gly Asp Ile Val Met Thr Gln Ser Pro Leu Ser Leu Thr
               20                  25                  30
   gtc acc cct gga gag ccg gcc tcc atc tcc tgc agg tcc agt cag agc    144
   Val Thr Pro Gly Glu Pro Ala Ser Ile Ser Cys Arg Ser Ser Gln Ser
           35                  40                  45
   ctc ctg tat agt aat gga tac aac tat ttg gat tgg tac ctg cag aag    192
   Leu Leu Tyr Ser Asn Gly Tyr Asn Tyr Leu Asp Trp Tyr Leu Gln Lys
        50                  55                  60
   cca ggg cag tct cca cag gtc ctg atc tct ttg ggt tct aat cgg gcc    240
   Pro Gly Gln Ser Pro Gln Val Leu Ile Ser Leu Gly Ser Asn Arg Ala
    65                  70                  75                  80
   tcc ggg gtc cct gac agg ttc agt ggc agt gga tca ggc aca gat ttt    288
   Ser Gly Val Pro Asp Arg Phe Ser Gly Ser Gly Ser Gly Thr Asp Phe
                   85                  90                  95
   aca ctg aaa atc agc aga gtg gag gct gag gat gtt ggg gtt tat tac     336
   Thr Leu Lys Ile Ser Arg Val Glu Ala Glu Asp Val Gly Val Tyr Tyr
               100                 105                 110
   tgc atg caa gct cga caa act cca ttc act ttc ggc cct ggg acc aaa     384
   Cys Met Gln Ala Arg Gln Thr Pro Phe Thr Phe Gly Pro Gly Thr Lys
           115                 120                 125
   gtg gat atc aga cga act gtg gct gca cca tct gtc ttc atc ttc ccg     432
   Val Asp Ile Arg Arg Thr Val Ala Ala Pro Ser Val Phe Ile Phe Pro
       130                 135                 140
   cca tct gat gag cag ttg aaa tct gga act gcc tct gtt gtg tgc ctg     480
   Pro Ser Asp Glu Gln Leu Lys Ser Gly Thr Ala Ser Val Val Cys Leu
   145                 150                 155                 160
   ctg aat aac ttc tat ccc aga gag gcc aaa gta cag tgg aag gtg gat     528
   Leu Asn Asn Phe Tyr Pro Arg Glu Ala Lys Val Gln Trp Lys Val Asp
                   165                 170                 175
   aac gcc ctc caa tcg ggt aac tcc cag gag agt gtc aca gag cag gac     576
   Asn Ala Leu Gln Ser Gly Asn Ser Gln Glu Ser Val Thr Glu Gln Asp
               180                 185                 190
   agc aag gac agc acc tac agc ctc agc agc acc ctg acg ctg agc aaa     624
   Ser Lys Asp Ser Thr Tyr Ser Leu Ser Ser Thr Leu Thr Leu Ser Lys
           195                 200                 205
   gca gac tac gag aaa cac aaa gtc tac gcc tgc gaa gtc acc cat cag     672
   Ala Asp Tyr Glu Lys His Lys Val Tyr Ala Cys Glu Val Thr His Gln
       210                 215                 220
   ggc ctg agc tcg ccc gtc aca aag agc ttc aac agg gga gag tgt tag     720
   Gly Leu Ser Ser Pro Val Thr Lys Ser Phe Asn Arg Gly Glu Cys  *
   225                 230                 235
<210> 2
   <211> 239
   <212> PRT
   <213> Artificial Sequence
<220>
   <223> Light chain of 12.12 human anti-CD40 antibody
<400> 2
   Met Ala Leu Pro Ala Gln Leu Leu Gly Leu Leu Met Leu Trp Val Ser
    1               5                  10                  15
   Gly Ser Ser Gly Asp Ile Val Met Thr Gln Ser Pro Leu Ser Leu Thr
               20                  25                  30
   Val Thr Pro Gly Glu Pro Ala Ser Ile Ser Cys Arg Ser Ser Gln Ser
           35                  40                  45
   Leu Leu Tyr Ser Asn Gly Tyr Asn Tyr Leu Asp Trp Tyr Leu Gln Lys
       50                  55                  60
   Pro Gly Gln Ser Pro Gln Val Leu Ile Ser Leu Gly Ser Asn Arg Ala
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Met Gln Ala Arg Gln Thr Pro Phe Thr Phe Gly Pro Gly Thr Lys
           115                 120                 125
   Val Asp Ile Arg Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 3
   <211> 2016
   <212> DNA
   <213> Artificial Sequence
<220>
   <223> Coding sequence for heavy chain of 12.12 human anti-CD40 antibody (with introns)
<400> 3
   atggagtttg ggctgagctg ggttttcctt gttgctattt taagaggtgt ccagtgtcag 60
   gtgcagttgg tggagtctgg gggaggcgtg gtccagcctg ggaggtccct gagactctcc 120
   tgtgcagcct ctggattcac cttcagtagc tatggcatgc actgggtccg ccaggctcca 180
   ggcaaggggc tggagtgggt ggcagttata tcatatgagg aaagtaatag ataccatgca 240
   gactccgtga agggccgatt caccatctcc agagacaatt ccaagatcac gctgtatctg 300
   caaatgaaca gcctcagaac tgaggacacg gctgtgtatt actgtgcgag agatgggggt 360
   atagcagcac ctgggcctga ctactggggc cagggaaccc tggtcaccgt ctcctcagca 420
   agtaccaagg gcccatccgt cttccccctg gcgcccgcta gcaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagagagt tggtgagagg 720
   ccagcacagg gagggagggt gtctgctgga agccaggctc agcgctcctg cctggacgca 780
   tcccggctat gcagtcccag tccagggcag caaggcaggc cccgtctgcc tcttcacccg 840
   gaggcctctg cccgccccac tcatgctcag ggagagggtc ttctggcttt ttccccaggc 900
   tctgggcagg cacaggctag gtgcccctaa cccaggccct gcacacaaag gggcaggtgc 960
   tgggctcaga cctgccaaga gccatatccg ggaggaccct gcccctgacc taagcccacc 1020
   ccaaaggcca aactctccac tccctcagct cggacacctt ctctcctccc agattccagt 1080
   aactcccaat cttctctctg cagagcccaa atcttgtgac aaaactcaca catgcccacc 1140
   gtgcccaggt aagccagccc aggcctcgcc ctccagctca aggcgggaca ggtgccctag 1200
   agtagcctgc atccagggac aggccccagc cgggtgctga cacgtccacc tccatctctt 1260
   cctcagcacc tgaactcctg gggggaccgt cagtcttcct cttcccccca aaacccaagg 1320
   acaccctcat gatctcccgg acccctgagg tcacatgcgt ggtggtggac gtgagccacg 1380
   aagaccctga ggtcaagttc aactggtacg tggacggcgt ggaggtgcat aatgccaaga 1440
   caaagccgcg ggaggagcag tacaacagca cgtaccgtgt ggtcagcgtc ctcaccgtcc 1500
   tgcaccagga ctggctgaat ggcaaggagt acaagtgcaa ggtctccaac aaagccctcc 1560
   cagcccccat cgagaaaacc atctccaaag ccaaaggtgg gacccgtggg gtgcgagggc 1620
   cacatggaca gaggccggct cggcccaccc tctgccctga gagtgaccgc tgtaccaacc 1680
   tctgtcccta cagggcagcc ccgagaacca caggtgtaca ccctgccccc atcccgggag 1740
   gagatgacca agaaccaggt cagcctgacc tgcctggtca aaggcttcta tcccagcgac 1800
   atcgccgtgg agtgggagag caatgggcag ccggagaaca actacaagac cacgcctccc 1860
   gtgctggact ccgacggctc cttcttcctc tatagcaagc tcaccgtgga caagagcagg 1920
   tggcagcagg ggaacgtctt ctcatgctcc gtgatgcatg aggctctgca caaccactac 1980
   acgcagaaga gcctctccct gtctccgggt aaatga                           2016
<210> 4
   <211> 469
   <212> PRT
   <213> Artificial Sequence
<220>
   <223> Heavy chain of 12.12 human anti-CD40 antibody
<400> 4
   Met Glu Phe Gly Leu Ser Trp Val Phe Leu Val Ala Ile Leu Arg Gly
    1               5                  10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Ser Tyr Gly Met His Trp Val Arg Gln Ala Pro Gly Lys Gly Leu
       50                  55                  60
   Glu Trp Val Ala Val Ile Ser Tyr Glu Glu Ser Asn Arg Tyr His Ala
   65                  70                  75                  80
   Asp Ser Val Lys Gly Arg Phe Thr Ile Ser Arg Asp Asn Ser Lys Ile
                   85                  90                  95
   Thr Leu Tyr Leu Gln Met Asn Ser Leu Arg Thr Glu Asp Thr Ala Val
               100                 105                 110
   Tyr Tyr Cys Ala Arg Asp Gly Gly Ile Ala Ala Pro Gly Pro Asp Tyr
           115                 120                 125
   Trp Gly Gln Gly Thr Leu Val Thr Val Ser Ser Ala Ser Thr Lys Gly
       130                 135                 140
   Pro Ser Val Phe Pro Leu Ala Pro Ala Ser Lys Ser Thr Ser Gly Gly
   145                 150                 155                 160
   Thr Ala Ala Leu Gly Cys Leu Val Lys Asp Tyr Phe Pro Glu Pro Val
                   165                 170                 175
   Thr Val Ser Trp Asn Ser Gly Ala Leu Thr Ser Gly Val His Thr Phe
               180                 185                 190
   Pro Ala Val Leu Gln Ser Ser Gly Leu Tyr Ser Leu Ser Ser Val Val
           195                 200                 205
   Thr Val Pro Ser Ser Ser Leu Gly Thr Gln Thr Tyr Ile Cys Asn Val
       210                 215                 220
   Asn His Lys Pro Ser Asn Thr Lys Val Asp Lys Arg Val Glu Pro Lys
   225                 230                 235                 240
   Ser Cys Asp Lys Thr His Thr Cys Pro Pro Cys Pro Ala Pro Glu Leu
                   245                 250                 255
   Leu Gly Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Lys Phe Asn Trp Tyr Val Asp Gly Val
       290                 295                 300
   Glu Val His Asn Ala Lys Thr Lys Pro Arg Glu Glu Gln Tyr Asn Ser
   305                 310                 315                 320
   Thr Tyr Arg Val Val Ser Val Leu Thr Val Leu His Gln Asp Trp Leu
                   325                 330                 335
   Asn Gly Lys Glu Tyr Lys Cys Lys Val Ser Asn Lys Ala Leu Pro Ala
               340                 345                 350
   Pro Ile Glu Lys Thr Ile Ser Lys Ala Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Val Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 5
   <211> 469
   <212> PRT
   <213> Artificial Sequence
<220>
   <223> Heavy chain of variant of 12.12 human anit-CD40 antibody
<400> 5
   Met Glu Phe Gly Leu Ser Trp Val Phe Leu Val Ala Ile Leu Arg Gly
    1               5                  10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Ser Tyr Gly Met His Trp Val Arg Gln Ala Pro Gly Lys Gly Leu
       50                  55                  60
   Glu Trp Val Ala Val Ile Ser Tyr Glu Glu Ser Asn Arg Tyr His Ala
   65                  70                  75                  80
   Asp Ser Val Lys Gly Arg Phe Thr Ile Ser Arg Asp Asn Ser Lys Ile
                   85                  90                  95
   Thr Leu Tyr Leu Gln Met Asn Ser Leu Arg Thr Glu Asp Thr Ala Val
               100                 105                 110
   Tyr Tyr Cys Ala Arg Asp Gly Gly Ile Ala Ala Pro Gly Pro Asp Tyr
           115                 120                 125
   Trp Gly Gln Gly Thr Leu Val Thr Val Ser Ser Ala Ser Thr Lys Gly
       130                 135                 140
   Pro Ser Val Phe Pro Leu Ala Pro Ser Ser Lys Ser Thr Ser Gly Gly
   145                 150                 155                 160
   Thr Ala Ala Leu Gly Cys Leu Val Lys Asp Tyr Phe Pro Glu Pro Val
                   165                 170                 175
   Thr Val Ser Trp Asn Ser Gly Ala Leu Thr Ser Gly Val His Thr Phe
               180                 185                 190
   Pro Ala Val Leu Gln Ser Ser Gly Leu Tyr Ser Leu Ser Ser Val Val
           195                 200                 205
   Thr Val Pro Ser Ser Ser Leu Gly Thr Gln Thr Tyr Ile Cys Asn Val
       210                 215                 220
   Asn His Lys Pro Ser Asn Thr Lys Val Asp Lys Arg Val Glu Pro Lys
   225                 230                 235                 240
   Ser Cys Asp Lys Thr His Thr Cys Pro Pro Cys Pro Ala Pro Glu Leu
                   245                 250                 255
   Leu Gly Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Lys Phe Asn Trp Tyr Val Asp Gly Val
       290                 295                 300
   Glu Val His Asn Ala Lys Thr Lys Pro Arg Glu Glu Gln Tyr Asn Ser
   305                 310                 315                 320
   Thr Tyr Arg Val Val Ser Val Leu Thr Val Leu His Gln Asp Trp Leu
                   325                 330                 335
   Asn Gly Lys Glu Tyr Lys Cys Lys Val Ser Asn Lys Ala Leu Pro Ala
               340                 345                 350
   Pro Ile Glu Lys Thr Ile Ser Lys Ala Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Val Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 6
   <211> 239
   <212> PRT
   <213> Artificial Sequence
<220>
   <223> Light chain of 5.9 human anti-CD40 antibody
<400> 6
   Met Ala Leu Leu Ala Gln Leu Leu Gly Leu Leu Met Leu Trp Val Pro
    1               5                  10                  15
   Gly Ser Ser Gly Ala Ile Val Met Thr Gln Pro Pro Leu Ser Ser Pro
               20                  25                  30
   Val Thr Leu Gly Gln Pro Ala Ser Ile Ser Cys Arg Ser Ser Gln Ser
           35                  40                  45
   Leu Val His Ser Asp Gly Asn Thr Tyr Leu Asn Trp Leu Gln Gln Arg
       50                  55                  60
   Pro Gly Gln Pro Pro Arg Leu Leu Ile Tyr Lys Phe Phe Arg Arg Leu
   65                  70                  75                  80
   Ser Gly Val Pro Asp Arg Phe Ser Gly Ser Gly Ala Gly Thr Asp Phe
                   85                  90                  95
   Thr Leu Lys Ile Ser Arg Val Glu Ala Glu Asp Val Gly Val Tyr Tyr
               100                 105                 110
   Cys Met Gln Val Thr Gln Phe Pro His Thr Phe Gly Gln Gly Thr Arg
           115                 120                 125
   Leu Glu Ile Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 7
   <211> 474
   <212> PRT
   <213> Artificial Sequence
<220>
   <223> Heavy chain of 5.9 human anti-CD40 antibody
<400> 7
   Met Gly Ser Thr Ala Ile Leu Ala Leu Leu Leu Ala Val Leu Gln Gly
    1               5                  10                  15
   Val Cys Ala Glu Val Gln Leu Val Gln Ser Gly Ala Glu Val Lys Lys
               20                  25                  30
   Pro Gly Glu Ser Leu Lys Ile Ser Cys Lys Gly Ser Gly Tyr Ser Phe
           35                  40                  45
   Thr Ser Tyr Trp Ile Gly Trp Val Arg Gln Met Pro Gly Lys Gly Leu
       50                  55                  60
   Glu Trp Met Gly Ile Ile Tyr Pro Gly Asp Ser Asp Thr Arg Tyr Ser
   65                  70                  75                  80
   Pro Ser Phe Gln Gly Gln Val Thr Ile Ser Ala Asp Lys Ser Ile Ser
                   85                  90                  95
   Thr Ala Tyr Leu Gln Trp Ser Ser Leu Lys Ala Ser Asp Thr Ala Met
               100                 105                 110
   Tyr Tyr Cys Ala Arg Gly Thr Ala Ala Gly Arg Asp Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Ala Ser Lys
   145                 150                 155                 160
   Ser Thr Ser Gly Gly Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Ser Leu Gly Thr Gln Thr
       210                 215                 220
   Tyr Ile Cys Asn Val Asn His Lys Pro Ser Asn Thr Lys Val Asp Lys
   225                 230                 235                 240
   Arg Val Glu Pro Lys Ser Cys Asp Lys Thr His Thr Cys Pro Pro Cys
                   245                 250                 255
   Pro Ala Pro Glu Leu Leu Gly Gly Pro Ser Val Phe Leu Phe Pro Pro
               260                 265                 270
   Lys Pro Lys Asp Thr Leu Met Ile Ser Arg Thr Pro Glu Val Thr Cys
           275                 280                 285
   Val Val Val Asp Val Ser His Glu Asp Pro Glu Val Lys Phe Asn Trp
       290                 295                 300
   Tyr Val Asp Gly Val Glu Val His Asn Ala Lys Thr Lys Pro Arg Glu
   305                 310                 315                 320
   Glu Gln Tyr Asn Ser Thr Tyr Arg Val Val Ser Val Leu Thr Val Leu
                   325                 330                 335
   His Gln Asp Trp Leu Asn Gly Lys Glu Tyr Lys Cys Lys Val Ser Asn
               340                 345                 350
   Lys Ala Leu Pro Ala Pro Ile Glu Lys Thr Ile Ser Lys Ala Lys Gly
           355                 360                 365
   Gln Pro Arg Glu Pro Gln Val Tyr Thr Leu Pro Pro Ser Arg Glu Glu
       370                 375                 380
   Met Thr Lys Asn Gln Val Ser Leu Thr Cys Leu Val Lys Gly Phe Tyr
   385                 390                 395                 400
   Pro Ser Asp Ile Ala Val Glu Trp Glu Ser Asn Gly Gln Pro Glu Asn
                   405                 410                 415
   Asn Tyr Lys Thr Thr Pro Pro Val Leu Asp Ser Asp Gly Ser Phe Phe
               420                 425                 430
   Leu Tyr Ser Lys Leu Thr Val Asp Lys Ser Arg Trp Gln Gln Gly Asn
           435                 440                 445
   Val Phe Ser Cys Ser Val Met His Glu Ala Leu His Asn His Tyr Thr
       450                 455                 460
   Gln Lys Ser Leu Ser Leu Ser Pro Gly Lys
   465                 470
<210> 8
   <211> 474
   <212> PRT
   <213> Artificial Sequence
<220>
   <223> Heavy chain of variant 5.9 human anti-CD40 antibody
<400> 8
   Met Gly Ser Thr Ala Ile Leu Ala Leu Leu Leu Ala Val Leu Gln Gly
    1               5                  10                  15
   Val Cys Ala Glu Val Gln Leu Val Gln Ser Gly Ala Glu Val Lys Lys
               20                  25                  30
   Pro Gly Glu Ser Leu Lys Ile Ser Cys Lys Gly Ser Gly Tyr Ser Phe
           35                  40                  45
   Thr Ser Tyr Trp Ile Gly Trp Val Arg Gln Met Pro Gly Lys Gly Leu
       50                  55                  60
   Glu Trp Met Gly Ile Ile Tyr Pro Gly Asp Ser Asp Thr Arg Tyr Ser
   65                  70                  75                  80
   Pro Ser Phe Gln Gly Gln Val Thr Ile Ser Ala Asp Lys Ser Ile Ser
                   85                  90                  95
   Thr Ala Tyr Leu Gln Trp Ser Ser Leu Lys Ala Ser Asp Thr Ala Met
               100                 105                 110
   Tyr Tyr Cys Ala Arg Gly Thr Ala Ala Gly Arg Asp Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Ser Ser Lys
   145                 150                 155                 160
   Ser Thr Ser Gly Gly Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Ser Leu Gly Thr Gln Thr
       210                 215                 220
   Tyr Ile Cys Asn Val Asn His Lys Pro Ser Asn Thr Lys Val Asp Lys
   225                 230                 235                 240
   Arg Val Glu Pro Lys Ser Cys Asp Lys Thr His Thr Cys Pro Pro Cys
                   245                 250                 255
   Pro Ala Pro Glu Leu Leu Gly Gly Pro Ser Val Phe Leu Phe Pro Pro
               260                 265                 270
   Lys Pro Lys Asp Thr Leu Met Ile Ser Arg Thr Pro Glu Val Thr Cys
           275                 280                 285
   Val Val Val Asp Val Ser His Glu Asp Pro Glu Val Lys Phe Asn Trp
       290                 295                 300
   Tyr Val Asp Gly Val Glu Val His Asn Ala Lys Thr Lys Pro Arg Glu
   305                 310                 315                 320
   Glu Gln Tyr Asn Ser Thr Tyr Arg Val Val Ser Val Leu Thr Val Leu
                   325                 330                 335
   His Gln Asp Trp Leu Asn Gly Lys Glu Tyr Lys Cys Lys Val Ser Asn
               340                 345                 350
   Lys Ala Leu Pro Ala Pro Ile Glu Lys Thr Ile Ser Lys Ala Lys Gly
           355                 360                 365
   Gln Pro Arg Glu Pro Gln Val Tyr Thr Leu Pro Pro Ser Arg Glu Glu
       370                 375                 380
   Met Thr Lys Asn Gln Val Ser Leu Thr Cys Leu Val Lys Gly Phe Tyr
   385                 390                 395                 400
   Pro Ser Asp Ile Ala Val Glu Trp Glu Ser Asn Gly Gln Pro Glu Asn
                   405                 410                 415
   Asn Tyr Lys Thr Thr Pro Pro Val Leu Asp Ser Asp Gly Ser Phe Phe
               420                 425                 430
   Leu Tyr Ser Lys Leu Thr Val Asp Lys Ser Arg Trp Gln Gln Gly Asn
           435                 440                 445
   Val Phe Ser Cys Ser Val Met His Glu Ala Leu His Asn His Tyr Thr
       450                 455                 460
   Gln Lys Ser Leu Ser Leu Ser Pro Gly Lys
   465                 470
<210> 9
   <211> 612
   <212> DNA
   <213> Homo sapiens
<220>
   <221> CDS
   <222> (1)...(612)
<221> misc_feature
   <222> (0)...(0)
   <223> Coding sequence for short isoform of human CD40
<400> 9
   atg gtt cgt ctg cct ctg cag tgc gtc ctc tgg ggc tgc ttg ctg acc     48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta     96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg     144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa     192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac     240
   Ser Glu Phe Leu Asp Thr Trp Asn Arg Glu Thr His Cys His Gln His
    65                  70                  75                  80
   aaa tac tgc gac ccc aac cta ggg ctt cgg gtc cag cag aag ggc acc     288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg     336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc     384
   Ser Glu Ala Cys Glu Ser Cys Val Leu His Arg Ser Cys Ser Pro Gly
           115                 120                 125
   ttt ggg gtc aag cag att gct aca ggg gtt tct gat acc atc tgc gag     432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa     480
   Pro Cys Pro Val Gly Phe Phe Ser Asn Val Ser Ser Ala Phe Glu Lys
   145                 150                 155                 160
   tgt cac cct tgg aca agg tcc cca gga tcg gct gag agc cct ggt ggt     528
   Cys His Pro Trp Thr Arg Ser Pro Gly Ser Ala Glu Ser Pro Gly Gly
                   165                 170                 175
   gat ccc cat cat ctt cgg gat cct gtt tgc cat cct ctt ggt gct ggt     576
   Asp Pro His His Leu Arg Asp Pro Val Cys His Pro Leu Gly Ala Gly
               180                 185                 190
   ctt tat caa aaa ggt ggc caa gaa gcc aac caa taa                     612
   Leu Tyr Gln Lys Gly Gly Gln Glu Ala Asn Gln  *
           195                 200
<210> 10
   <211> 203
   <212> PRT
   <213> Homo sapiens
<400> 10
   Met Val Arg Leu Pro Leu Gln Cys Val Leu Trp Gly Cys Leu Leu Thr
    1               5                  10                  15
   Ala Val His Pro Glu Pro Pro Thr Ala Cys Arg Glu Lys Gln Tyr Leu
               20                  25                  30
   Ile Asn Ser Gln Cys Cys Ser Leu Cys Gln Pro Gly Gln Lys Leu Val
           35                  40                  45
   Ser Asp Cys Thr Glu Phe Thr Glu Thr Glu Cys Leu Pro Cys Gly Glu
       50                  55                  60
   Ser Glu Phe Leu Asp Thr Trp Asn Arg Glu Thr His Cys His Gln His
   65                  70                  75                  80
   Lys Tyr Cys Asp Pro Asn Leu Gly Leu Arg Val Gln Gln Lys Gly Thr
                   85                  90                  95
   Ser Glu Thr Asp Thr Ile Cys Thr Cys Glu Glu Gly Trp His Cys Thr
               100                 105                 110
   Ser Glu Ala Cys Glu Ser Cys Val Leu His Arg Ser Cys Ser Pro Gly
           115                 120                 125
   Phe Gly Val Lys Gln Ile Ala Thr Gly Val Ser Asp Thr Ile Cys Glu
       130                 135                 140
   Pro Cys Pro Val Gly Phe Phe Ser Asn Val Ser Ser Ala Phe Glu Lys
   145                 150                 155                 160
   Cys His Pro Trp Thr Arg Ser Pro Gly Ser Ala Glu Ser Pro Gly Gly
                   165                 170                 175
   Asp Pro His His Leu Arg Asp Pro Val Cys His Pro Leu Gly Ala Gly
               180                 185                 190
   Leu Tyr Gln Lys Gly Gly Gln Glu Ala Asn Gln
           195                 200
<210> 11
   <211> 834
   <212> DNA
   <213> Homo sapiens
<220>
   <221> CDS
   <222> (1)...(834)
<221> misc_feature
   <222> (0)...(0)
   <223> Coding sequence for long isoform of human CD40
<400> 11
   atg gtt cgt ctg cct ctg cag tgc gtc ctc tgg ggc tgc ttg ctg acc    48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta    96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg    144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa    192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac    240
   Ser Glu Phe Leu Asp Thr Trp Asn Arg Glu Thr His Cys His Gln His
   65                  70                  75                  80
   aaa tac tgc gac ccc aac cta ggg ctt cgg gtc cag cag aag ggc acc    288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg    336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc     384
   Ser Glu Ala Cys Glu Ser Cys Val Leu His Arg Ser Cys Ser Pro Gly
           115                 120                 125
   ttt ggg gtc aag cag att gct aca ggg gtt tct gat acc atc tgc gag     432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa     480
   Pro Cys Pro Val Gly Phe Phe Ser Asn Val Ser Ser Ala Phe Glu Lys
   145                 150                 155                 160
   tgt cac cct tgg aca agc tgt gag acc aaa gac ctg gtt gtg caa cag     528
   Cys His Pro Trp Thr Ser Cys Glu Thr Lys Asp Leu Val Val Gln Gln
                   165                 170                 175
   gca ggc aca aac aag act gat gtt gtc tgt ggt ccc cag gat cgg ctg     576
   Ala Gly Thr Asn Lys Thr Asp Val Val Cys Gly Pro Gln Asp Arg Leu
               180                 185                 190
   aga gcc ctg gtg gtg atc ccc atc atc ttc ggg atc ctg ttt gcc atc     624
   Arg Ala Leu Val Val Ile Pro Ile Ile Phe Gly Ile Leu Phe Ala Ile
           195                 200                 205
   ctc ttg gtg ctg gtc ttt atc aaa aag gtg gcc aag aag cca acc aat     672
   Leu Leu Val Leu Val Phe Ile Lys Lys Val Ala Lys Lys Pro Thr Asn
       210                 215                 220
   aag gcc ccc cac ccc aag cag gaa ccc cag gag atc aat ttt ccc gac     720
   Lys Ala Pro His Pro Lys Gln Glu Pro Gln Glu Ile Asn Phe Pro Asp
   225                 230                 235                 240
   gat ctt cct ggc tcc aac act gct gct cca gtg cag gag act tta cat     768
   Asp Leu Pro Gly Ser Asn Thr Ala Ala Pro Val Gln Glu Thr Leu His
                   245                 250                 255
   gga tgc caa ccg gtc acc cag gag gat ggc aaa gag agt cgc atc tca     816
   Gly Cys Gln Pro Val Thr Gln Glu Asp Gly Lys Glu Ser Arg Ile Ser
               260                 265                 270
   gtg cag gag aga cag tga                                             834
   Val Gln Glu Arg Gln  *
           275
<210> 12
   <211> 277
   <212> PRT
   <213> Homo sapiens
<400> 12
   Met Val Arg Leu Pro Leu Gln Cys Val Leu Trp Gly Cys Leu Leu Thr
    1              5                   10                  15
   Ala Val His Pro Glu Pro Pro Thr Ala Cys Arg Glu Lys Gln Tyr Leu
               20                  25                  30
   Ile Asn Ser Gln Cys Cys Ser Leu Cys Gln Pro Gly Gln Lys Leu Val
           35                  40                  45
   Ser Asp Cys Thr Glu Phe Thr Glu Thr Glu Cys Leu Pro Cys Gly Glu
       50                  55                  60
   Ser Glu Phe Leu Asp Thr Trp Asn Arg Glu Thr His Cys His Gln His
   65                  70                  75                  80
   Lys Tyr Cys Asp Pro Asn Leu Gly Leu Arg Val Gln Gln Lys Gly Thr
                   85                  90                  95
   Ser Glu Thr Asp Thr Ile Cys Thr Cys Glu Glu Gly Trp His Cys Thr
               100                 105                 110
   Ser Glu Ala Cys Glu Ser Cys Val Leu His Arg Ser Cys Ser Pro Gly
           115                 120                 125
   Phe Gly Val Lys Gln Ile Ala Thr Gly Val Ser Asp Thr Ile Cys Glu
       130                 135                 140
   Pro Cys Pro Val Gly Phe Phe Ser Asn Val Ser Ser Ala Phe Glu Lys
   145                 150                 155                 160
   Cys His Pro Trp Thr Ser Cys Glu Thr Lys Asp Leu Val Val Gln Gln
                   165                 170                 175
   Ala Gly Thr Asn Lys Thr Asp Val Val Cys Gly Pro Gln Asp Arg Leu
               180                 185                 190
   Arg Ala Leu Val Val Ile Pro Ile Ile Phe Gly Ile Leu Phe Ala Ile
           195                 200                 205
   Leu Leu Val Leu Val Phe Ile Lys Lys Val Ala Lys Lys Pro Thr Asn
       210                 215                 220
   Lys Ala Pro His Pro Lys Gln Glu Pro Gln Glu Ile Asn Phe Pro Asp
   225                 230                 235                 240
   Asp Leu Pro Gly Ser Asn Thr Ala Ala Pro Val Gln Glu Thr Leu His
                   245                 250                 255
   Gly Cys Gln Pro Val Thr Gln Glu Asp Gly Lys Glu Ser Arg Ile Ser
               260                 265                 270
   Val Gln Glu Arg Gln
           275